# EUROPEAN PATENT SPECIFICATION

(11) **EP 3 906 012 B1**
(45) Date of publication and mention of the grant of the patent: **24.08.2022**
(21) Application number: 19702501.8
(22) Date of filing: 04.01.2019
(51) Int. Cl.: A61K 8/64, A61Q 19/08

(54) **OXIDIZED DERIVATIVES OF GDF-11 FRAGMENTS**
OXIDIERTE DERIVATE VON GDF-11-FRAGMENTEN
DÉRIVÉS OXYDÉS DE FRAGMENTS DE GDF-11

(43) Date of publication of application: 10.11.2021
(73) Proprietor: Avon Products, Inc., Suffern, NY 10901 (US)
(72) Inventor: IDKOWIAK BALDYS, Jolanta, New York 10901 (US); LYGA, John W., Basking Ridge, New Jersey 07920 (US); LUO, Robert Zhiyong, New York 10956 (US)
(74) Representative: Abel & Imray LLP
(86) International application number: PCT/US2019/012325
(87) International publication number: WO 2020/142103

(56) References cited:
- EP-A1- 0 436 189
- EP-A1- 2 649 984
- WO-A1-2016/204841
- WO-A2-2010/082175
- WO-A2-2010/120691
- RU-C1- 2 206 573
- US-A- 5 962 292

## Description

### FIELD OF INVENTION

The present invention relates generally to peptides derivatives, in particular peptides derived from the Growth Differentiation Factor 11 (GDF-11) protein and topical formulations containing them, as well as associated non-therapeutic methods for improving the health of skin and/or diminishing the dermatological signs of aging in human skin. The peptide derivatives derived from GDF-11 protein exhibit increased bioactivity than the nonderivatized GDF-11 fragments. For example, oxidation of one or more amino acid residues of GDF-11 fragments leads to increases in hyaluronic acid production and/or collagen production in fibroblasts.

### BACKGROUND

Growth factors are naturally occurring substances, usually proteins, that act as signaling molecules between cells. Their primary function is promoting cell differentiation and maturation. Growth factors play an important role in many functions, such as stimulating cell growth, proliferation, and wound healing. Many large classes, or superfamilies, of related growth factors are known.

Growth Differentiation Factor 11 (GDF-11) is a protein belonging to the transforming growth factor (TGF) superfamily (*e.g*., TGF-β), which encompasses a group of structurally-related proteins. Blood-derived GDF-11 was recently shown to be involved in reverting the aging phenotype in mice, including cardiac hypertrophy (see Loffredo et al., Cell, 2013, 153, 828-839), age-related sarcopenia (see Sinha et al., Science, 2014, 344:649-52), and decreased cognitive functions (see Villeda et al., Nat. Med. 2014, 20:659-63). Due to the many important roles growth factors play in maintaining healthy tissues, there has been some interest in using them in dermatological formulations. WO 2010/082175 A2 discloses peptidic compounds, in particular of the KXK (lysine-AA-lysine) type, and their use in cosmetic compositions to stimulate extracellular matrix components. There are, however, drawbacks associated with the use of growth factors in topical formulations. Additionally, GDF-11 fragments have been shown to result in increases in hyaluronic acid and/or collagen production in fibroblasts. However, these fragments are also beholden to various drawbacks including stability issues. WO 2016/204841 A1 discloses cosmetic compositions comprising a topically acceptable vehicle and an active agent comprising a GDF-11 derived peptide and their non-therapeutic method for diminishing signs of aging.

Additionally, derivatizing peptides has known drawbacks as well. For example, oxidation of peptide residues such as methionine may reduce efficacy (see Unnikrishnan et al., Agents and Actions, 1990, 31:110-112) or may destabilize the peptides.

It is therefore an object of the invention to provide new derivatives of peptides thereof derived from GDF-11 and topical compositions containing them uninhibitited by these drawbacks. It is also an object of the invention to provide non-therapeutic methods for improving the health and/or appearance of skin, combatting signs of intrinsic and photoaging, and/or treating skin disorders. It is a further object of the invention to provide compositions and non-therapeutic methods for treating, reversing, forestalling and/or ameliorating skin wrinkles and fine lines, tightening sagging skin, firming skin, and for treating hyperpigmentation and unwanted pigmentation with cosmetic compositions comprising effective amounts of a peptide derivative of the invention. WO 2010/120691 A2 discloses compositions comprising a cosmetically or pharmaceutically acceptable base and at least 0.01% by weight of one or more oxidized peptides and methods of reducing the amount of oxidized, methionine residues in human skin. EP 2 649 984 A1 discloses peptidic compounds, their cosmetic or pharmaceutical compositions, and their use in medicine to inhibit neuronal exocytosis. RU 2 206 573 C1 discloses peptidic compounds and their use in neurotropic preparations to elicit neurotropic activity.

The foregoing discussion is presented solely to provide a better understanding of the nature of the problems confronting the art and should not be construed in any way as an admission as to prior art.

### SUMMARY OF THE INVENTION

The present invention provides a topical composition comprising an active agent as defined in claim 1; and a non-therapeutic method for diminishing the appearance of dermatological signs of aging as defined in claim 12. Preferred embodiments are set out in the dependent claims. The active agents are believed to be useful for improving one or more signs of dermatological aging when topically applied to human integuments (e.g., skin, lips, nails, hair, etc.), particularly skin. They are also contemplated to be useful in treating a variety of dermatological disorders and improving the overall health of skin. The peptides of the invention may be derived from human growth factor GDF-11. In some embodiments, the peptide derivatives are oxidized GDF-11 fragments (e.g., small peptide sequences of GDF-11 wherein one or more amino acid residues have bene oxidized, etc.). In some embodiments, the active agents of the invention are capable of increasing collagen and/or hyaluronic acid (HA) production of skin cells and therefore will have a beneficial effect on reducing the appearance of aging on skin (e.g., diminishing the appearance of wrinkles and/or fine lines, tightening sagging skin, thickening thinning skin, evening skin tone, treating hyperpigmentation and unwanted pigmentation, etc.).

The present invention relates to a topical composition comprising an active agent comprising a peptide having an amino acid sequence comprising or consisting of M(O)₂VV or M(O)VV, wherein the peptide may be smaller peptide sequence derivatives (e.g., 3-11 or 3 or 4 or 5 or 6 or 7 or 8 or 9 or 10 or 11 amino acids) of the full-length GDF-11 protein which increase hyaluronic acid (HA) and/or collagen in fibroblasts as compared to the nonderivatized versions of the peptide. Nonderivatized versions of the peptide may be disclosed in US Pat No 10,034,826. These peptides, especially peptides similar or homologous to putative functional regions of the protein, are contemplated to have biological activity, including antiaging benefits in skin. It has been found that derivatives of these smaller peptides have increased biological activity than the nonderivatized peptide. For example, smaller sequences of the GDF-11 protein may be derivatized by modification of one or more amino acid residue. According to the present invention, the peptides have an amino acid sequence comprising or consisting of M(O)₂VV or M(O)VV, wherein the methionine amino acid residue of said sequence is oxidized. In certain embodiments, the peptide derivative of the invention comprises a thiol or thioether functional group which has been oxidized. For example, the peptide derivative may comprise methionine sulfone or methionine sulfoxide, and cysteine disulfide. In some embodiments, the peptide derivative is methionine sulfone valine valine (M(O)₂VV) which has the structure: 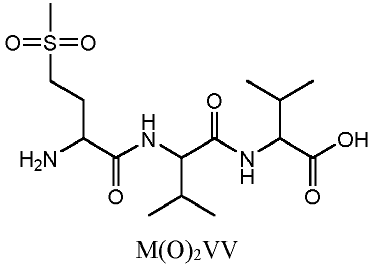 or a derivative thereof (including without limitation C₁₋₁₈ acyl derivatives at the amino terminus and/or C₁₋₁₈ ester derivatives at the acid terminus) or a salt thereof. In some embodiments, the peptide derivative is methionine sulfoxide valine valine (M(O)VV) which has the structure: 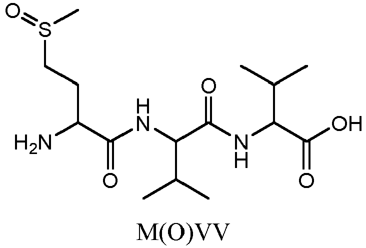 or a derivative thereof (including without limitation C₁₋₁₈ acyl derivatives at the amino terminus and/or C₁₋₁₈ ester derivatives at the acid terminus) or a salt thereof.

Each amino acid residue or derivatized residue may be in the natural enantiomeric form. In some embodiments, the amino acid residues or derivatized residues may independently be in the *L* optical form or the *D* enantiomeric form. In some embodiments, each amino acid residue and derivatized residue may have the *L* form. In some embodiments, the stereochemical assignment of the amino acid or derivatized residue may be independently selected from *R* or *S.* For example, peptide derivative may have the structure: 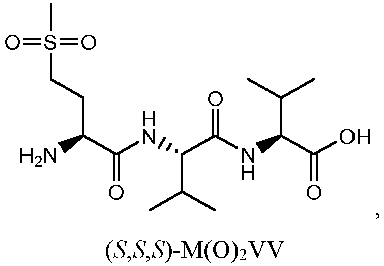 or

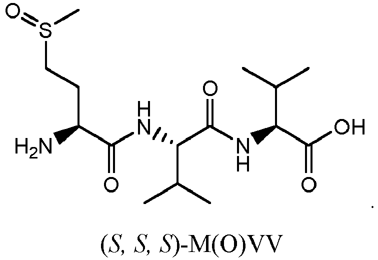

The peptide derivative may also be independently functionalized at the amino and/or carboxylic acid terminus with one or more hydrocarbons. In some embodiments, the peptide derivative (including M(O)VV and M(O)₂VV) may acetylated at the amino terminus. For example, the peptide derivative may be Ac-M(O)₂VV which has the structure: 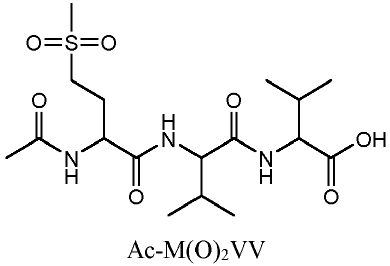 or a derivative thereof (including without limitation C₁₋₁₈ acyl derivatives at the amino terminus and/or C₁₋₁₈ ester derivatives at the acid terminus) or a salt thereof. In some embodiments, the peptide derivative may be Ac-M(O)VV which has the structure: 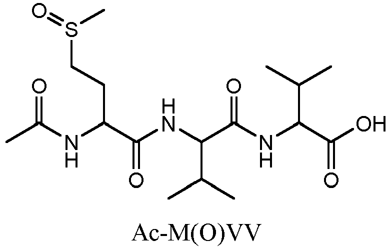 or a derivative thereof (including without limitation C₁₋₁₈ acyl derivatives at the amino terminus and/or C₁₋₁₈ ester derivatives at the acid terminus) or a salt thereof.

The present invention provides compositions for topical use comprising an active agent comprising a peptide (*e.g.*, having from 3-11 consecutive amino acids from the GDF-11 sequence) having an amino acid sequence comprising or consisting of M(O)₂VV or M(O)VV, wherein the methionine amino acid residue of said sequence is oxidized. The peptide may include cyclic peptide fragments of the invention in a physiologically acceptable carrier, wherein the methionine amino acid residue of said fragmentshas been oxidized. In certain embodiments, one or more *(e.g.,* one, two, three, four, five, six, seven, etc.) amino acid residues of the peptide sequence have been derivatized (*e.g*., oxidized, etc.). In some embodiments, the peptide having an amino acid sequence comprising or consisting of M(O)₂VV or M(O)VV further comprises cysteine. In some embodiments, the peptide derivative comprises oxidized cysteine. The peptide derivativesmay be present in the composition in an amount between 0.000001% to 10% *(e.g.,* 0.0001-1%) by weight of the composition. Peptides useful in the practice of the invention include, for example, those comprising 3 amino acids (SEQ ID NO: 2-375); 4 amino acids (SEQ ID NO: 376-767); 5 amino acids (SEQ ID NO:768-1161); 6 amino acids (SEQ ID NO: 1162-1556); 7 amino acids (SEQ ID NO: 1557-1951); 8 amino acids (SEQ ID NO: 1952-2346); 9 amino acids (SEQ ID NO: 2347-2741); 10 amino acids (SEQ ID NO: 2742-3136); 11 amino acids (SEQ ID NO: 3137-3531) or even larger fragments of GDF-11. The peptide derivative may be derived from GDF-11 fragments wherein one or more amino acid residues have been oxidized. The present invention also provides for a non-therapeutic method for diminishing the appearance of dermatological signs of aging comprising topically applying to the skin in need thereof a topical composition as described above. Non-therapeutic methods are provided for ameliorating and/or preventing signs of human skin photoaging and intrinsic aging (*e.g*., diminishing the appearance of wrinkles and/or fine lines, tightening sagging skin, thickening thinning skin, evening skin tone, treating hyperpigmentation, etc.) comprising topically applying to the skin (*e.g*., skin of the face) a composition comprising, in a topically acceptable vehicle, a peptide within the topical composition of the invention.

These and other aspects of the present invention will become apparent to those skilled in the art after a reading of the following detailed description of the invention, including the illustrative embodiments and examples.

### BRIEF DESCRIPTION OF THE FIGURES

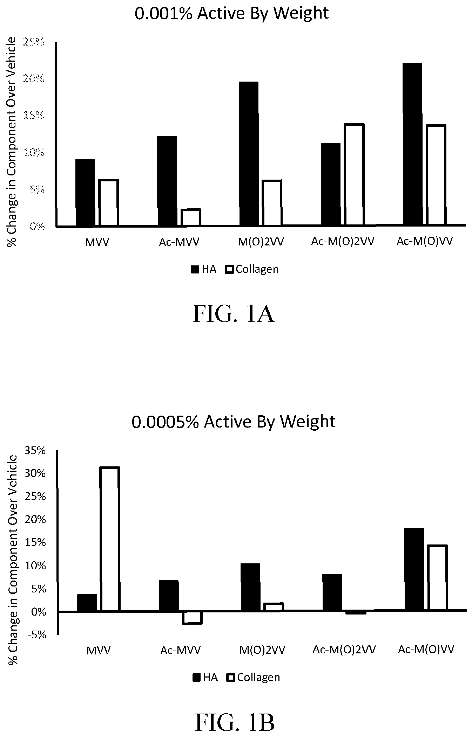

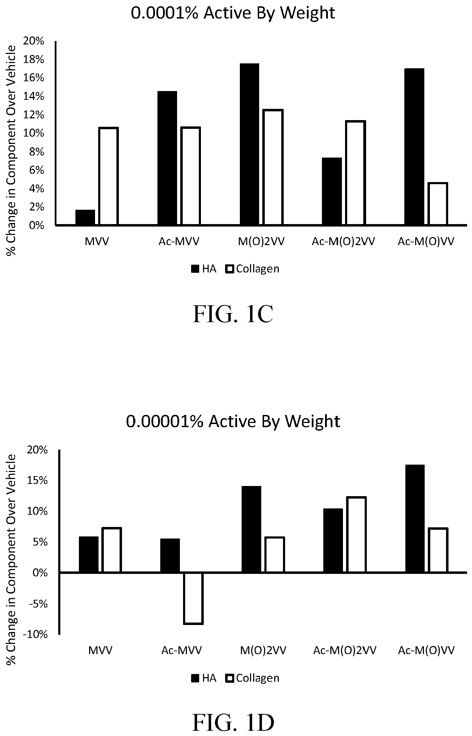

FIG. 1A shows the results of administration of various peptides or peptide derivatives in an *in vitro* assay at 0.001% by weight (10 ppm) in control (DMSO). FIG. 1B shows the results of administration of various peptides or peptide derivatives in an *in vitro* assay at 0.0005% by weight (5 ppm) in control (DMSO). FIG. 1C shows the results of administration of various peptides or peptide derivatives in an *in vitro* assay at 0.0001% by weight (1 ppm) in control (DMSO). FIG. ID shows the results of administration of various peptides or peptide derivatives in an *in vitro* assay at 0.00001% by weight (0.1 ppm) in control (DMSO).

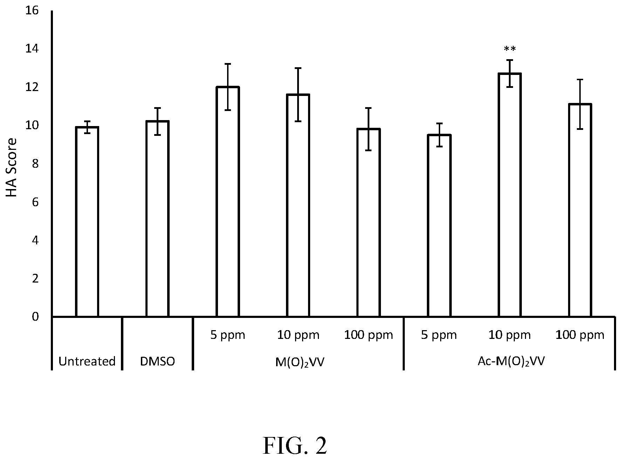

FIG. 2 shows the measured HA score of the *ex vivo* assay following administration of either M(O)₂VV or Ac-M(O)₂VV at 0.0005%, 0.001%, or 0.01% by weight of the composition. Columns marked with "^{∗∗}" were highly significant (p<0.05) with respect to untreated samples and with respect to samples administered vehicle alone (DMSO).

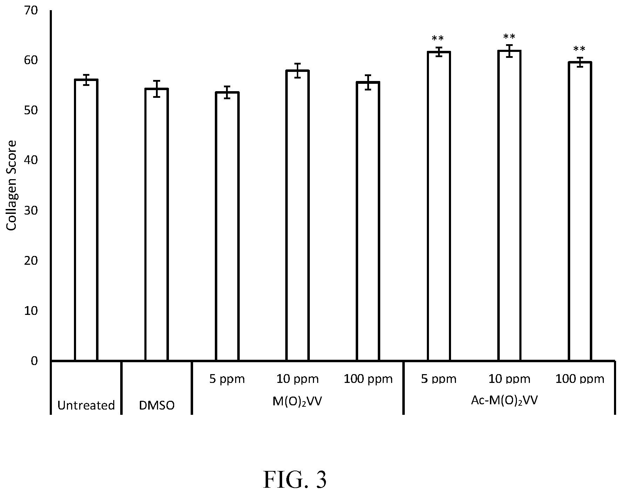

FIG. 3 shows the measured collagen score of the *ex vivo* assay following administration of either M(O)₂VV or Ac-M(O)₂VV at 0.0005%, 0.001%, or 0.01% by weight of the composition. Columns marked with "^{∗∗}" were highly significant (p<0.05) with respect to untreated samples and with respect to samples administered vehicle alone.

### DETAILED DESCRIPTION

Detailed embodiments of the present invention are disclosed herein; however, it is to be understood that the disclosed embodiments are merely illustrative of the invention that may be embodied in various forms. In addition, each of the examples given in connection with the various embodiments of the invention is intended to be illustrative, and not restrictive. Therefore, specific structural and functional details disclosed herein are not to be interpreted as limiting, but merely as a representative basis for teaching one skilled in the art to variously employ the present invention.

All percentages given herein refer to the weight percentages of a particular component relative to the entire composition, including the vehicle, unless otherwise indicated. It will be understood that the sum of all weight % of individual components within a composition will not exceed 100%. If the amount of an ingredient is not otherwise specified, it may be present in an amount from 0.00001-90% by weight.

All terms used herein are intended to have their ordinary meaning unless otherwise provided. The phrases "physiologically acceptable," "topically acceptable," and "dermatologically acceptable" are used interchangeably and are intended to mean that a particular component is generally regarded as safe and non-toxic for application to a human integument (*e.g*., skin) at the levels employed. The term "prevent," as used herein, includes delaying, slowing or forestalling the onset of or progression of a particular sign of skin aging. The phrase "individual in need thereof' refers to a human that could benefit from improved dermal appearance or health, including males or females. In some embodiments, the individual in need thereof is a female. The term "skin" includes, without limitation, the lips, skin of the face, hands, arms, neck, scalp, and chest. The term "thin" skin includes, but is not limited to, skin that is prematurely thinned, and may be diagnosed as such by a dermatologist. In some embodiments, the thin skin is skin of a female under the age of 60; 50; 40; and/or skin of a pre-menopausal, peri-menopausal or post-menopausal female.

As used herein, the term "consisting essentially of' is intended to limit the invention to the specified materials or steps and those that do not materially affect the basic and novel characteristics of the claimed invention, as understood from a reading of this specification.

As used herein, a hydrocarbon, alkyl, alkenyl, alkynyl, aryl, aryl-alkyl, alkyl-aryl, heteroaryl, or combination of any of those will have from 1-30 carbon atoms, optionally substituted with O, N, S, unless otherwise specified. Any of the alkyl, alkenyl, and alkynyl groups disclosed herein, unless otherwise specified, may be straight-chained, branched, and/or cyclic.

The term "amino acid" is intended to include naturally occurring amino acids and non-proteinogenic amino acids as well as non-naturally occurring amino acids and includes any small molecule (MW < 1,000 Daltons) having at least one carboxyl group and at least one primary or secondary amine group capable of forming peptide bonds. The term "peptide" is intended to include any molecule comprising at least two amino acids joined by a peptide bond and therefore includes di-peptides, tri-peptides, oligopeptides, and polypeptides having up to 20 consecutive amino acid residues linked by peptide bonds. The term "peptide" also embraces structures having one or more linkers, spacers, or terminal groups which are not amino acids. It also includes cyclic peptides.

The term "derivative" of a peptide refers to a chemically modified peptide *(e.g.,* polypeptide) that has been chemically modified, for example, by natural processes such as processing and other other post-translational modifications and/or by chemical modification techniques such as addition of one or more polyethylene glycol molecules, sugars, and phosphates. The modification may occur anywhere in the peptide to produce the peptide derivative including the peptide backbone, the amino acid side-chains, and the amino and/or carboxyl termini. In most embodiments, the peptide derivatives described herein comprise one or more amino acid residues that have been chemically modified. The peptide derivative may have the same modification in two or more locations or different modifications in two or more locations. In some embodiments, the peptide derivative comprises one or more amino acid residues that have been chemically modified in addition to one or more modifications on the amino or carboxyl termini. Modifications include, for example, acetylation, acylation, amidation, covalent attachment of a lipid, covalent attachment of a fatty acid acyl residue, cross-linking, cyclization, disulfide bond formation, demthylation, formation of covalent crosslinks, hydroxylation, iodination, methylation, myritoylation, oxidation, phosphorylation, prenylation, racemization, glycosylation, selenoylation, or sulfation. Suitable modifications may be found, for example, in Proteins-Structure and Molecular Properties 2nd Ed., T.E. Crieghton, W.H. Freeman and Company, New York (1993). In certain embodiments, the peptide derivative comprises one or more (*e.g.*, two, three, four, five, etc.) amino acid residues (*e.g*., methionine, cysteine) modified by oxidation. In some implementations, the peptide derivative comprises one or more (*e.g.*, two, three, four, five, etc.) amino acid residues modified by oxidation and acetylation at the amino and/or carboxylic acid terminus.

### Peptides

The compositions of the present invention may comprise one or more peptide fragments of GDF-11 which have been derivatized. In most embodiments, the peptide derivatives comprise one or more amino acid residues modified by oxidation. The peptides may comprise, consist essentially of, or consist of amino acid sequences derived from the Growth Differentiation Factor 11 (GDF-11) protein. Consisting essentially of, as used herein, is intended to mean that additional amino acids may be present at either terminus provided they do not substantially impair the activity of the peptide. For example, in embodiments where a peptide "consists essentially of' SEQ ID NOs 2-3531, any additional amino acids may be excluded from the peptide if their inclusion produces a measurable improvement (*e.g*., greater than 50% reduction) of the beneficial activity, including, without limitation, upregulation of pro-collagen, collagen, elastin, fibronectin, and/or hyaluronic acid.

In one embodiment, the peptide comprises from 3-11 *(e.g.,* 3, 4, 5, 6, 7, 8, 9, 10, or 11) consecutive amino acids derived from the sequence of Growth Differentiation Factor 11 (GDF-11) precursor [Homo sapiens], NCBI Reference Sequence Accession No.: NP_005802.1, shown in Table 1 (SEQ ID NO: 1).

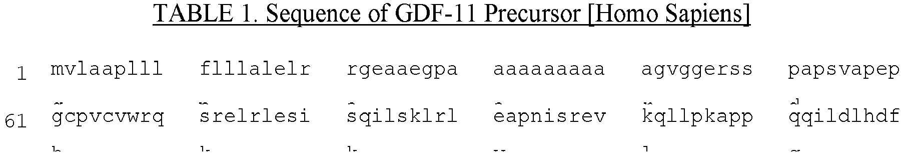

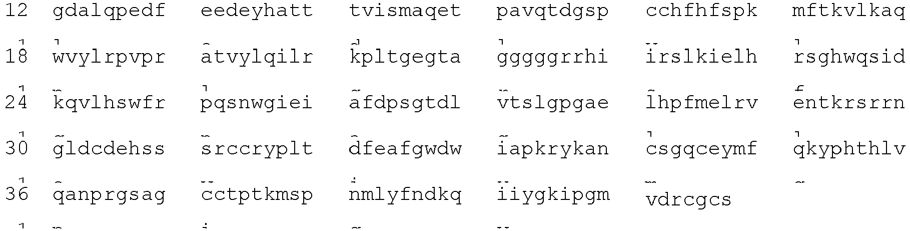

The peptide within the topical compostion of the present invention comprises or consists essentially of the methionine-oxidized derivative of the 3-mer amino acid sequence including SEQ ID NO: 369 listed in Table 2. In some embodiments, the peptide further comprise, of any one or more of the 3-mer amino acid sequences (SEQ ID NO: 2-375) listed in Table 2.

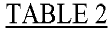

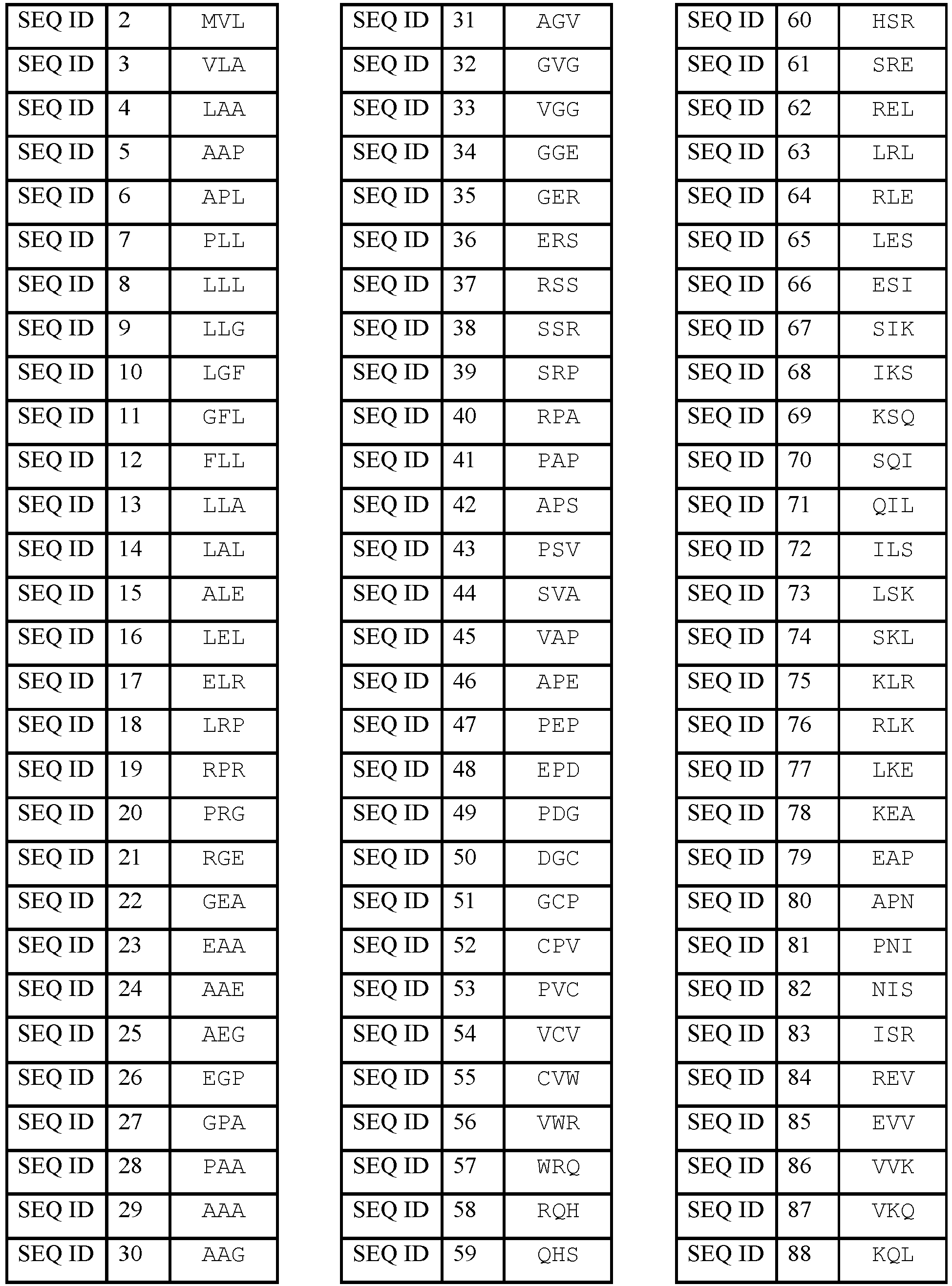

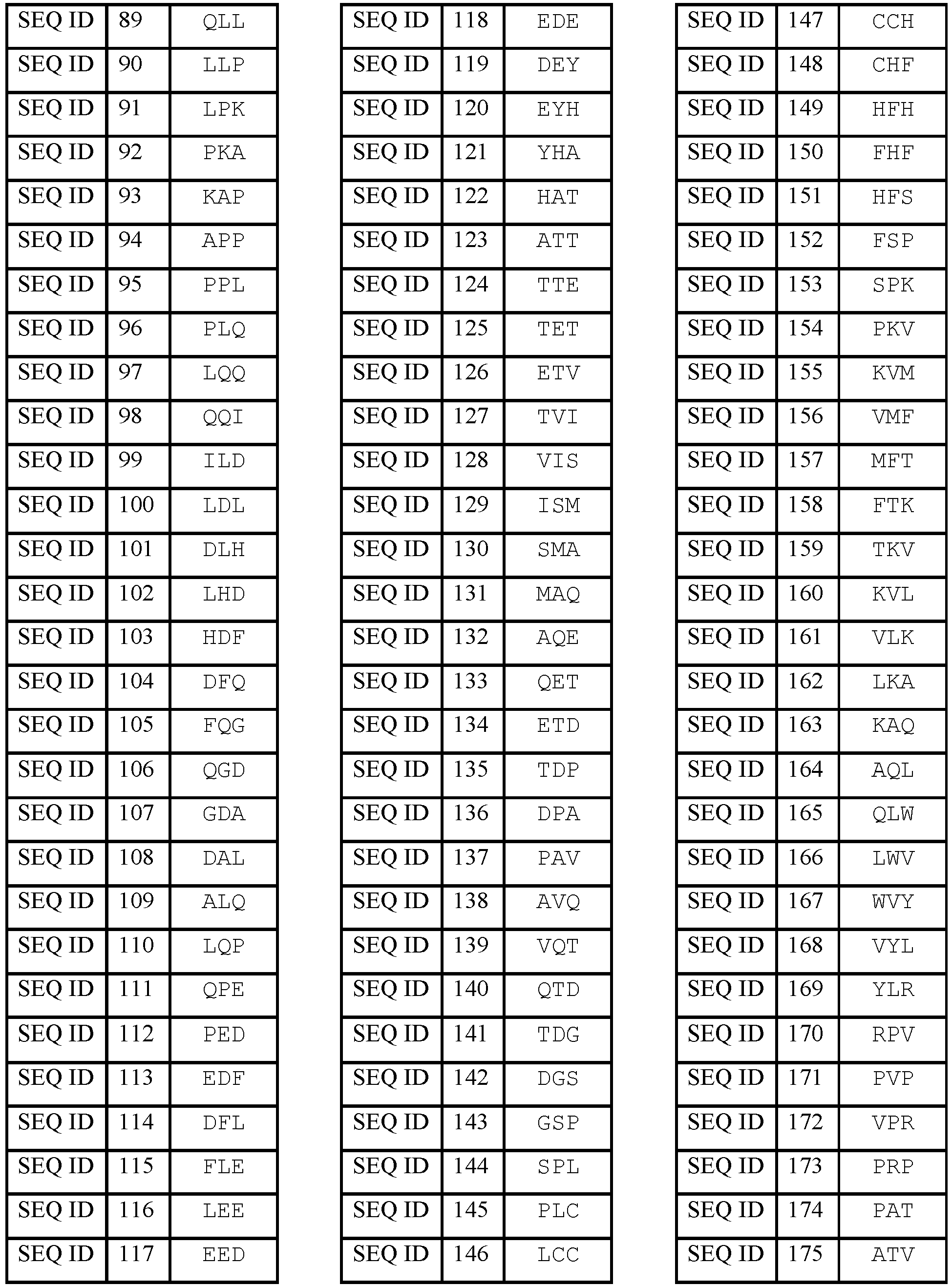

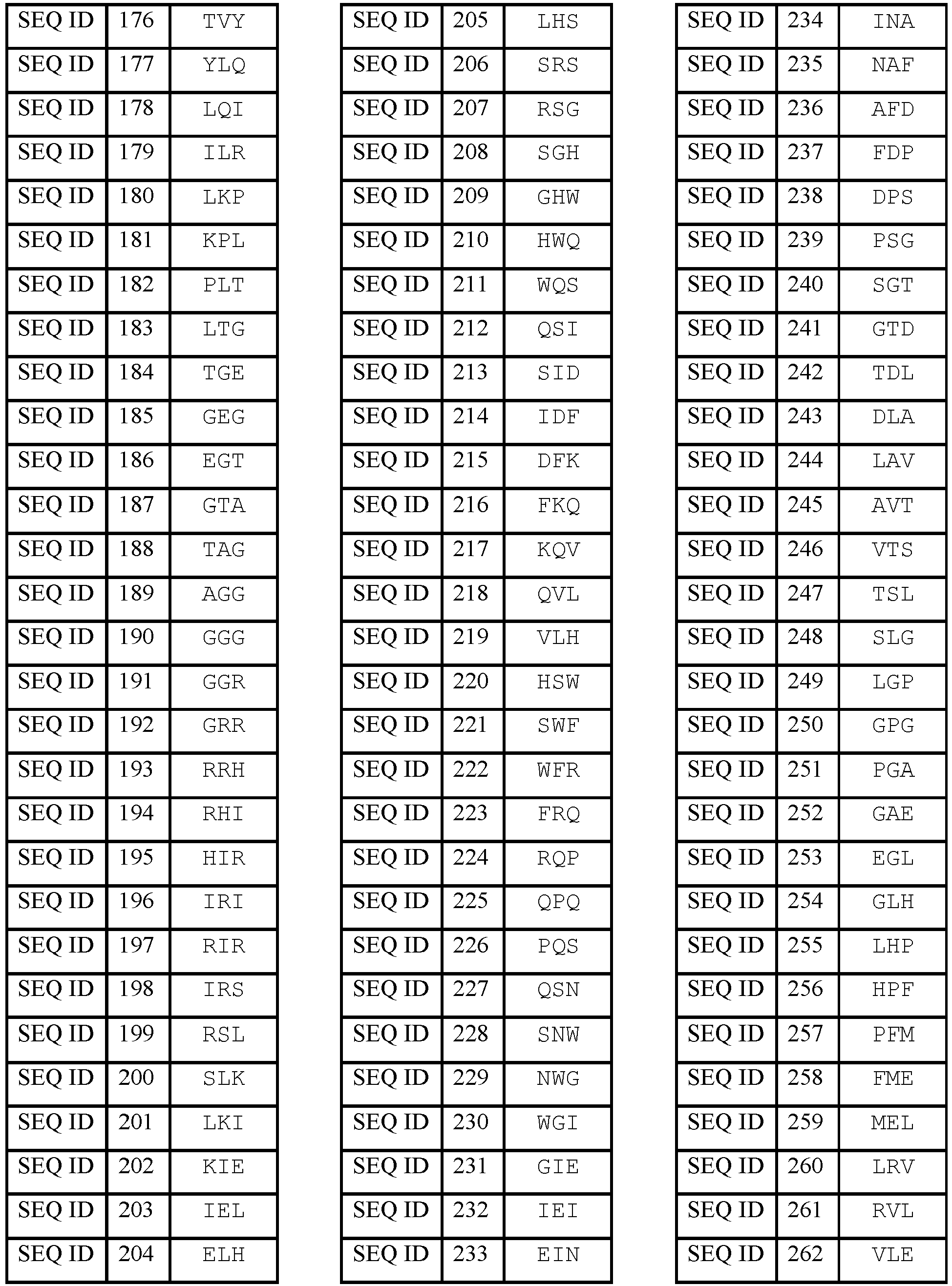

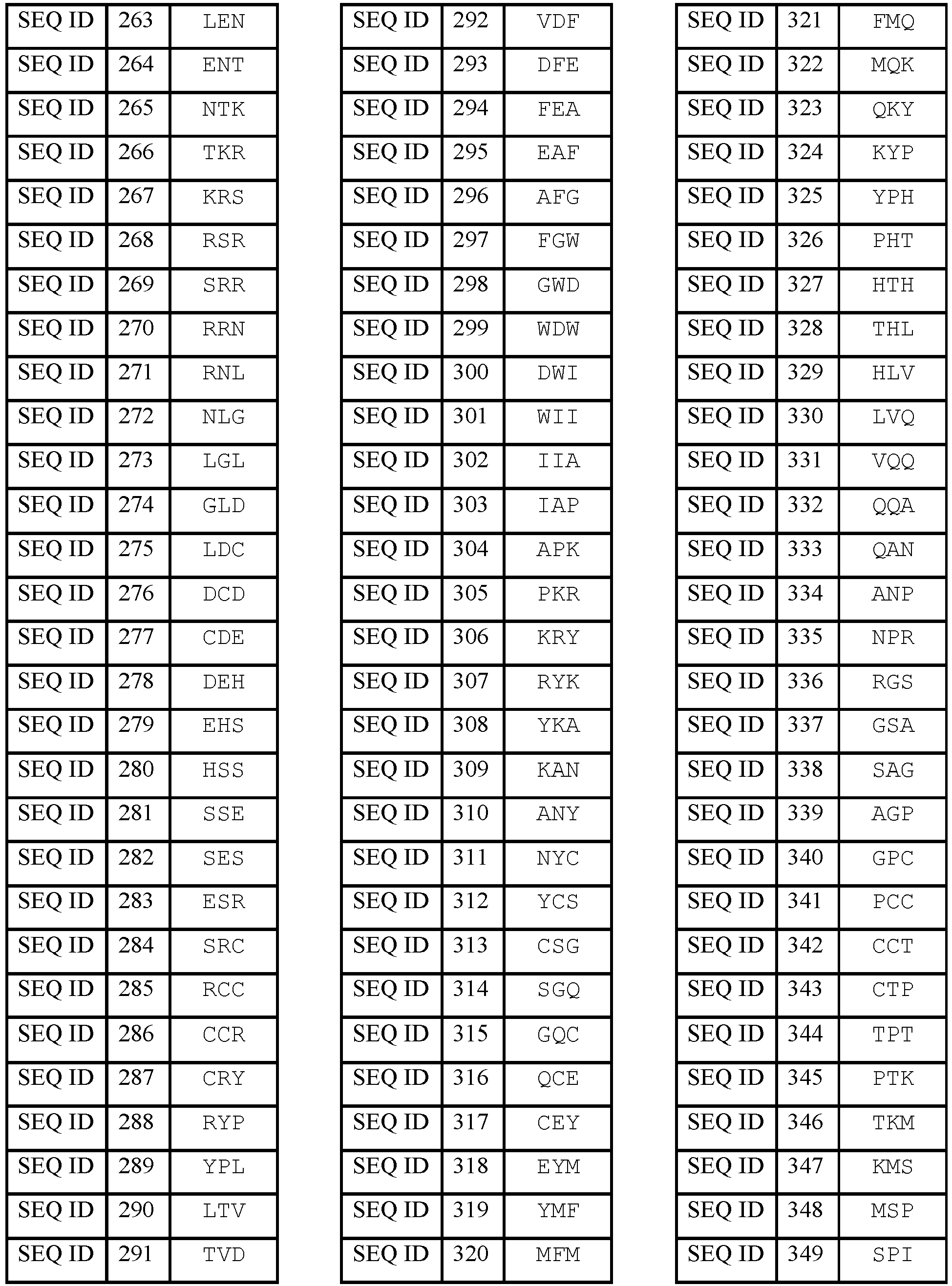

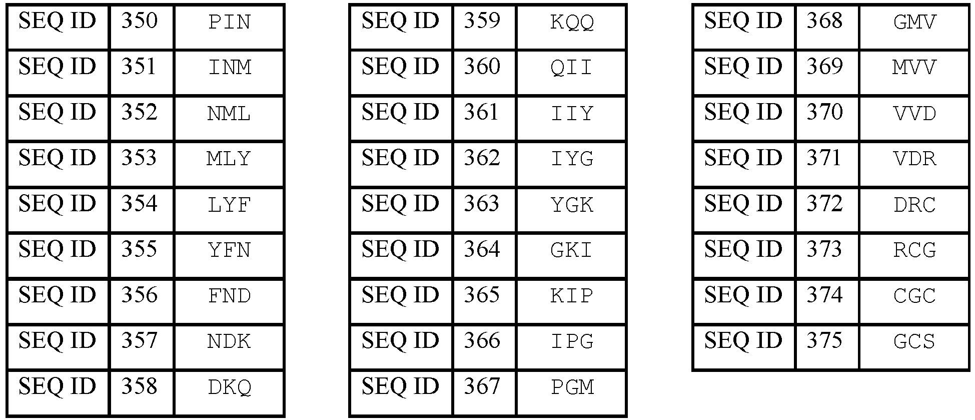

In some embodiments, the peptide comprises, consists essentially of, or consists of any one or more of the 4-mer amino acid sequences (SEQ ID NO: 376-767) listed in Table 3.

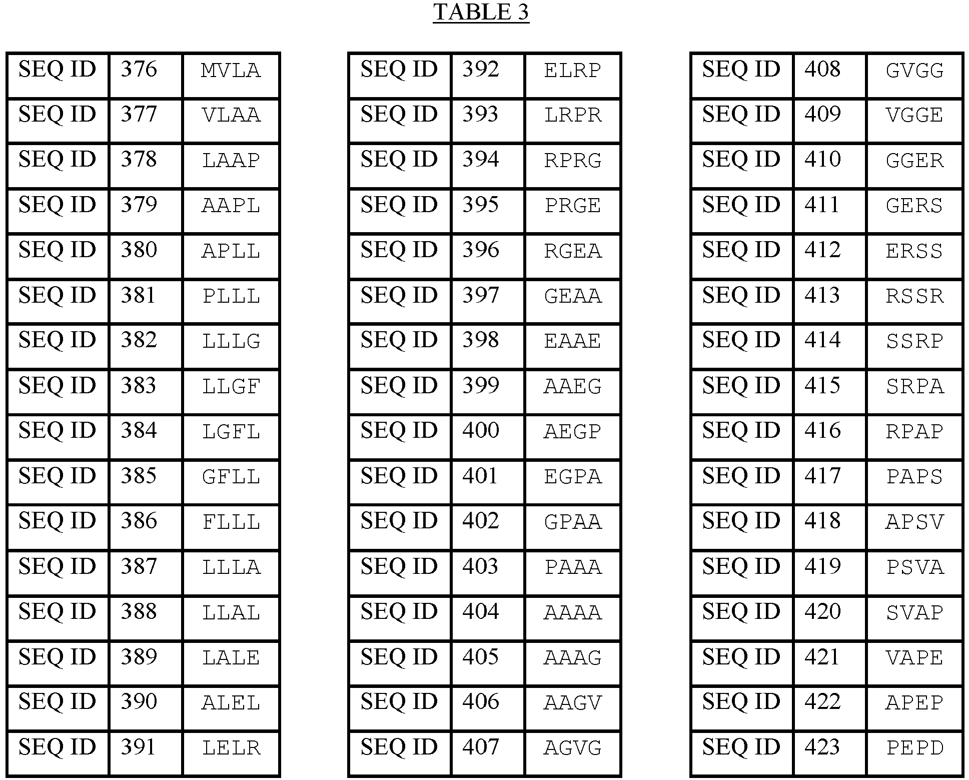

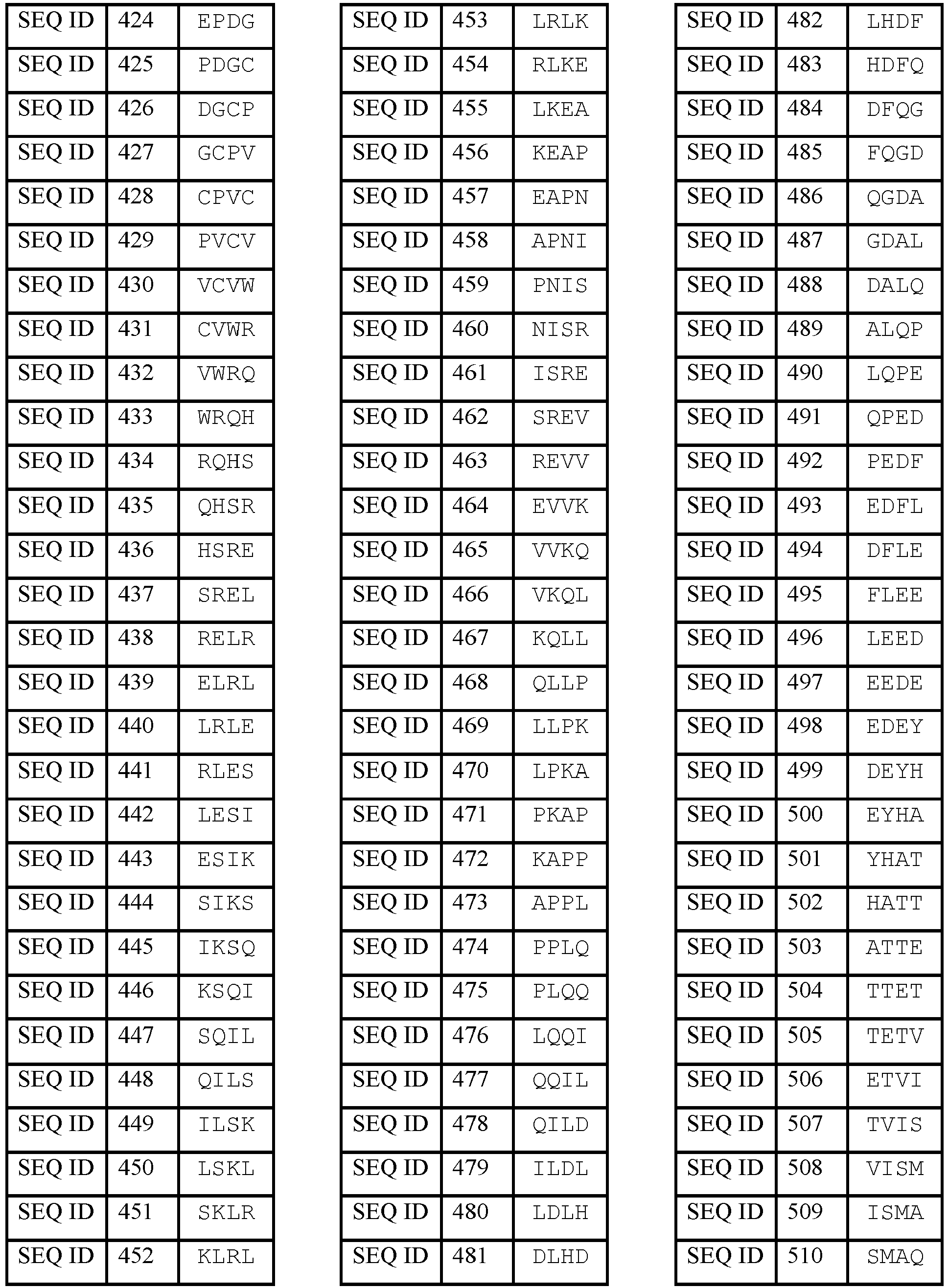

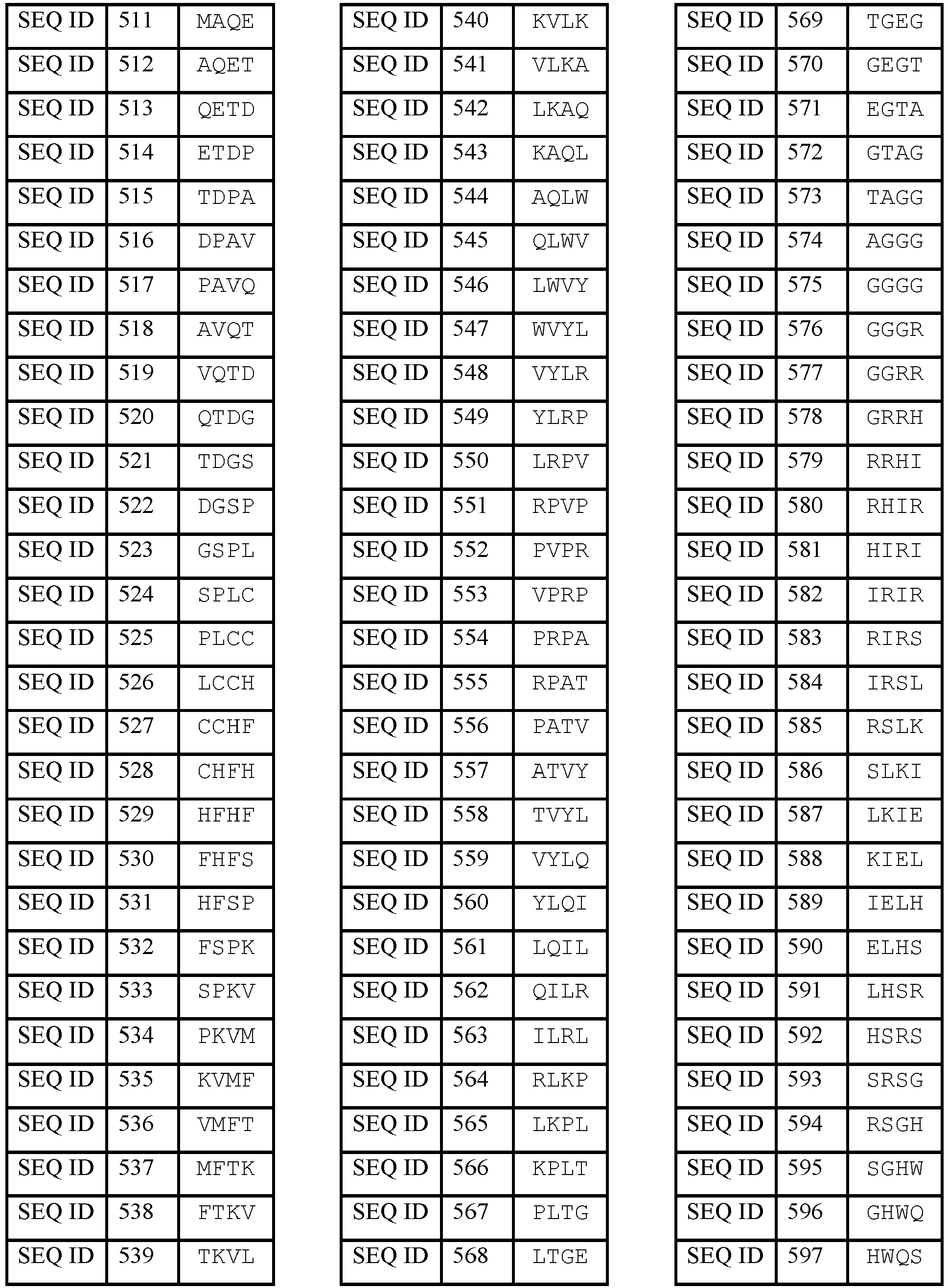

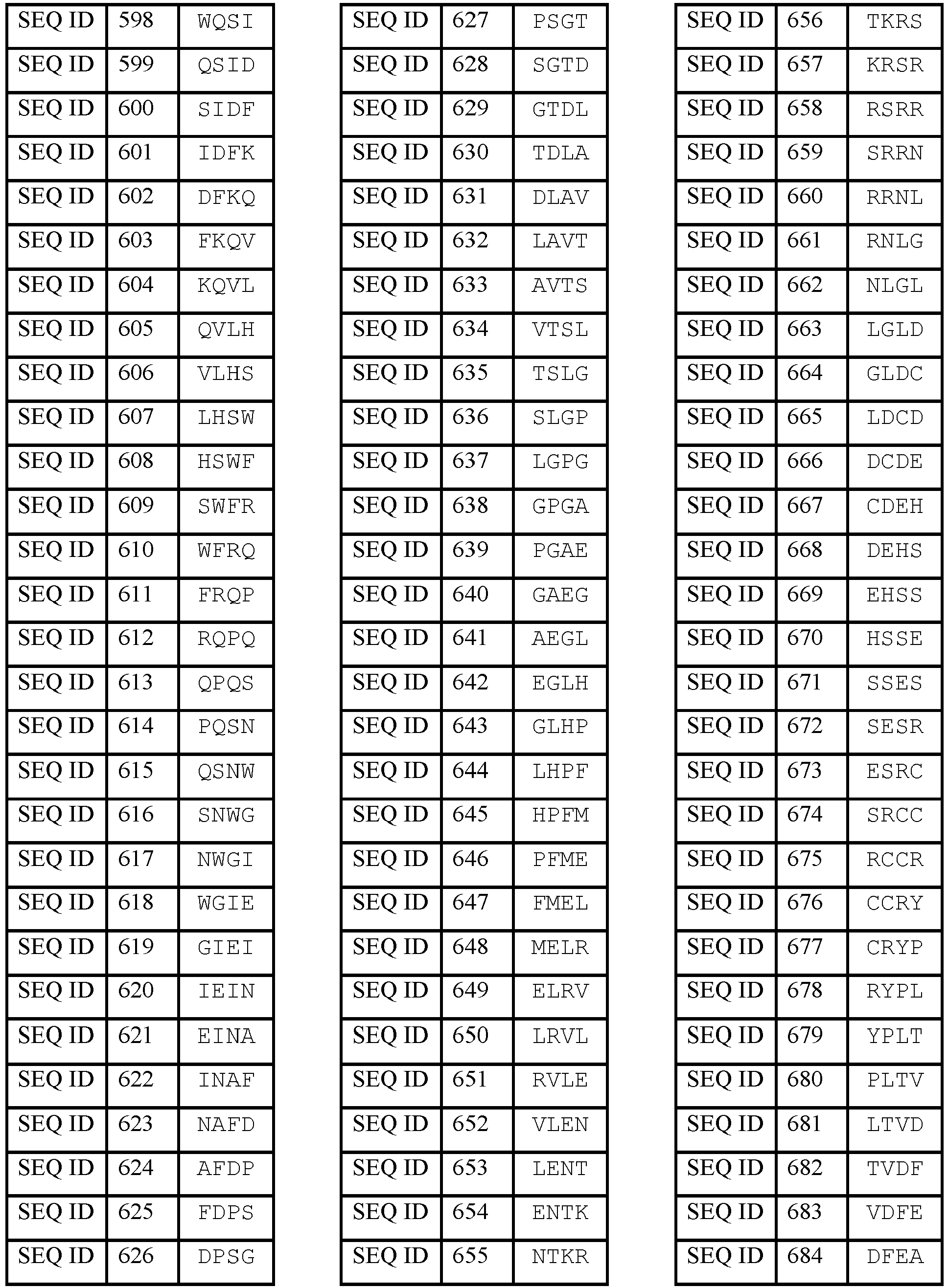

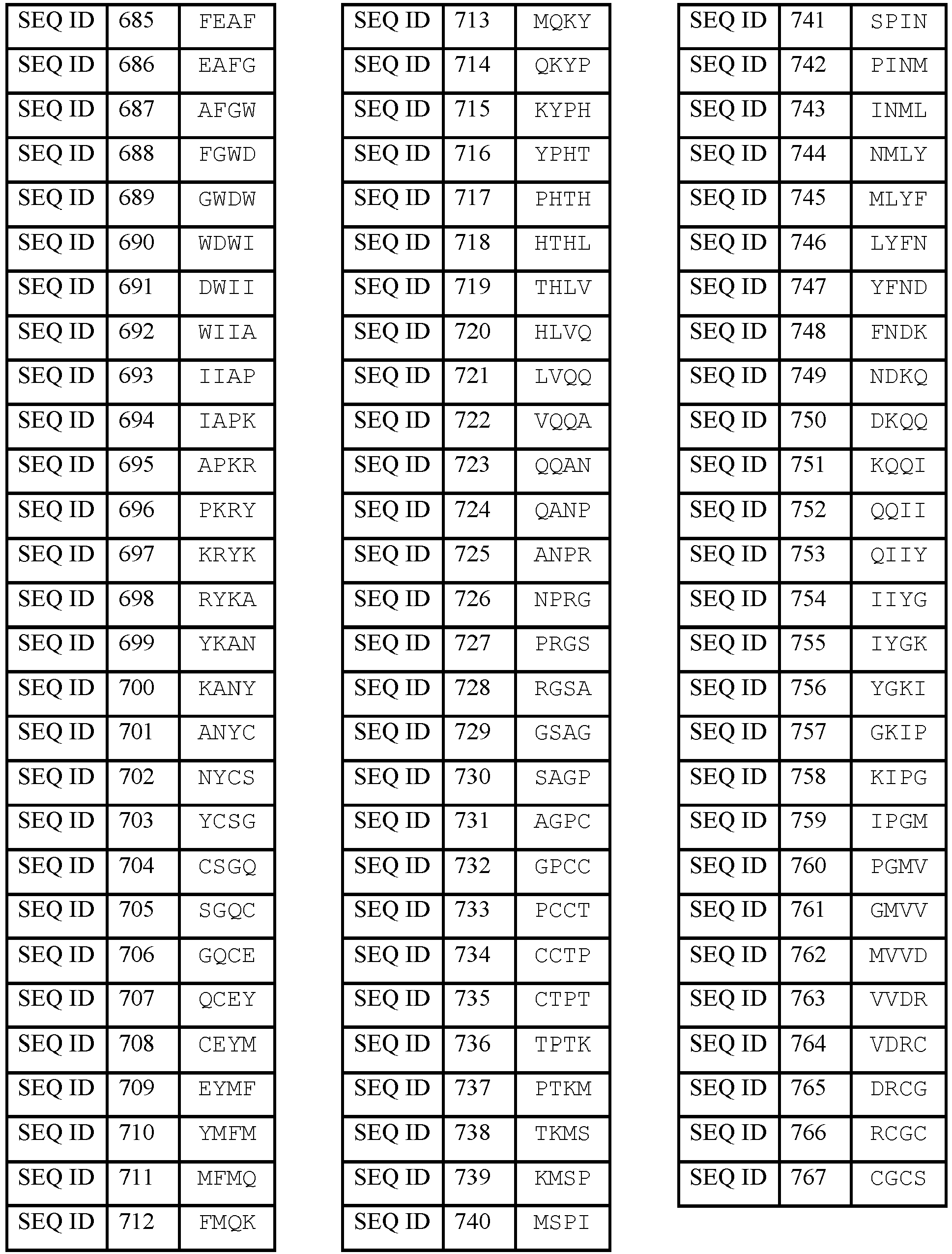

In some embodiments, the active agent comprise a peptide which further comprises any one or more of the 5-mer amino acid sequences (SEQ ID NO: 768-1161) listed in Table 4.

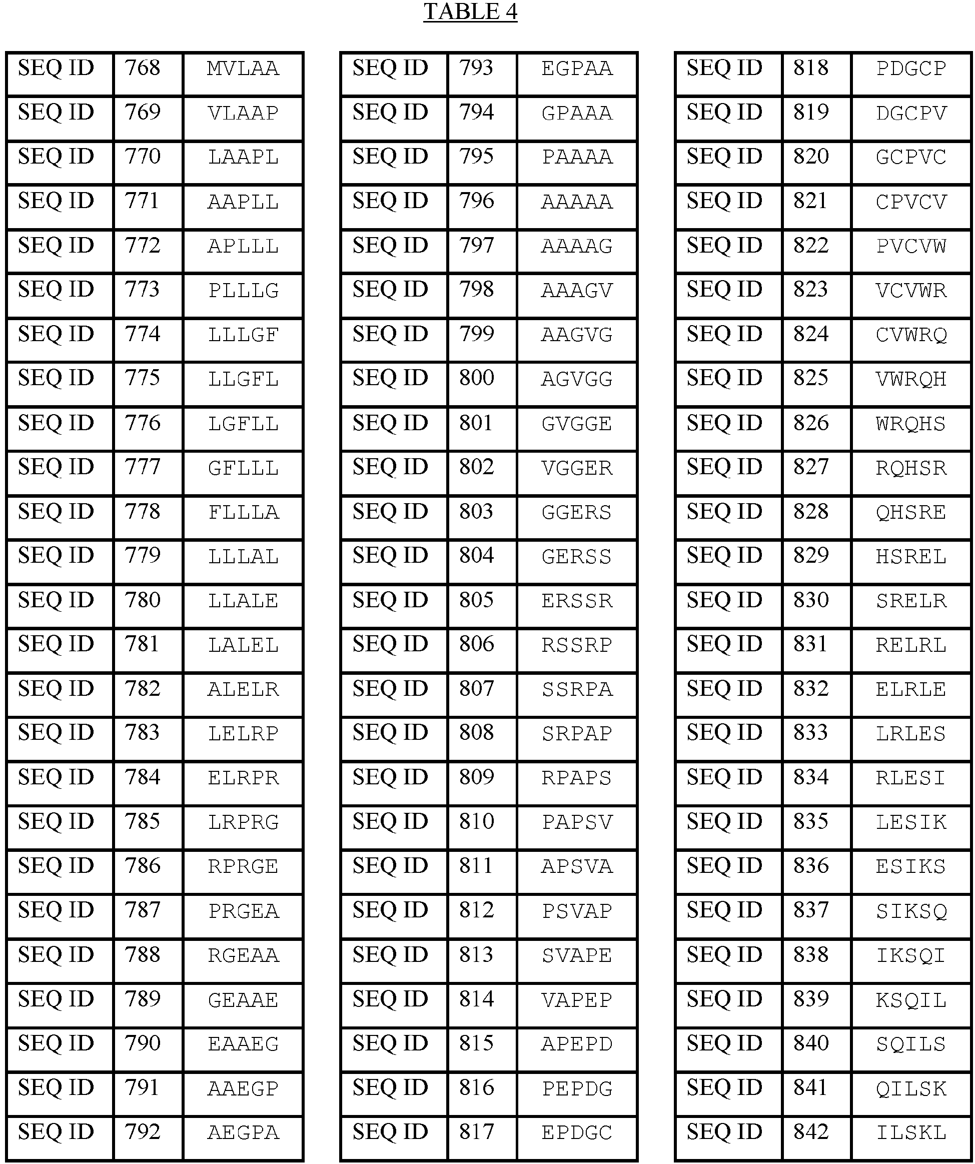

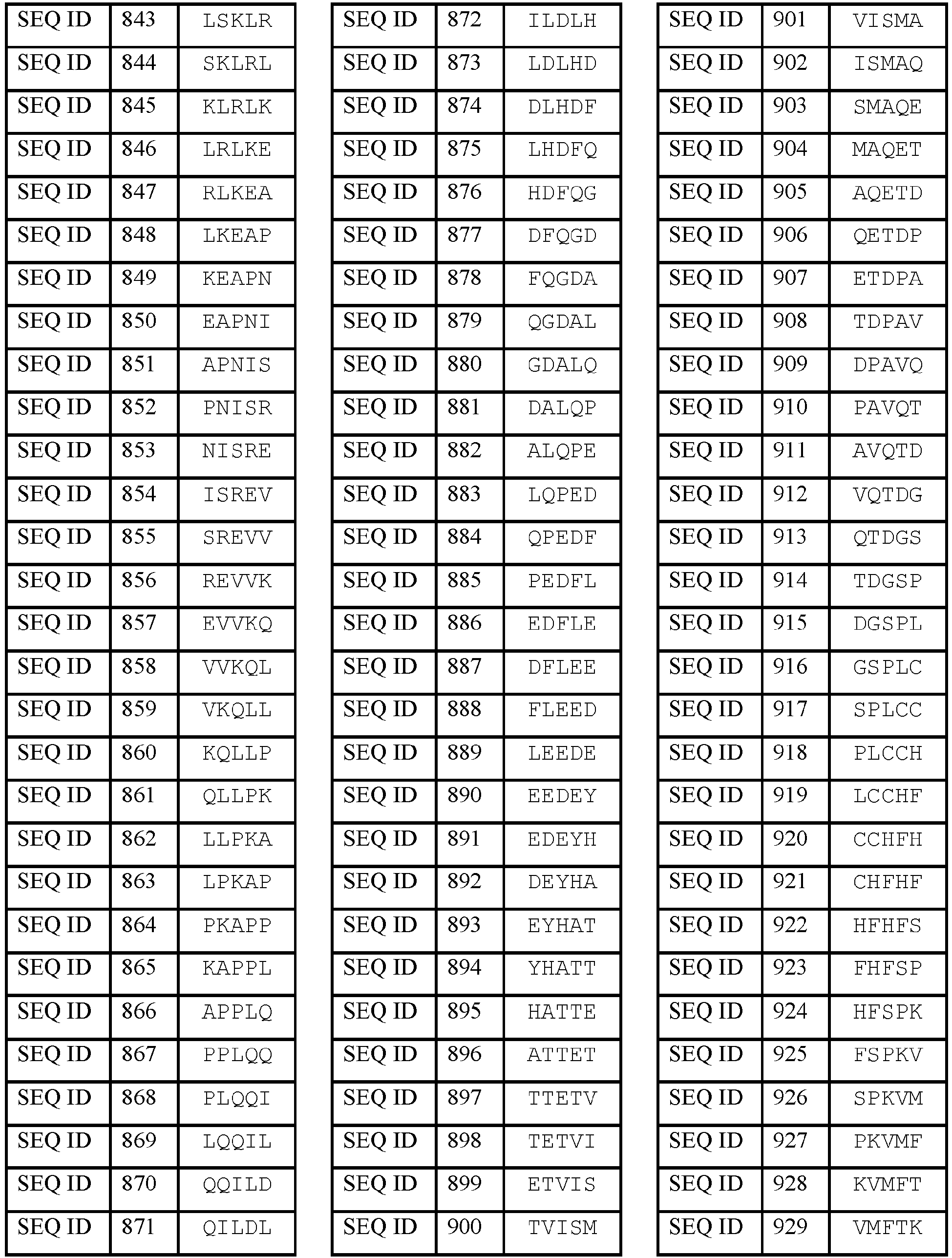

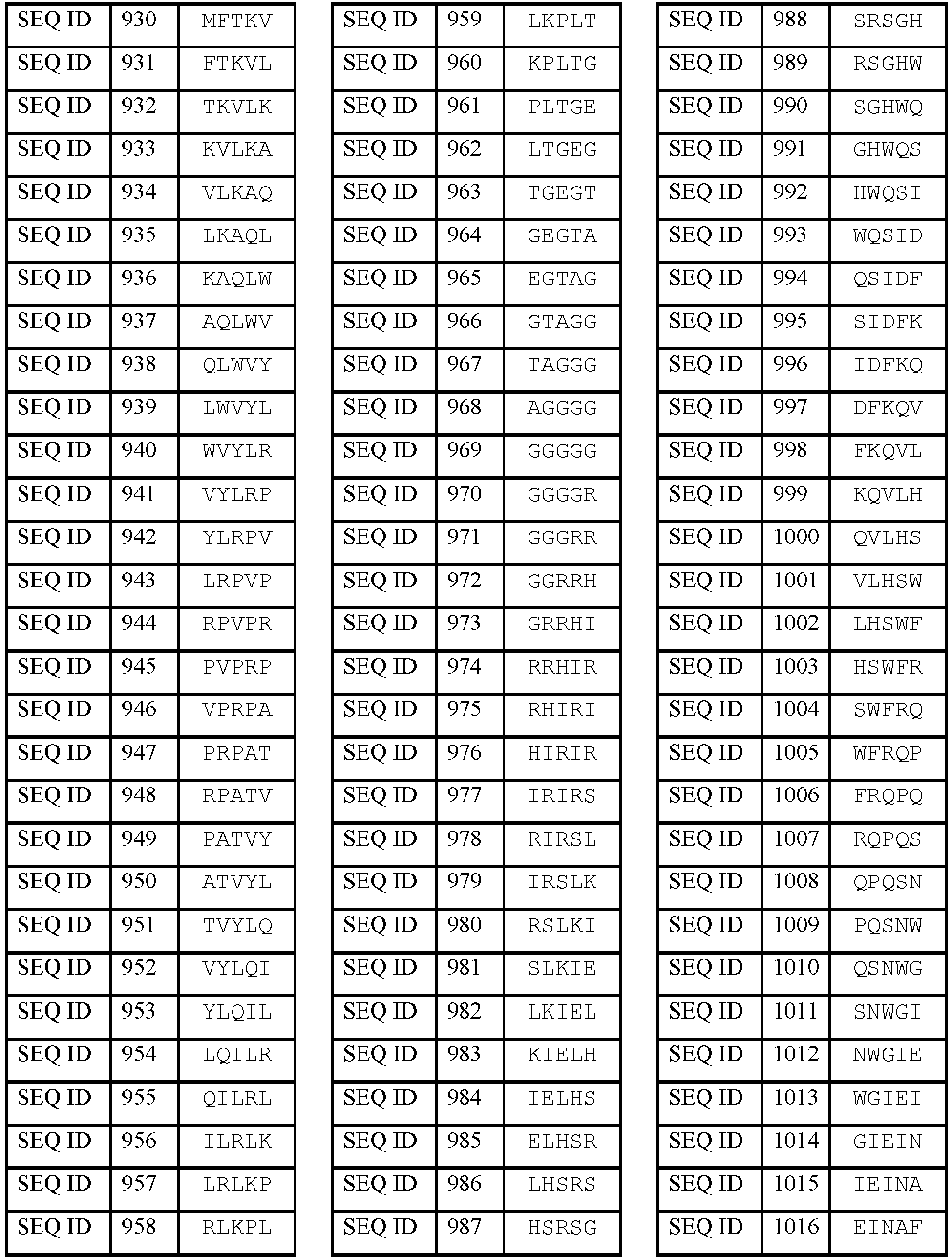

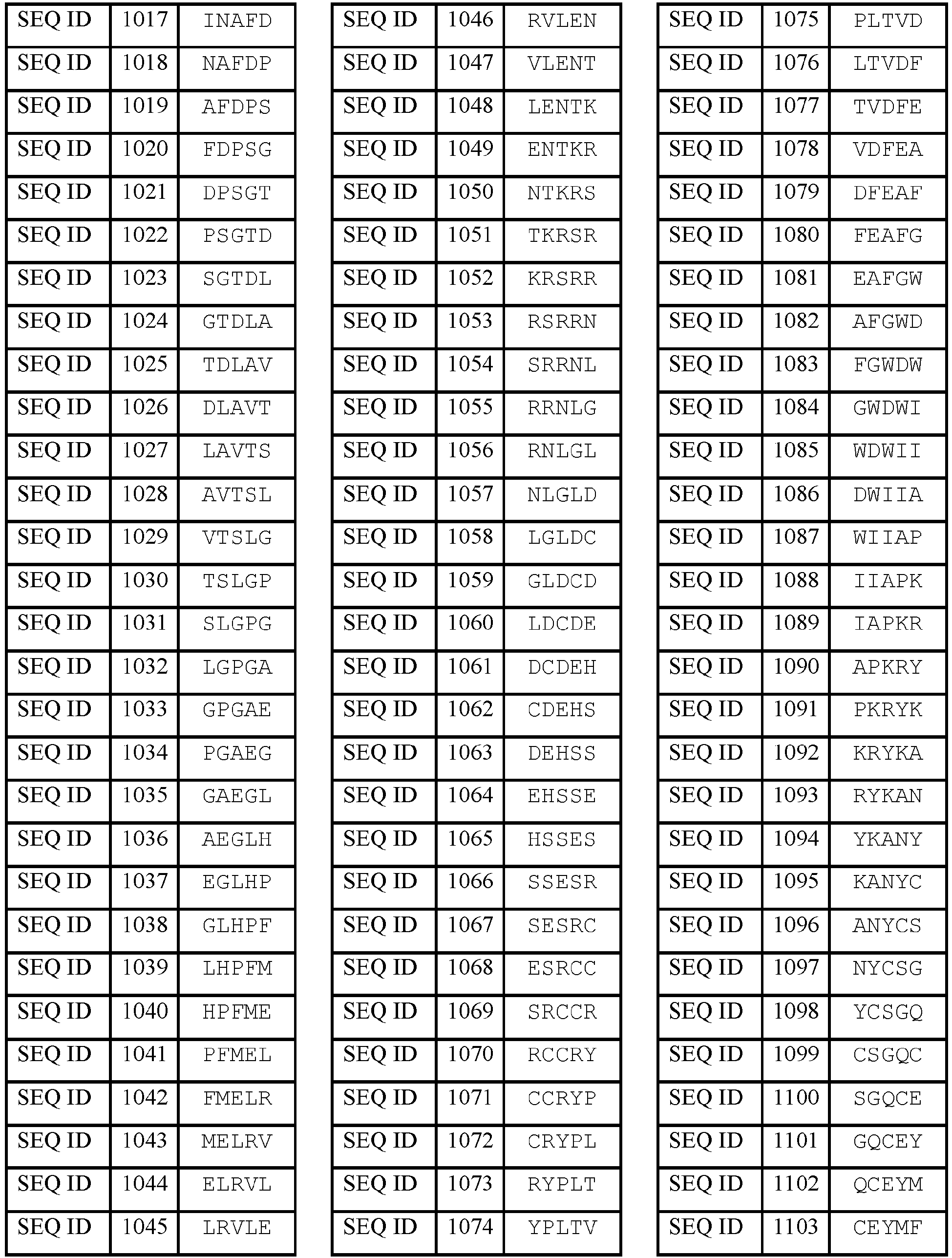

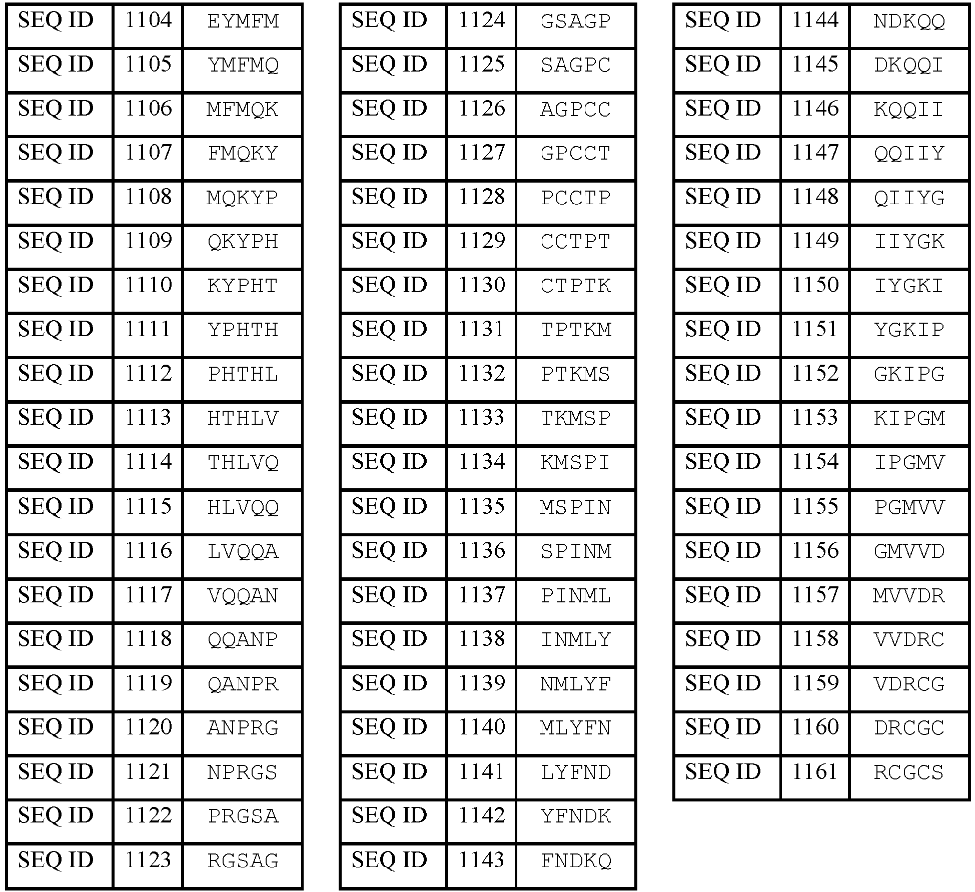

In some embodiments, the active agent comprises a peptide which further comprises any one or more of the 6-mer amino acid sequences (SEQ ID NO: 1162-1556) listed in Table 5.

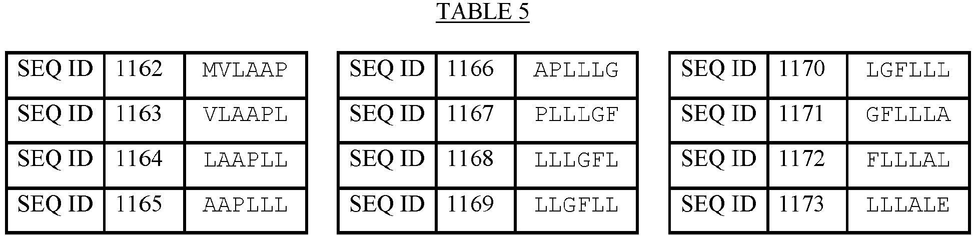

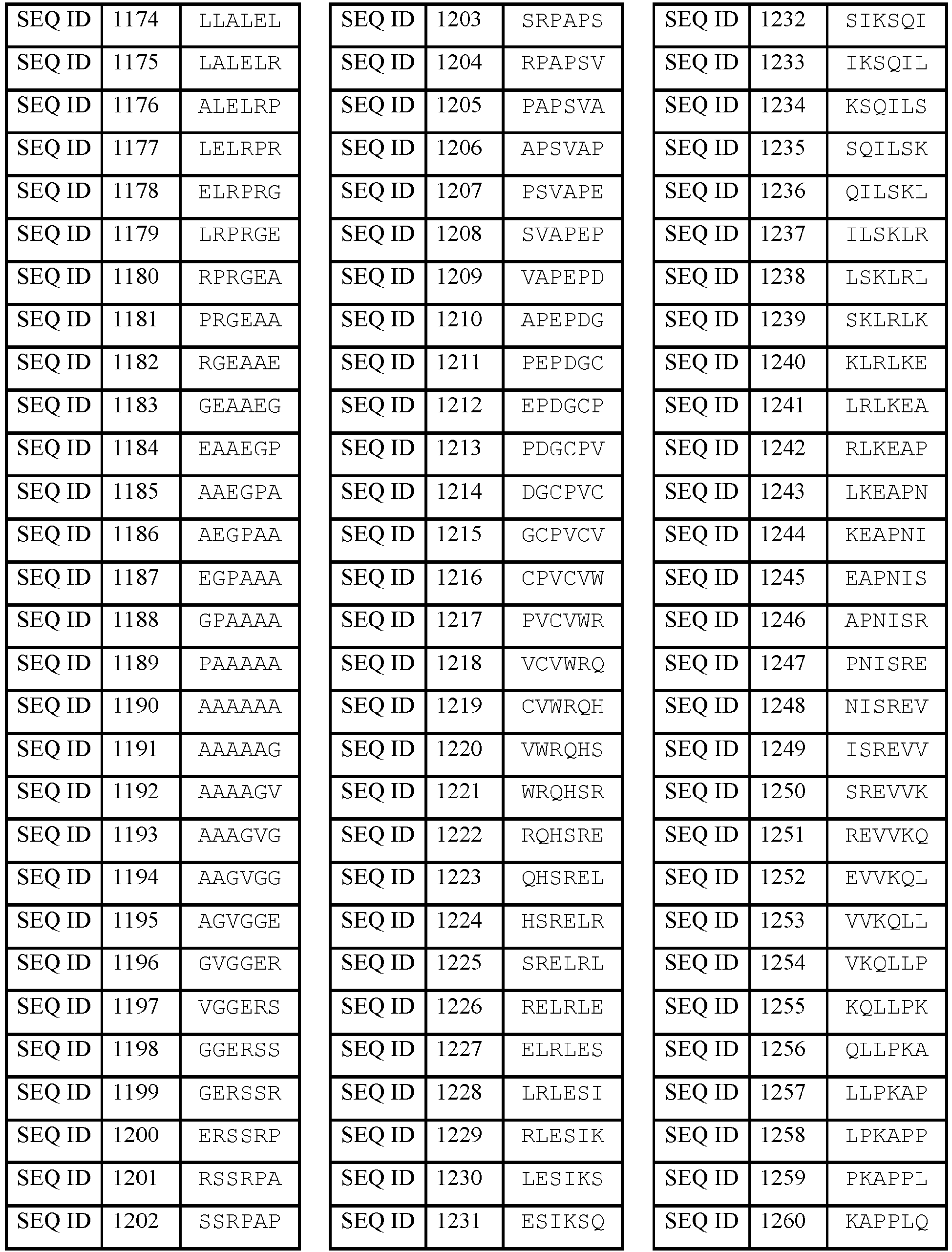

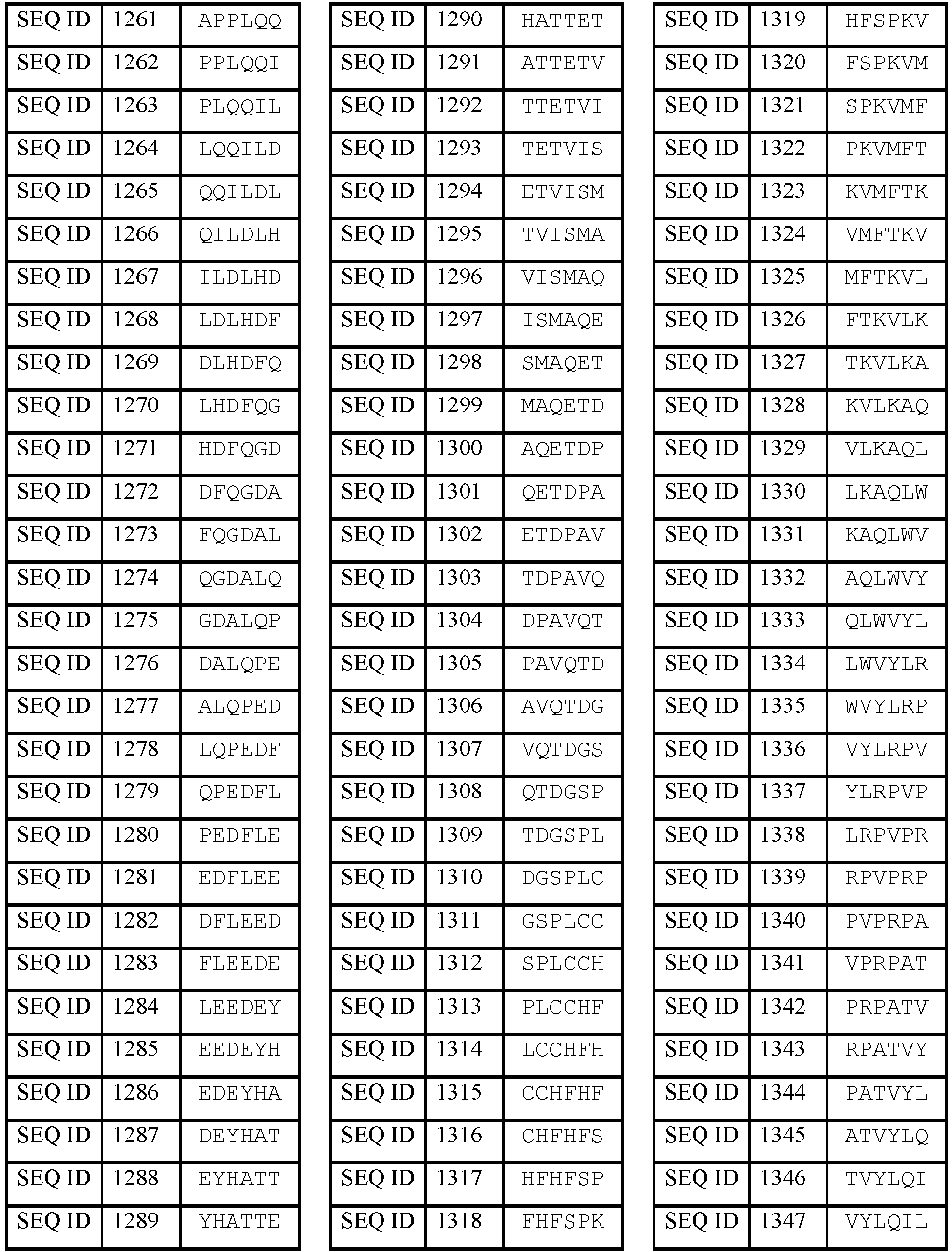

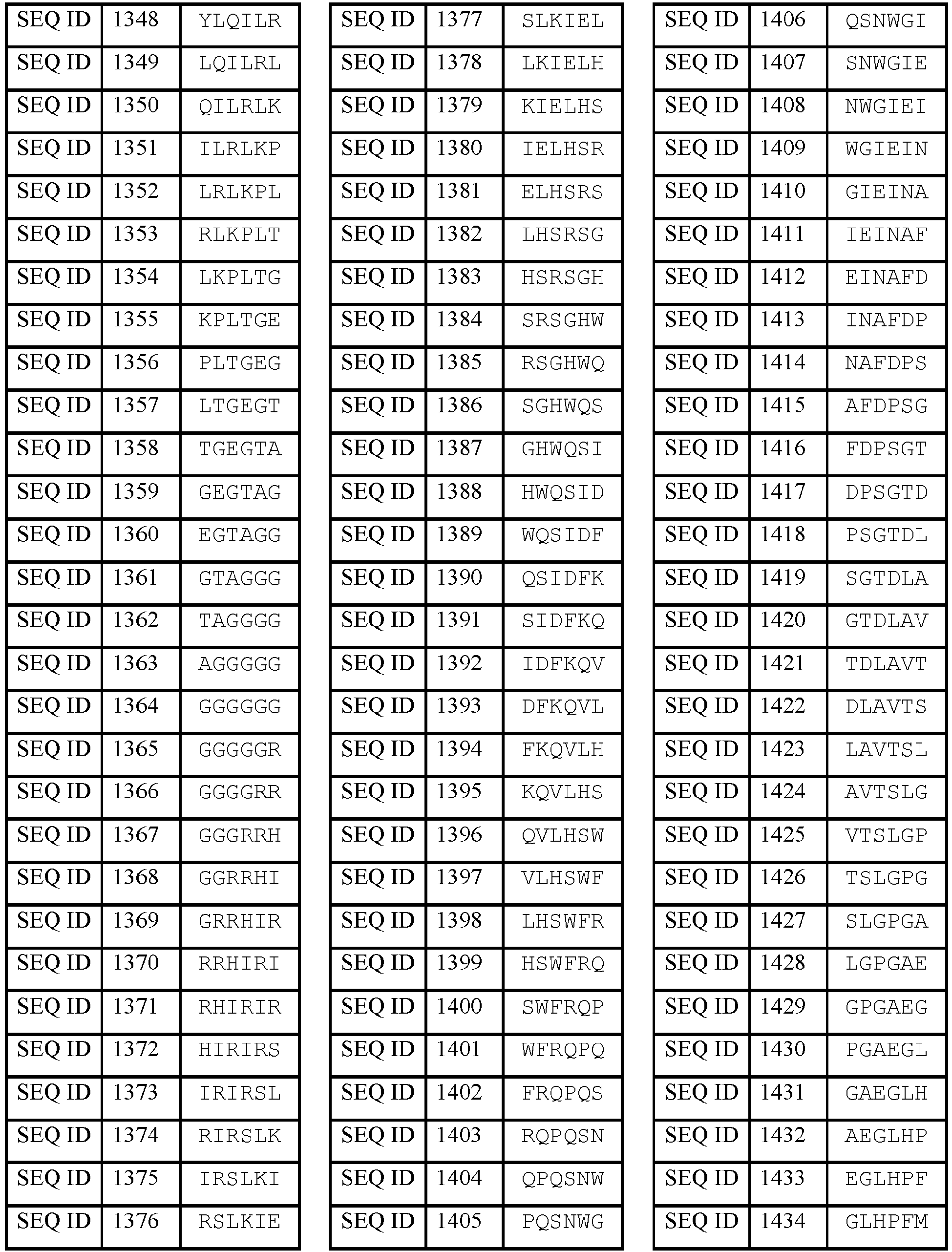

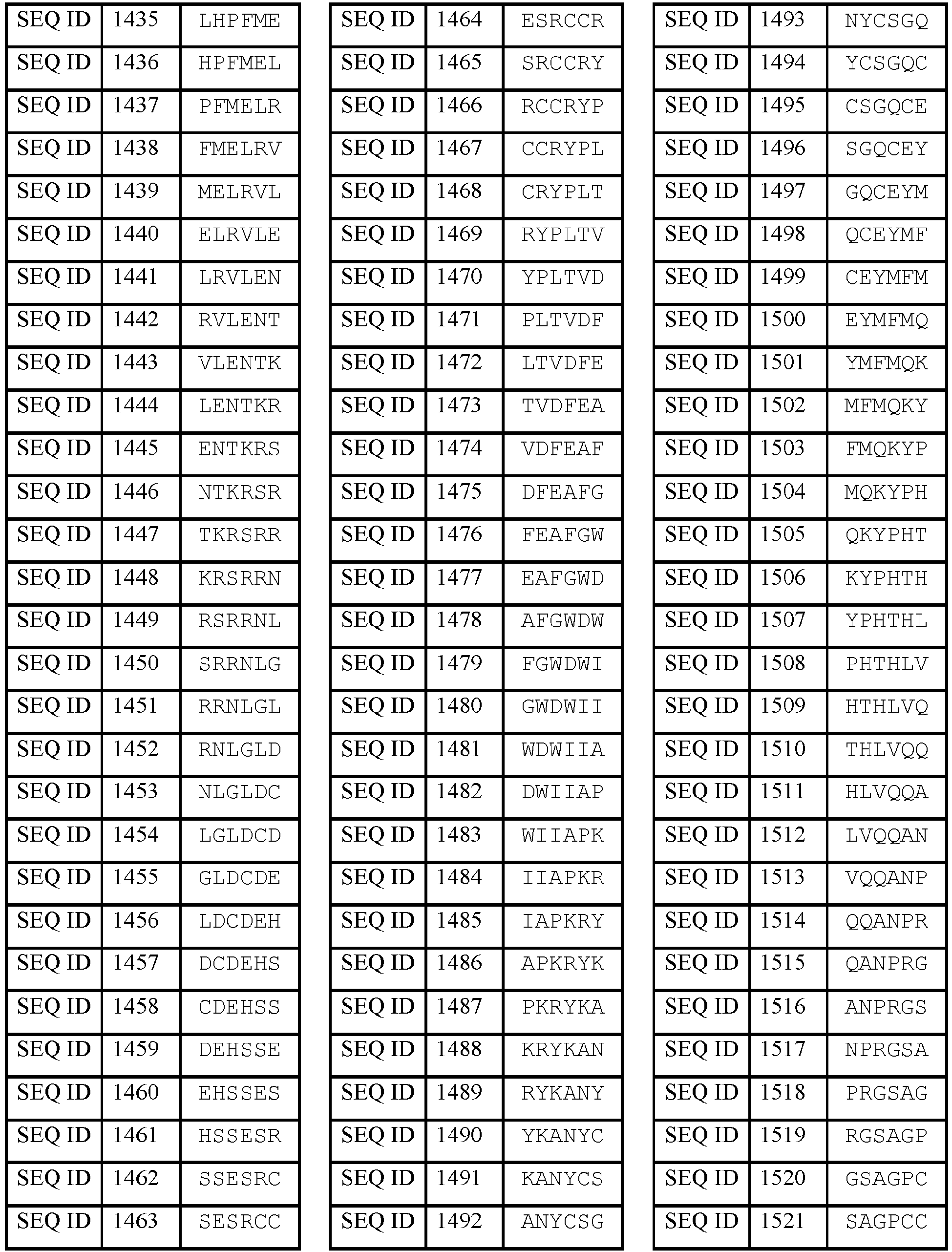

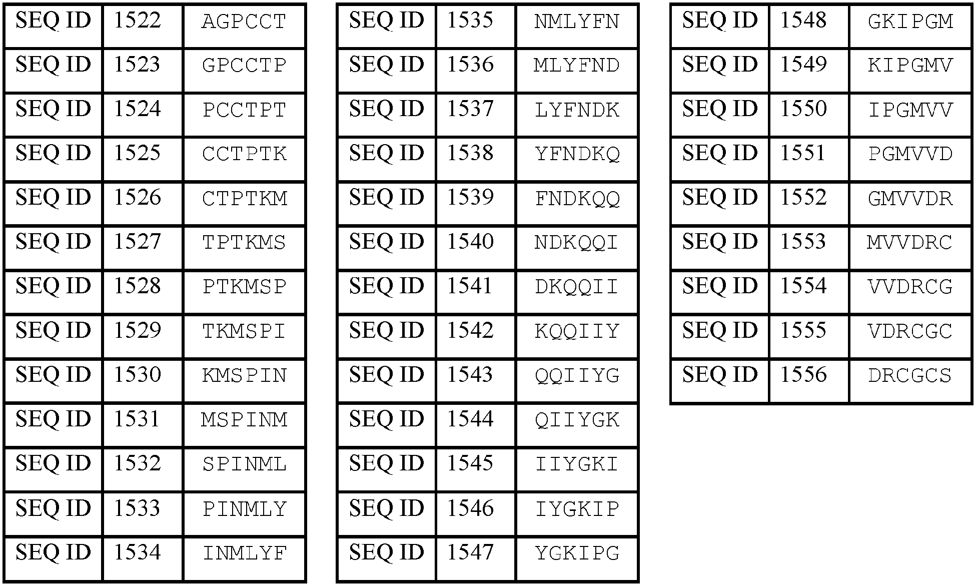

In some embodiments, the active agent comprises a peptide which further comprises any one or more of the 7-mer amino acid sequences (SEQ ID NO: 1557-1951) listed in Table 6.

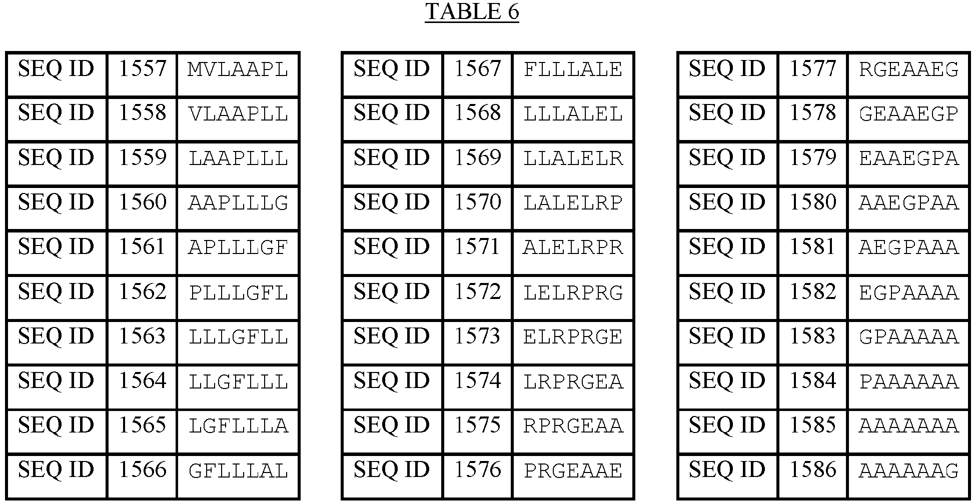

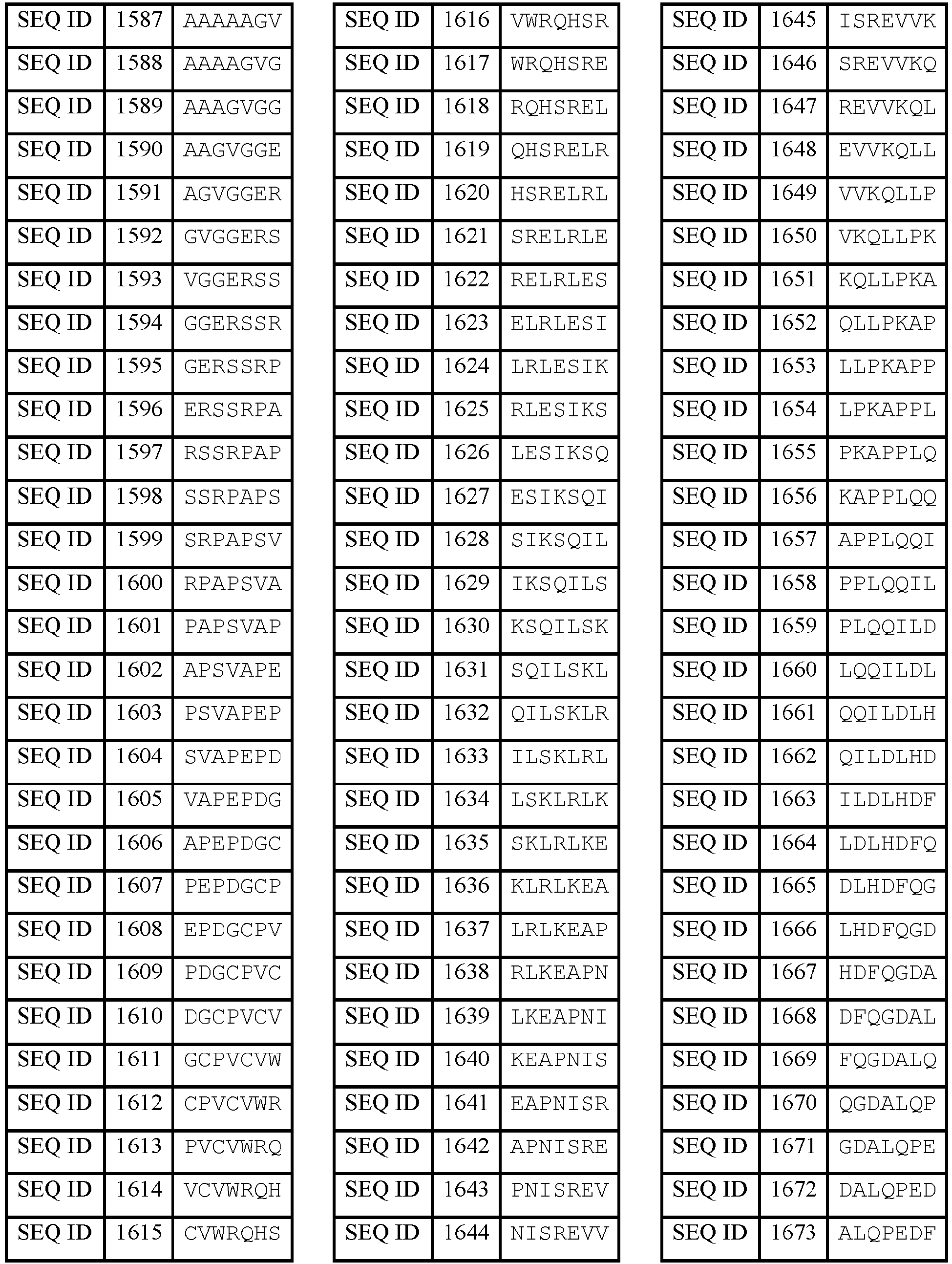

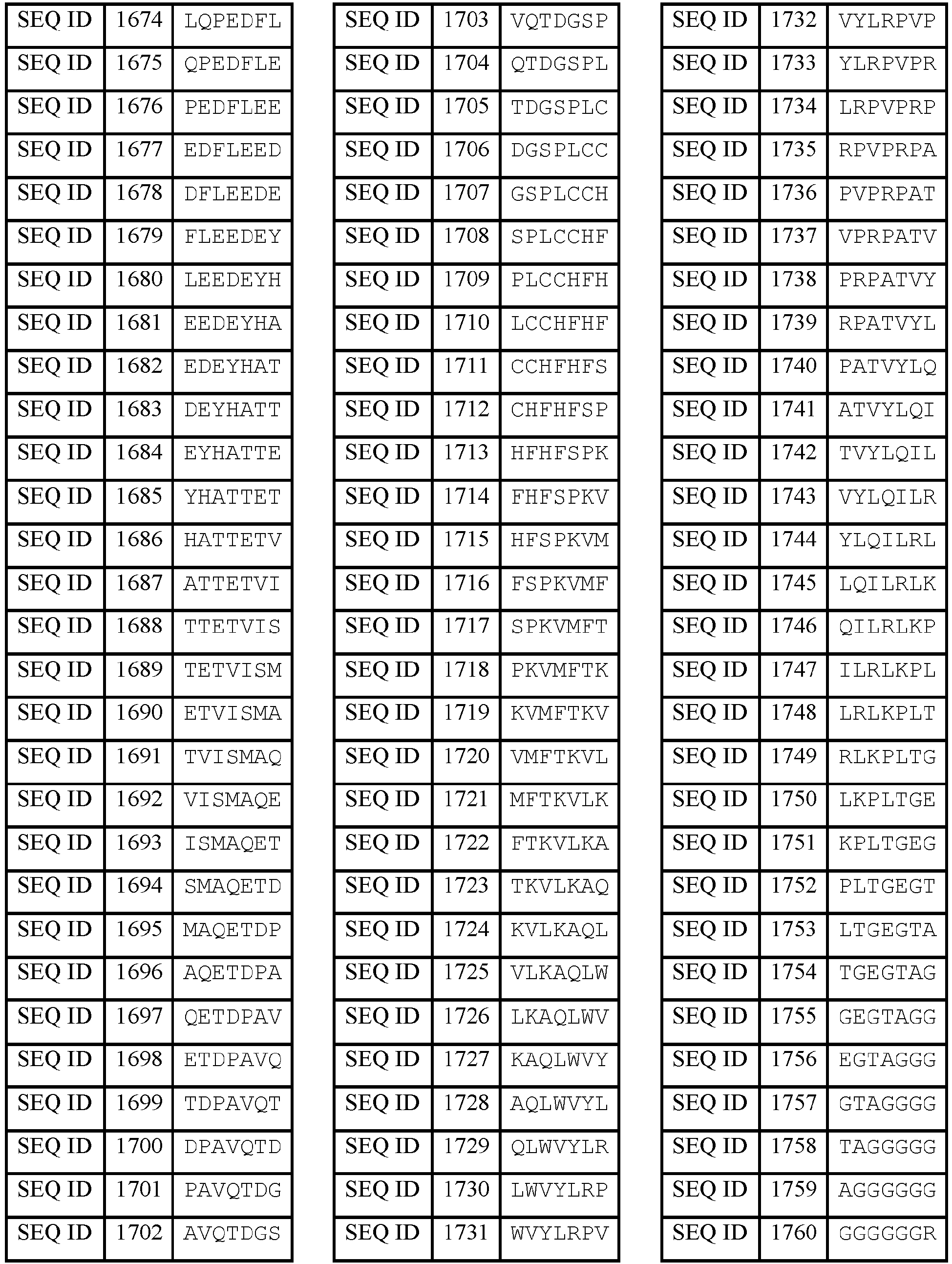

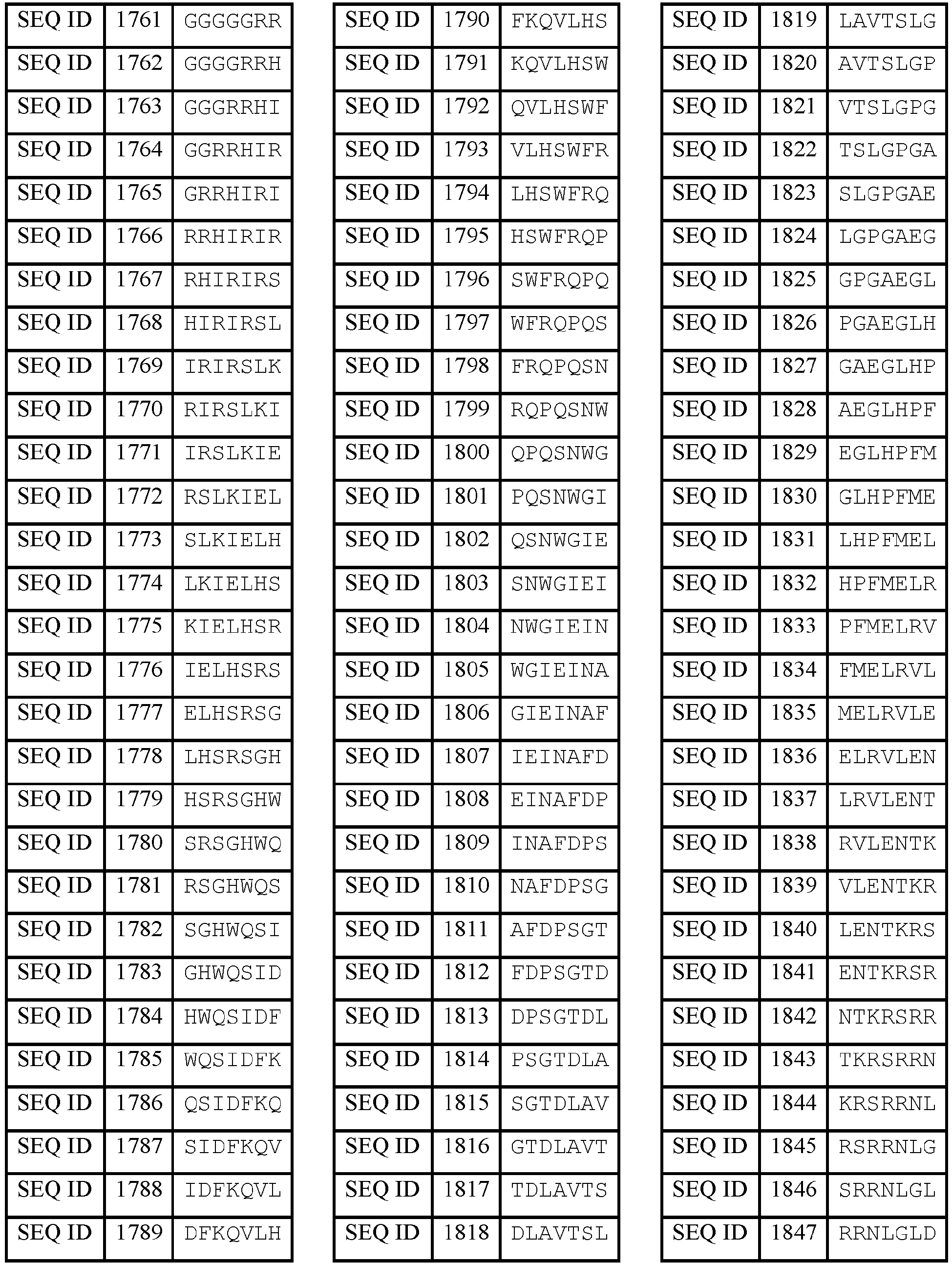

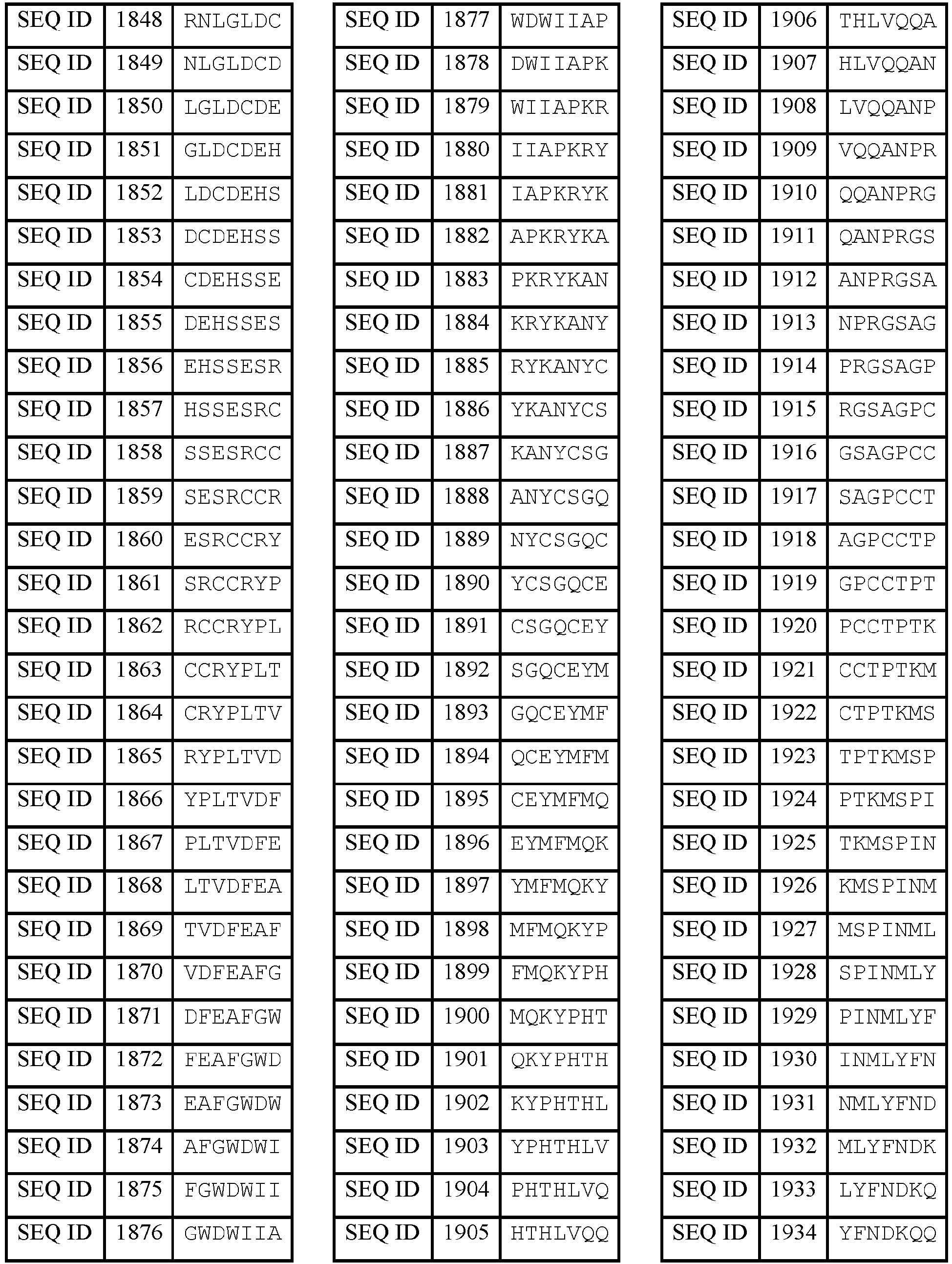

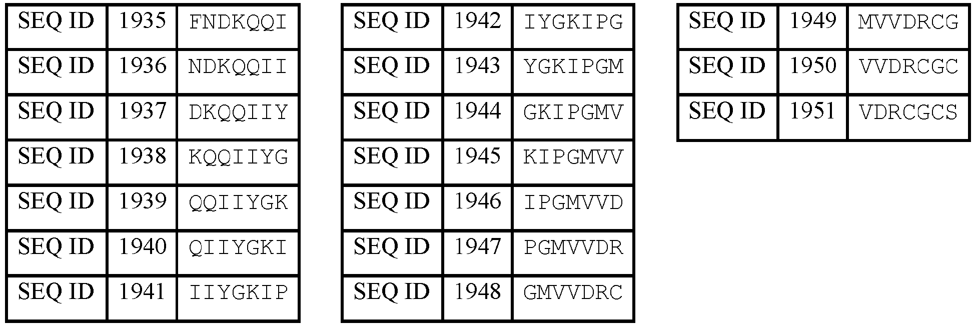

In some embodiments, the active agent comprises a peptide which further comprises any one or more of the 8-mer amino acid sequences (SEQ ID NO: 1952-2346) listed in Table 7.

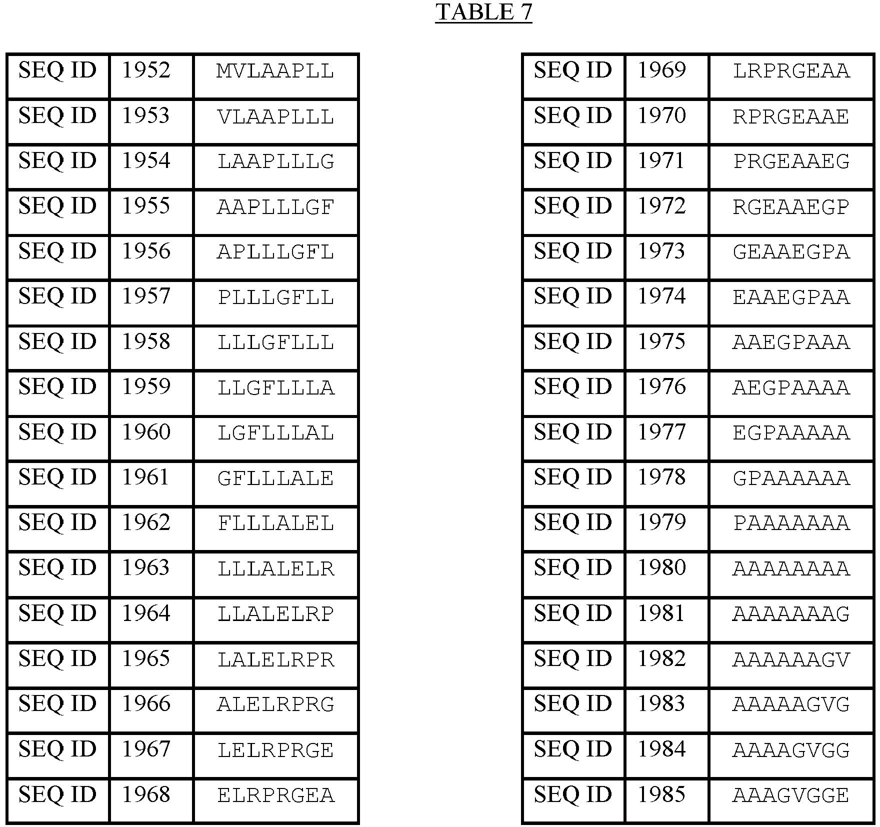

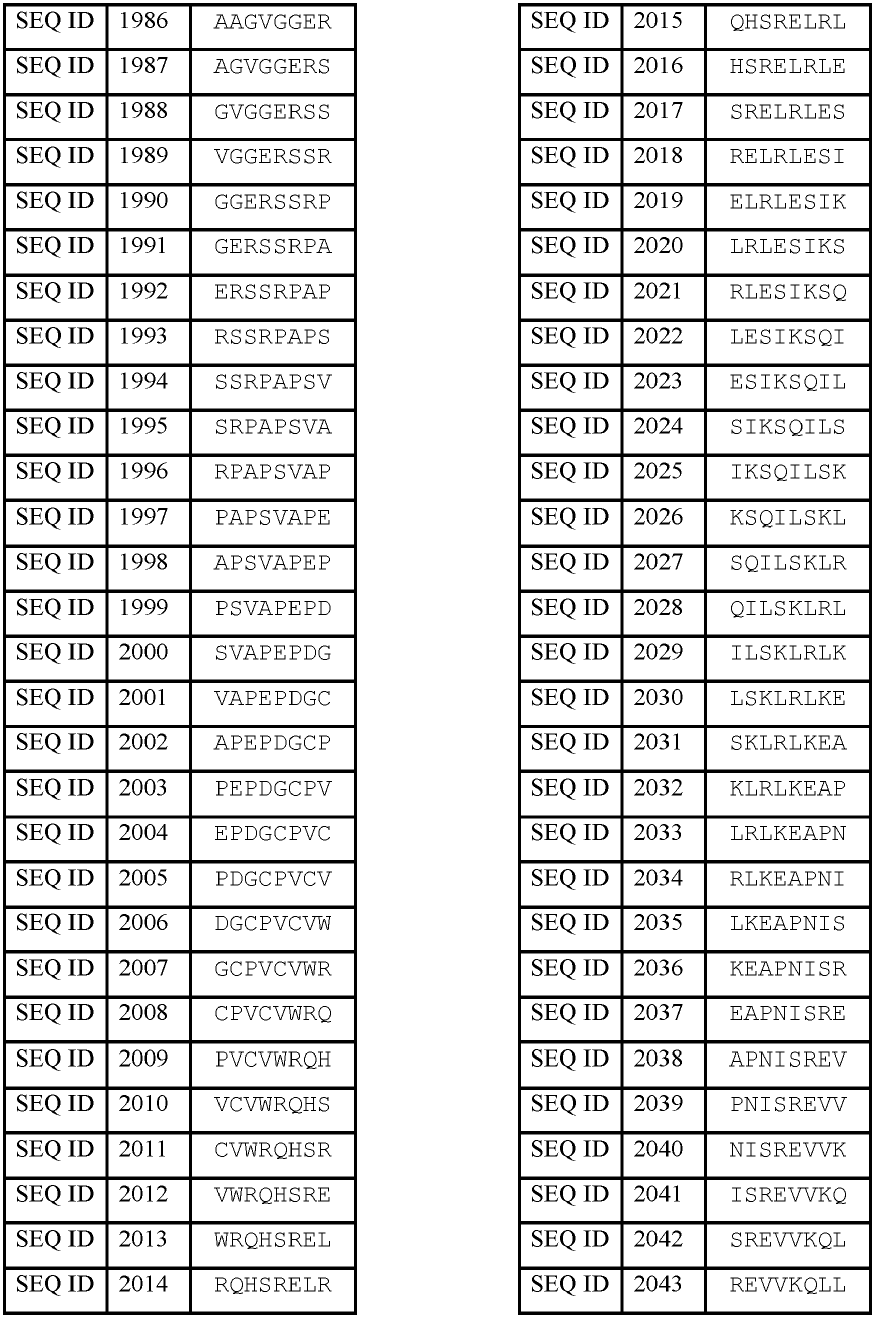

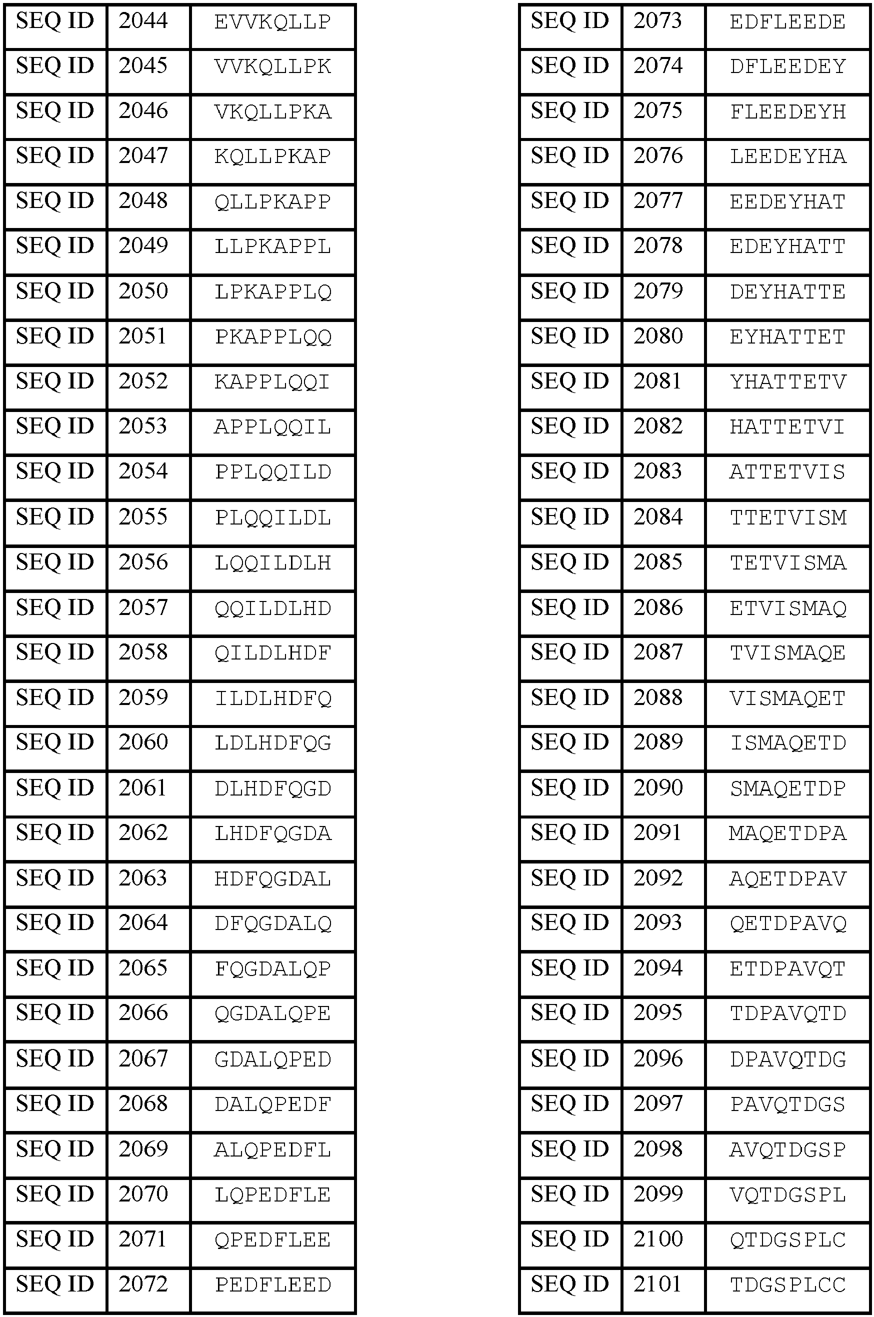

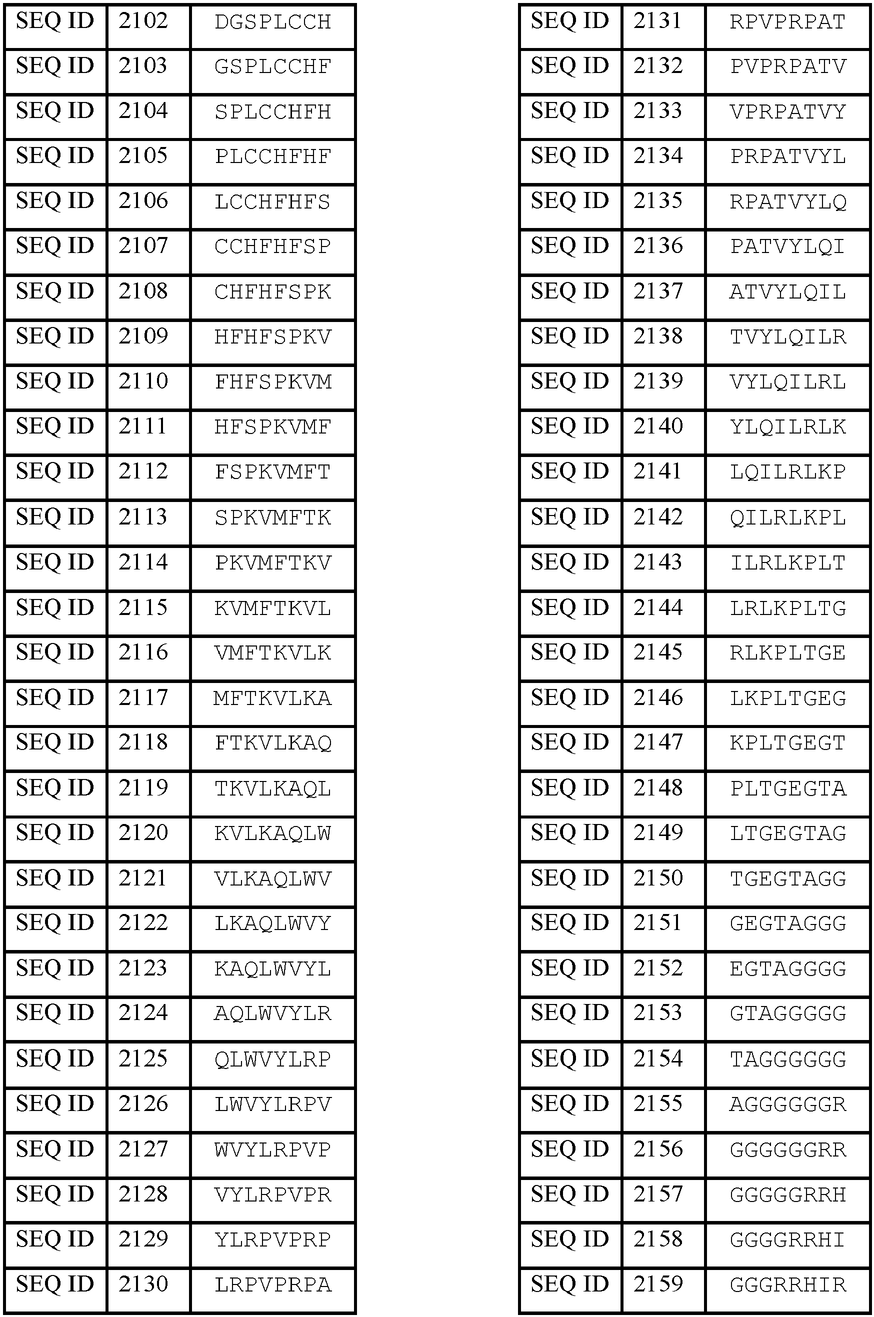

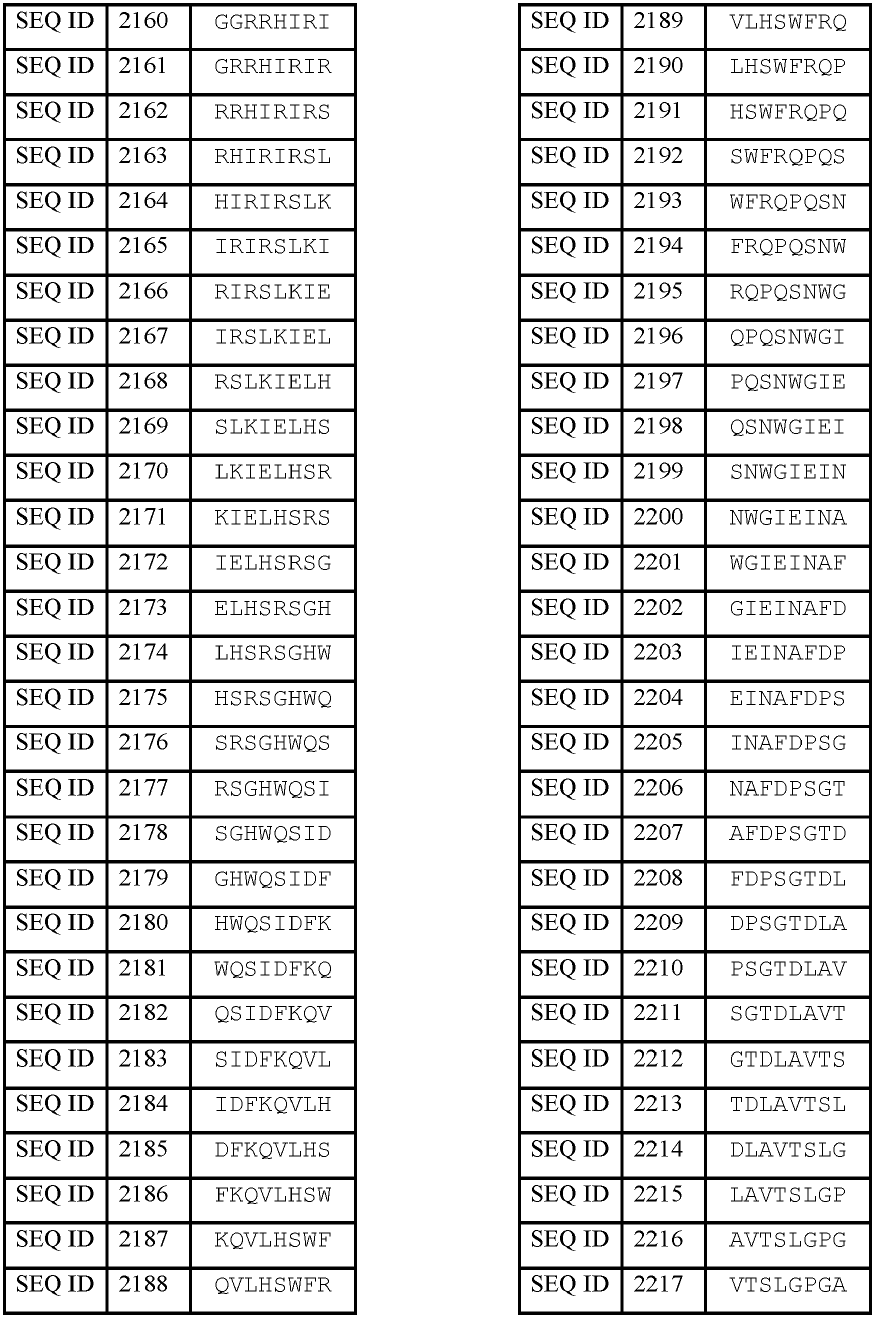

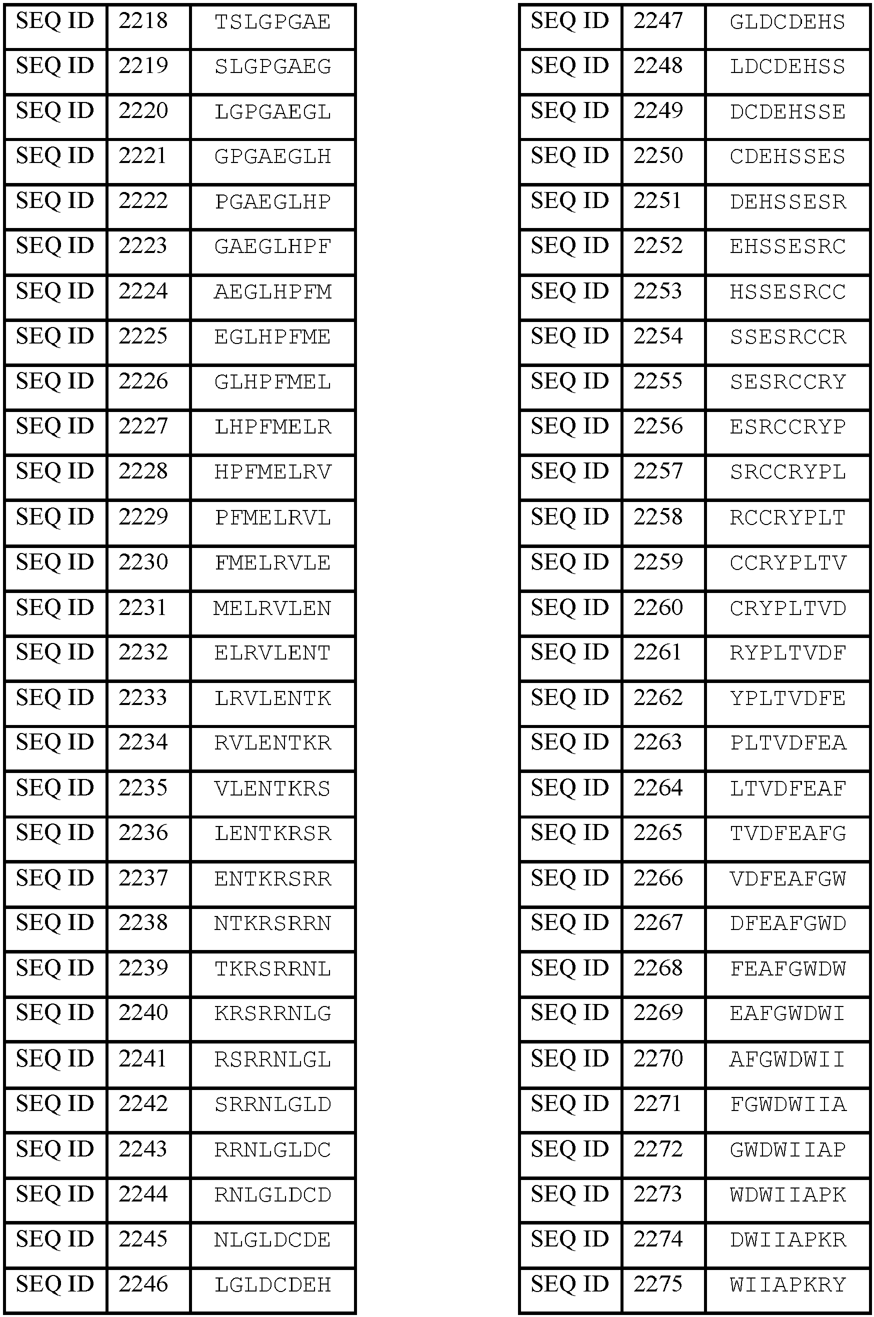

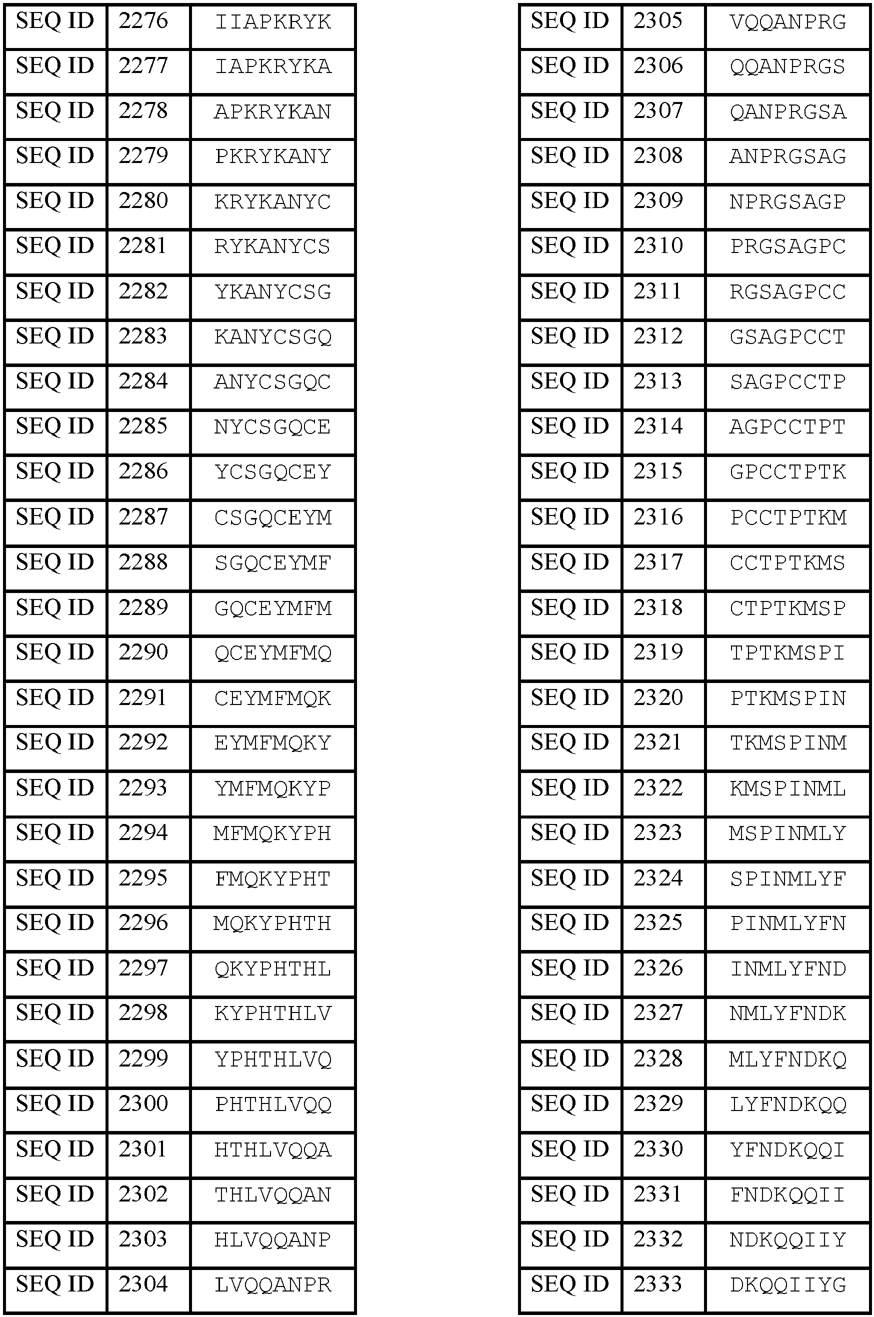

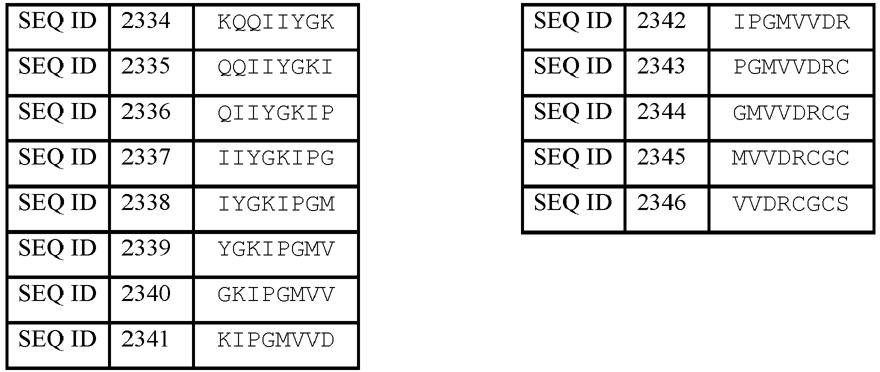

In some embodiments, the active agent comprises a peptide which further comprises any one or more of the 9-mer amino acid sequences (SEQ ID NO: 2347-2741) listed in Table 8.

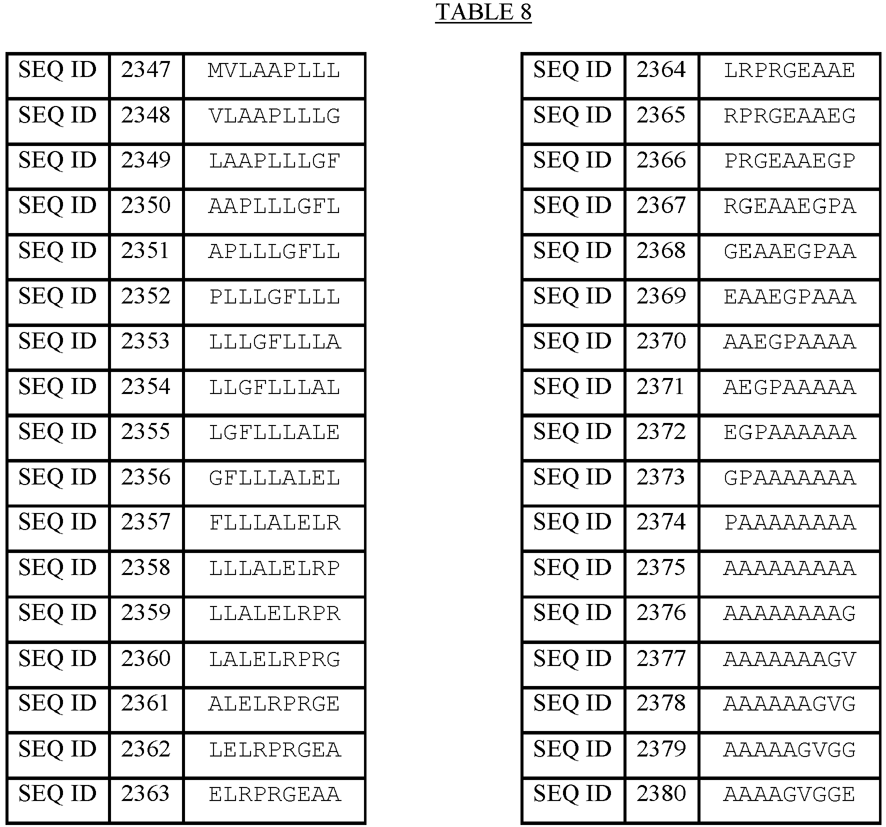

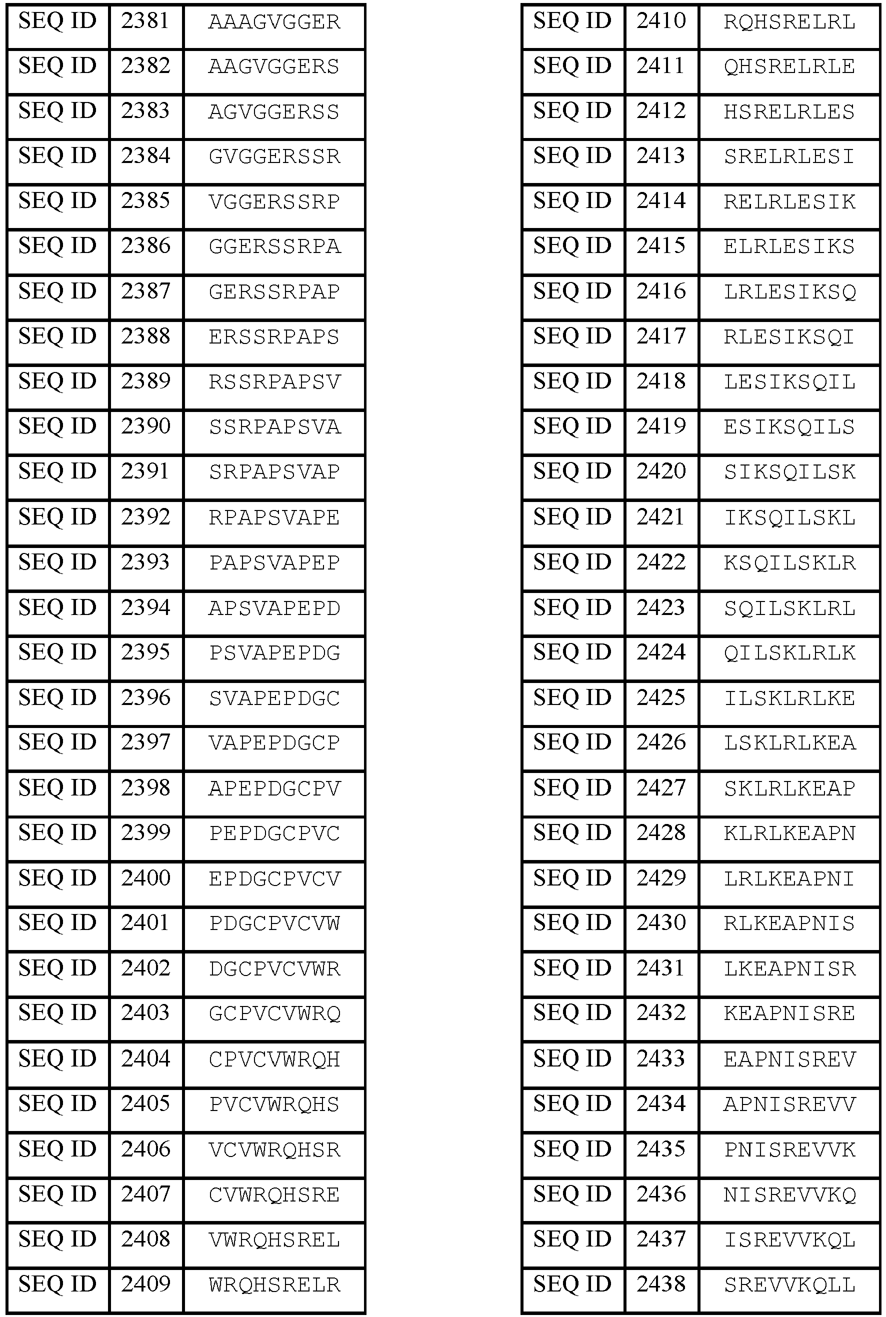

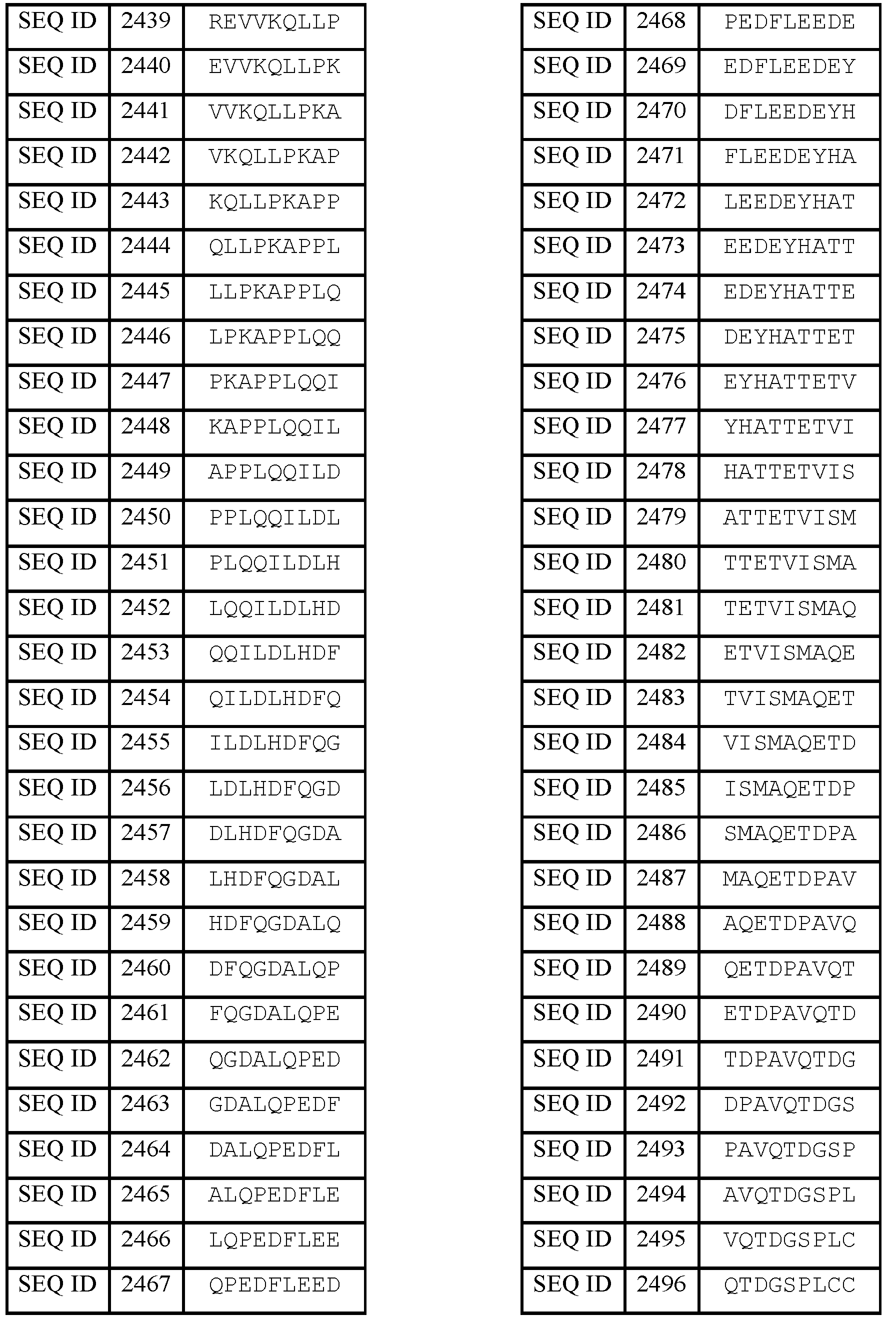

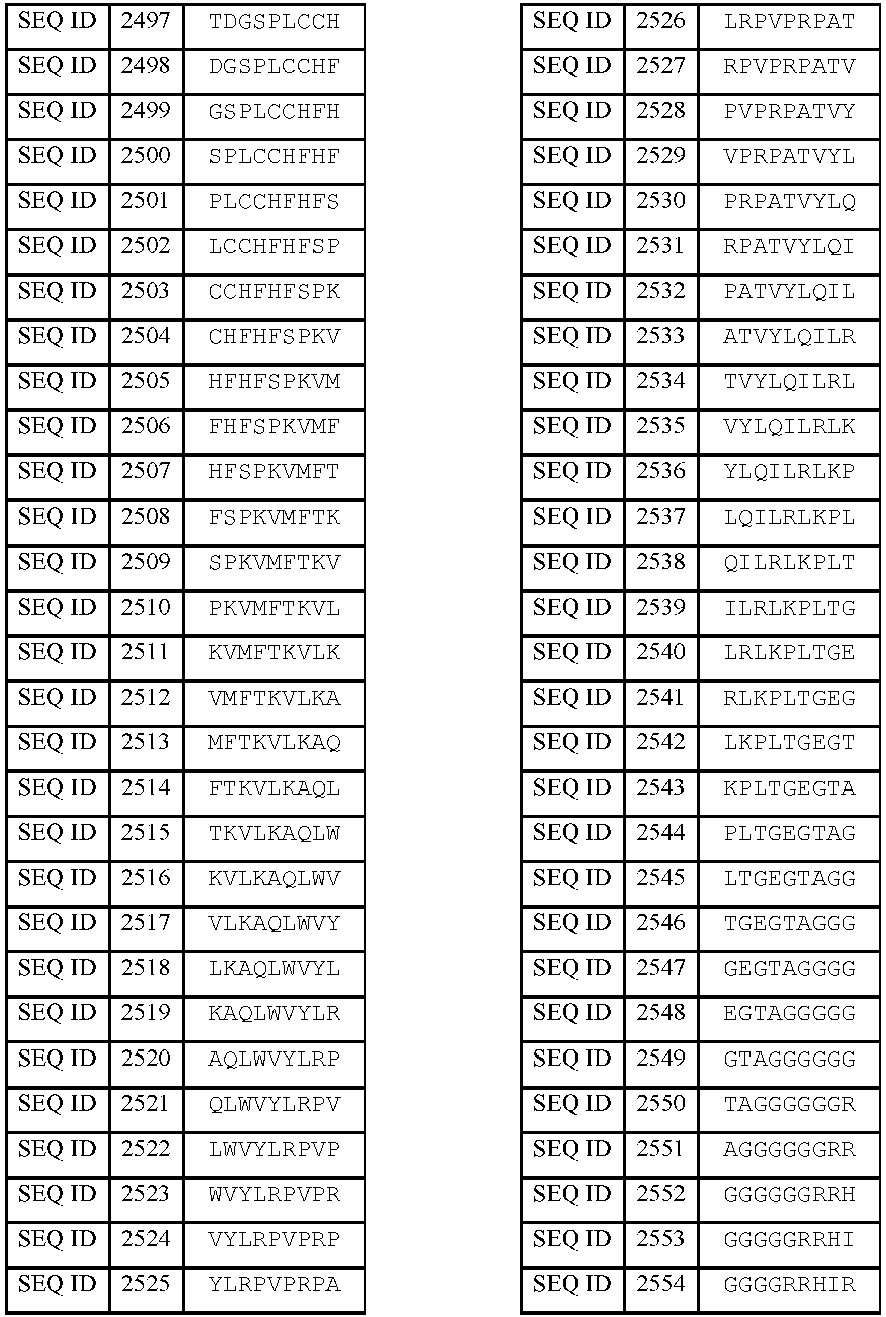

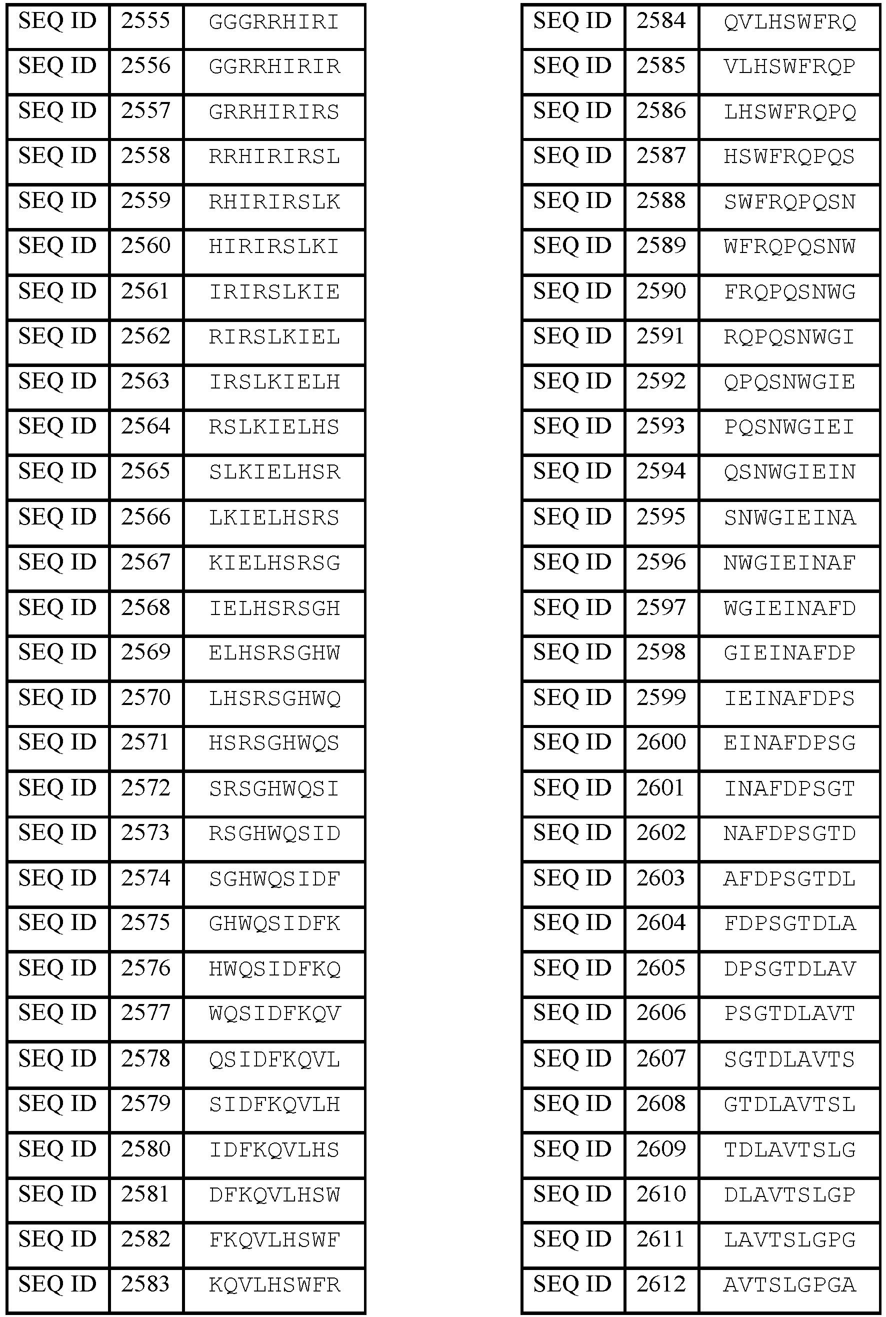

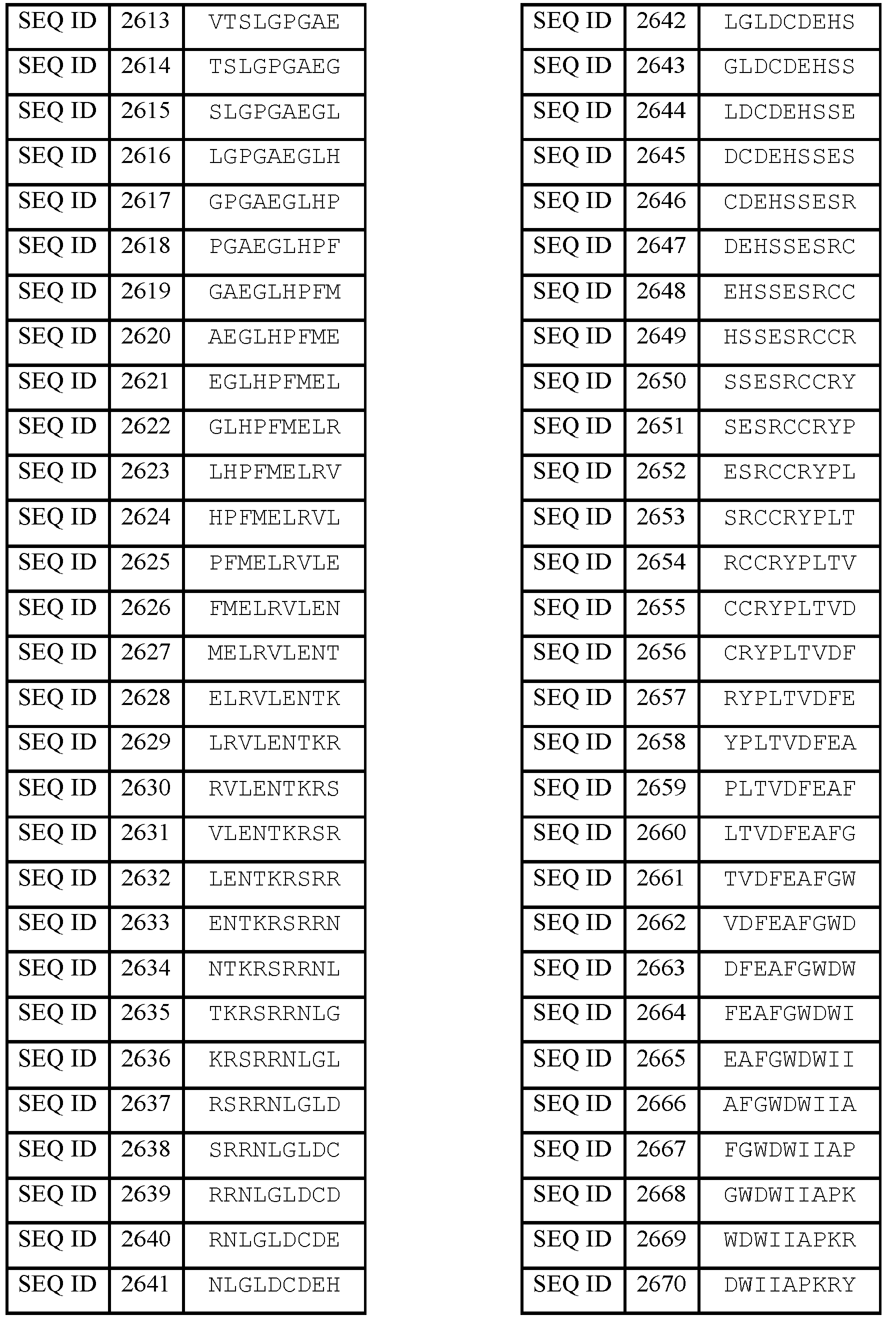

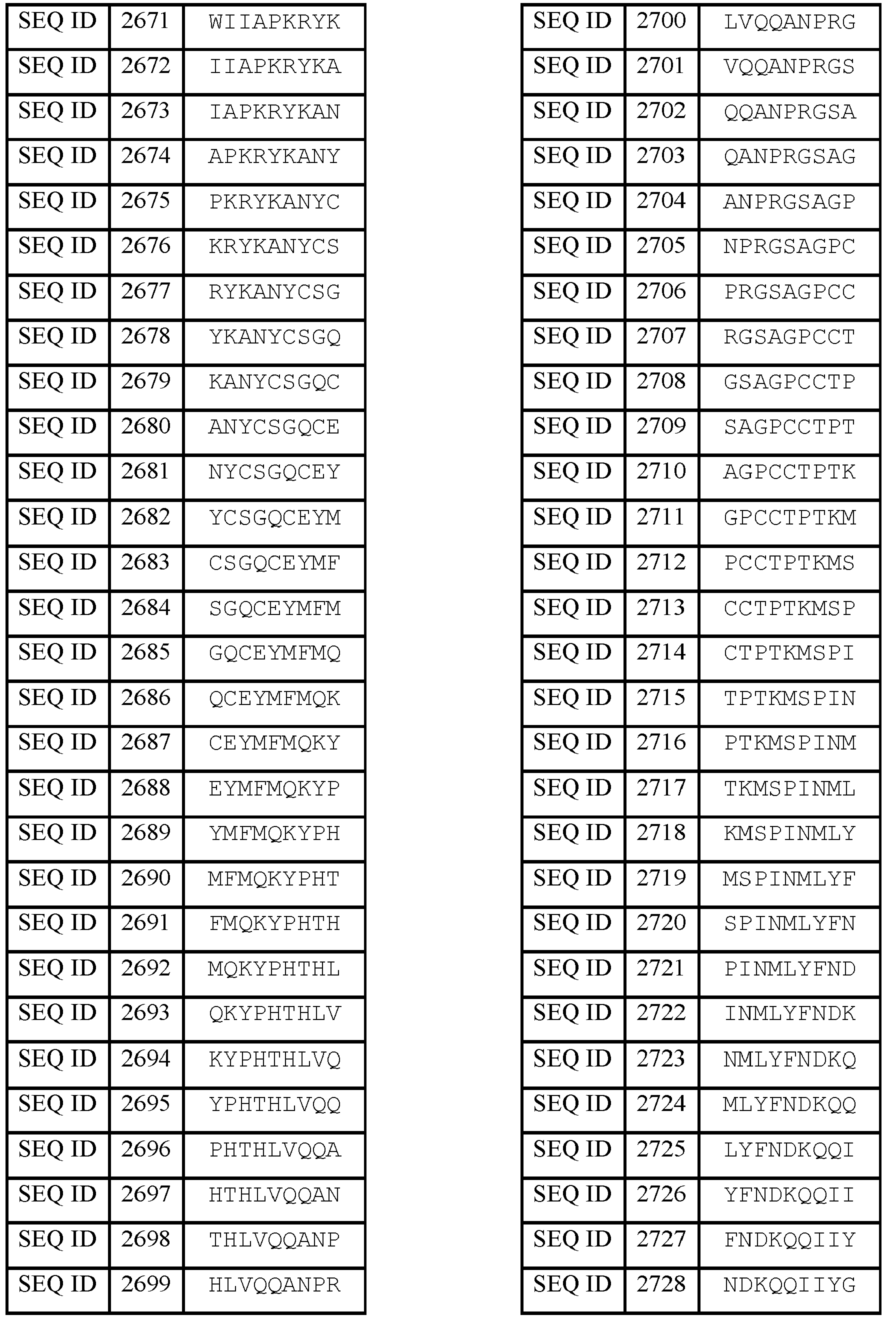

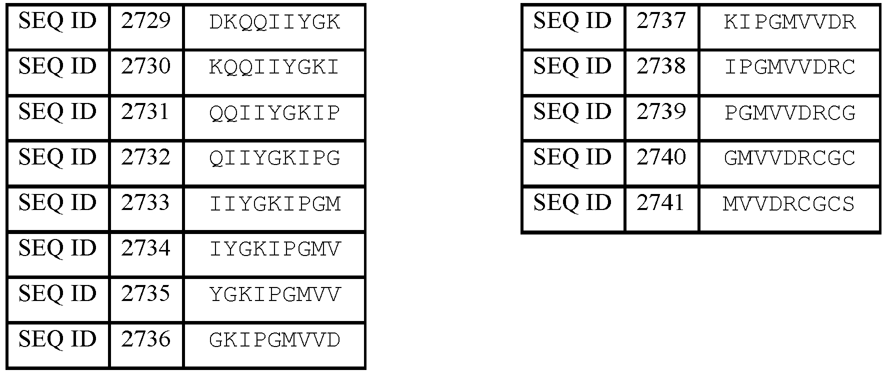

In some embodiments, the active agent comprises a peptide which further comprises any one or more of the 10-mer amino acid sequences (SEQ ID NO: 2742-3136) listed in Table 9.

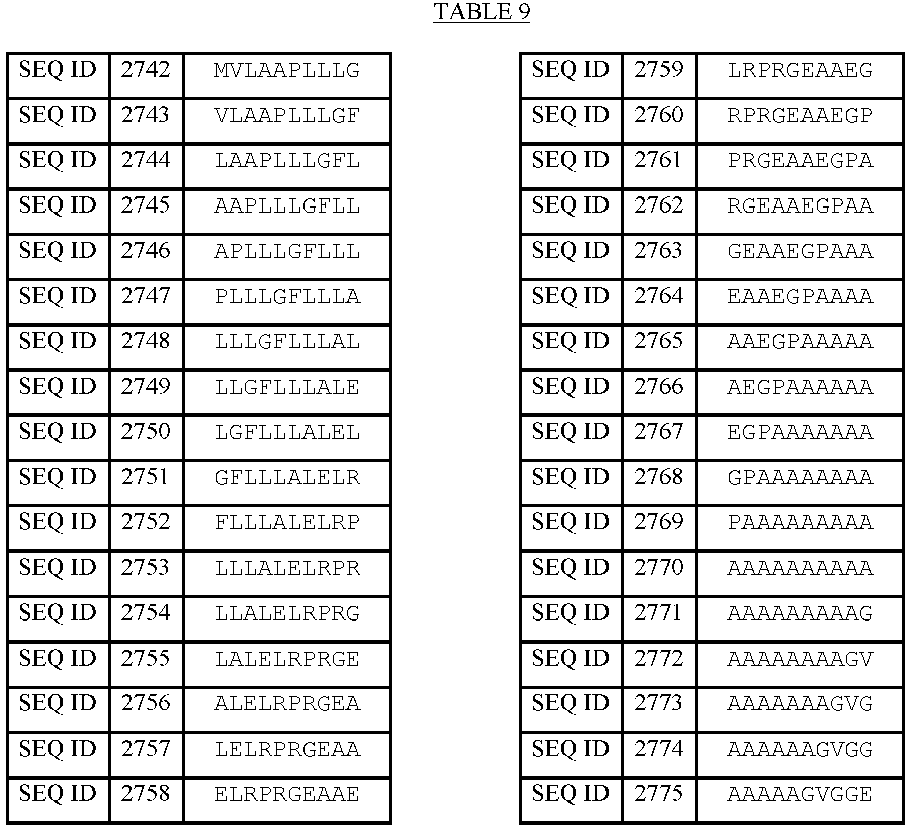

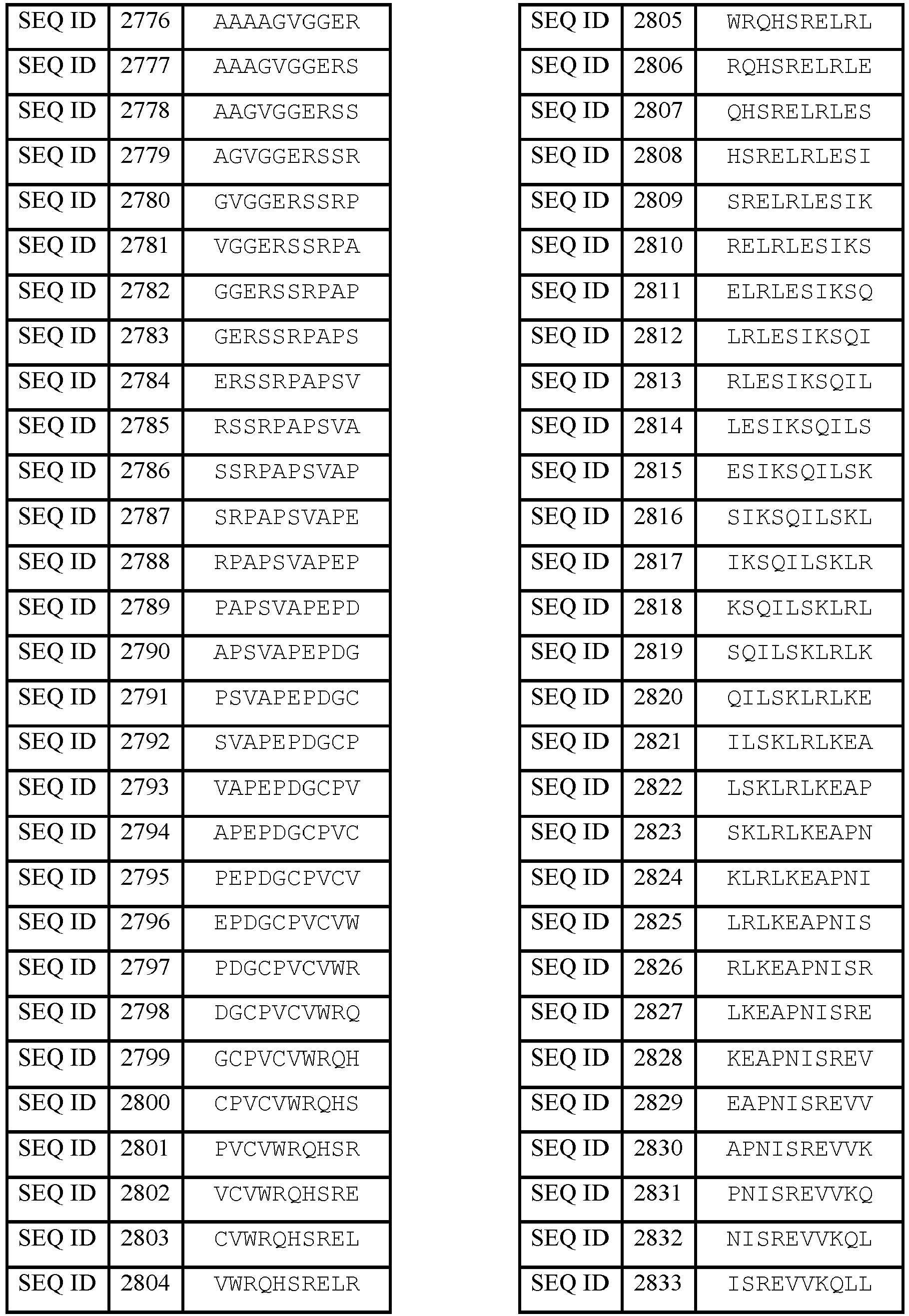

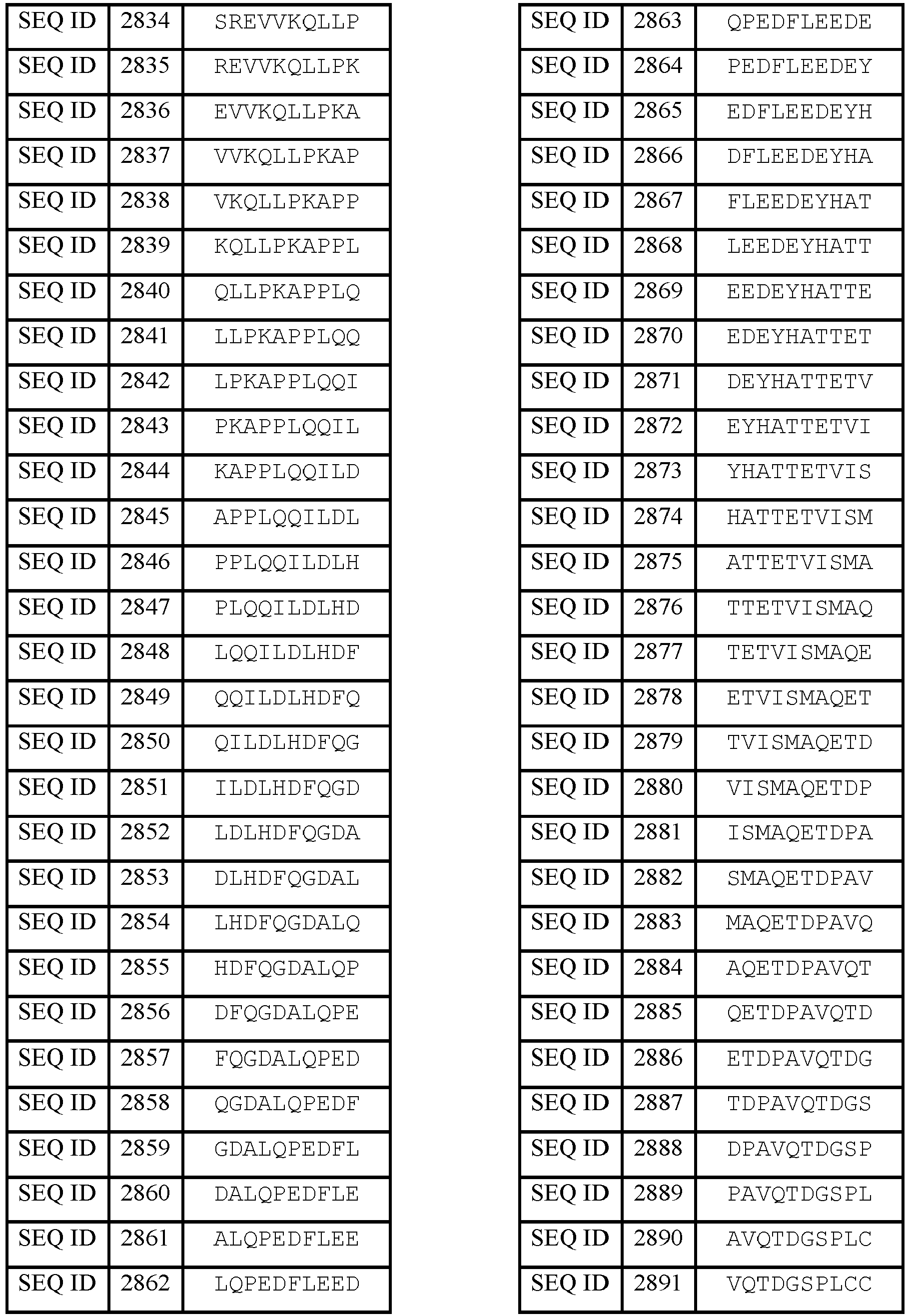

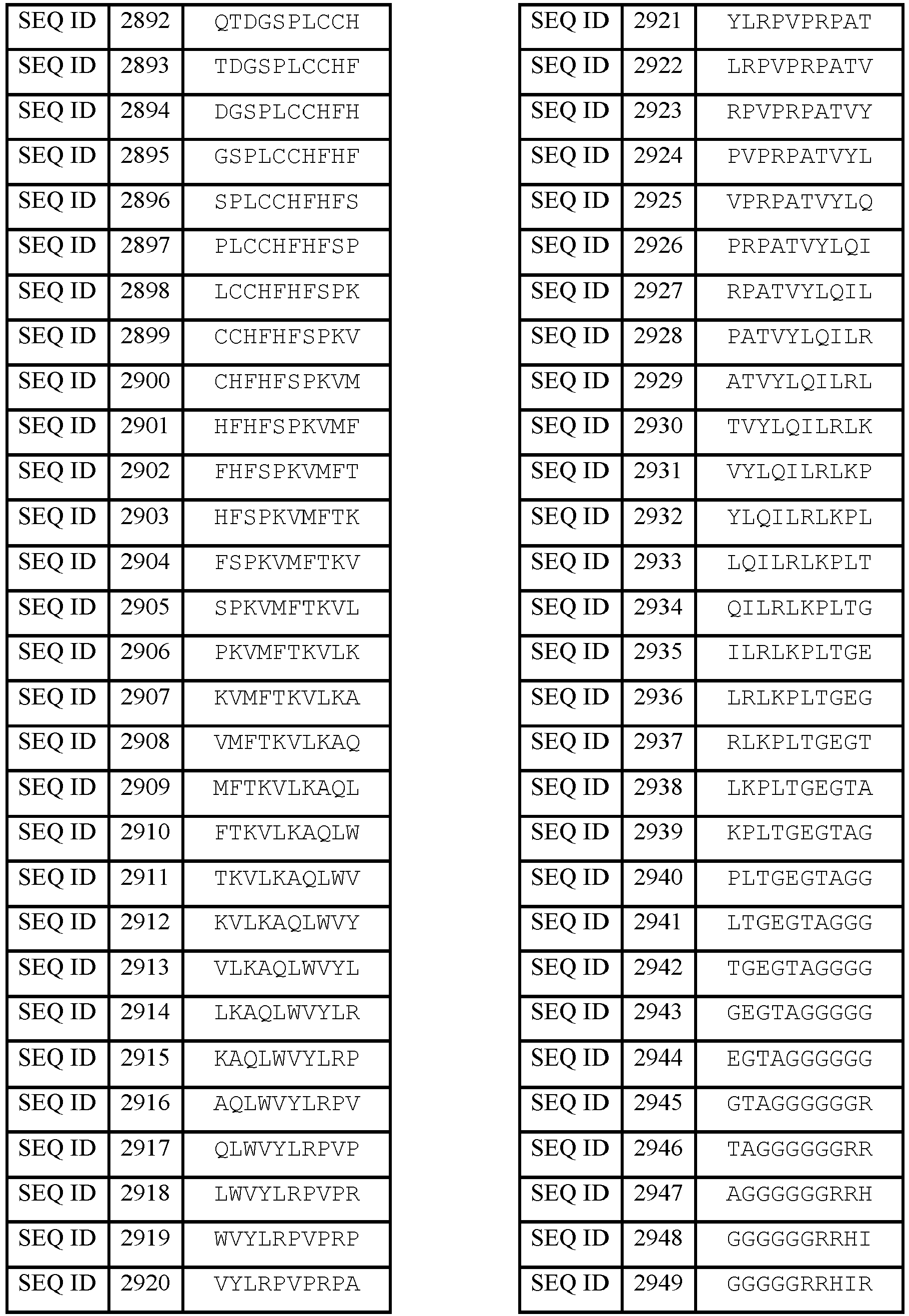

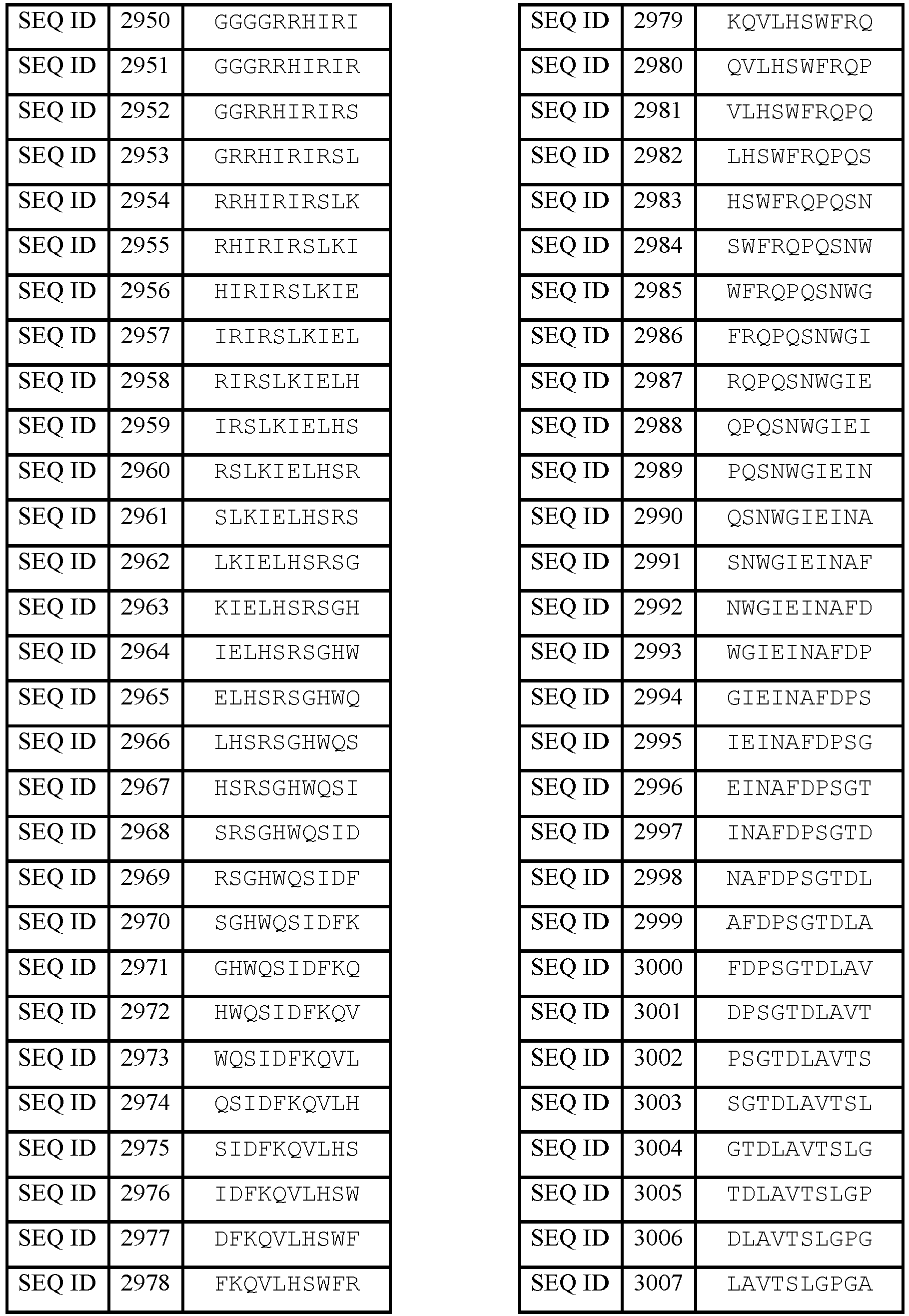

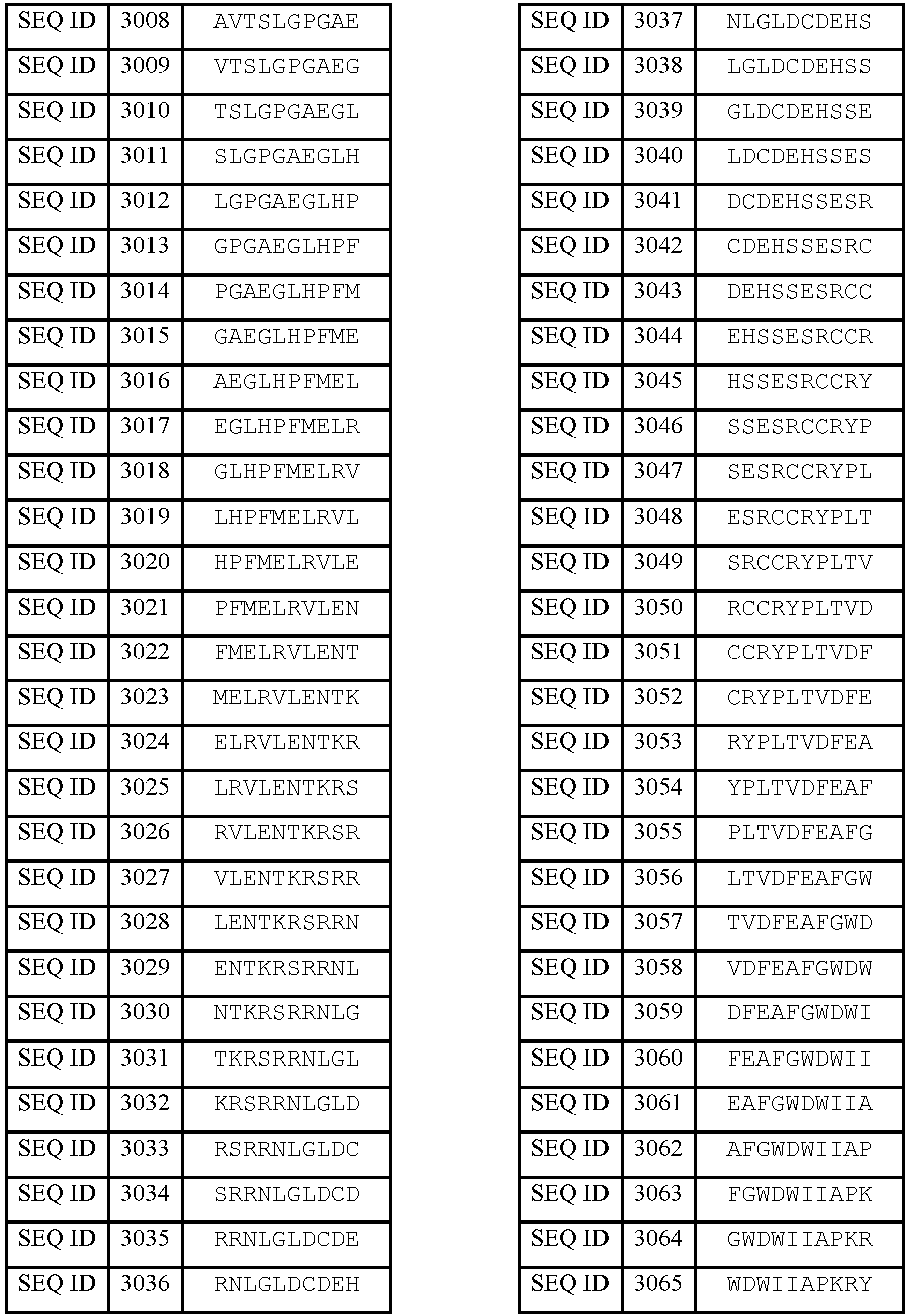

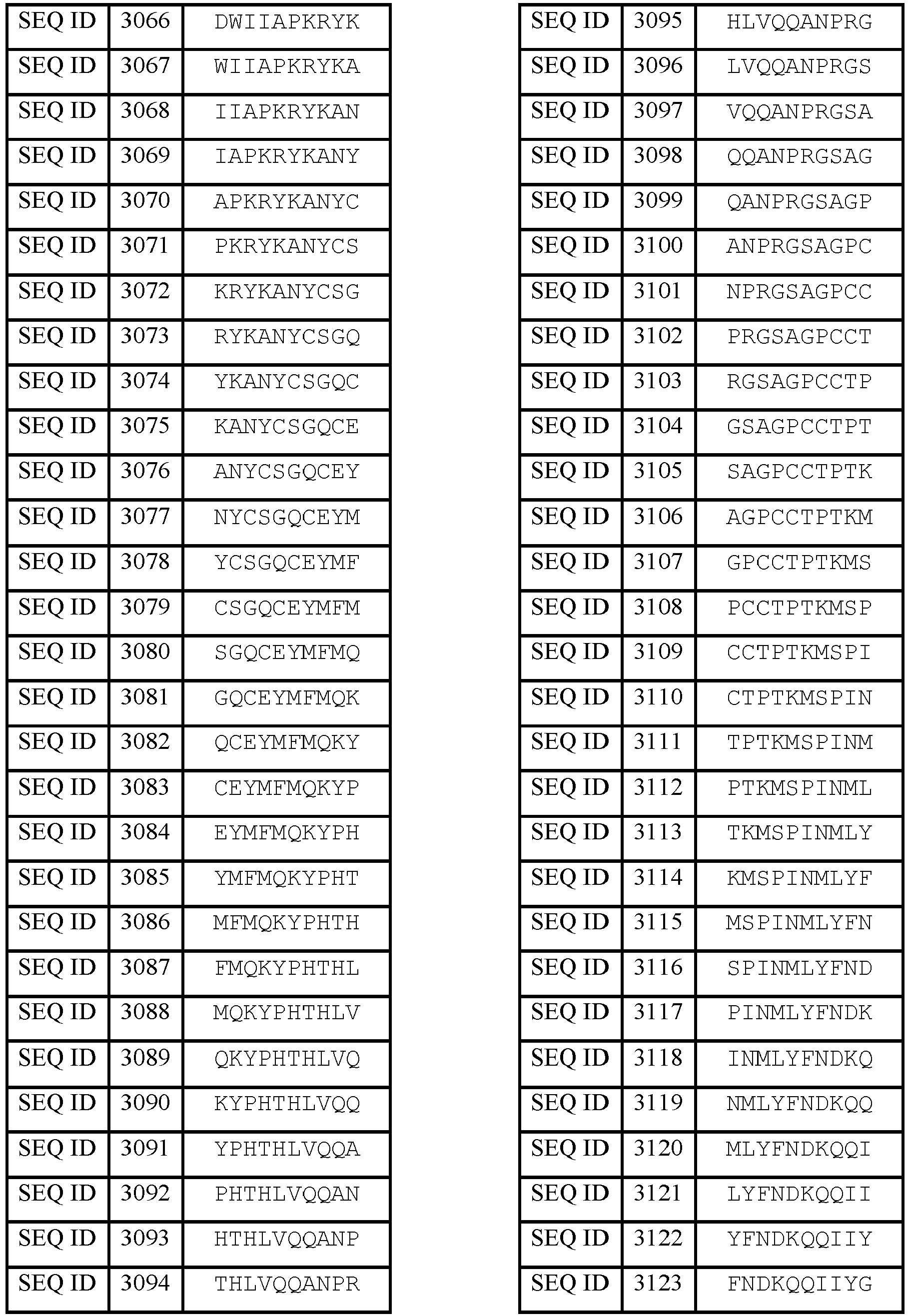

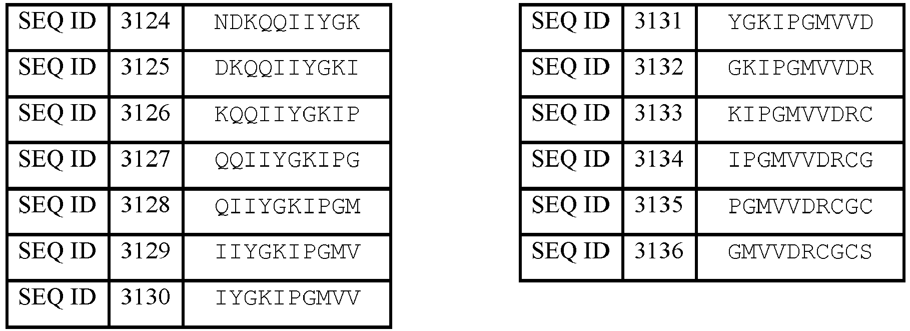

In some embodiments, the active agent comprises a peptide which further comprises any one or more of the 11-mer amino acid sequences (SEQ ID NO: 3137-3531) listed in Table 10.

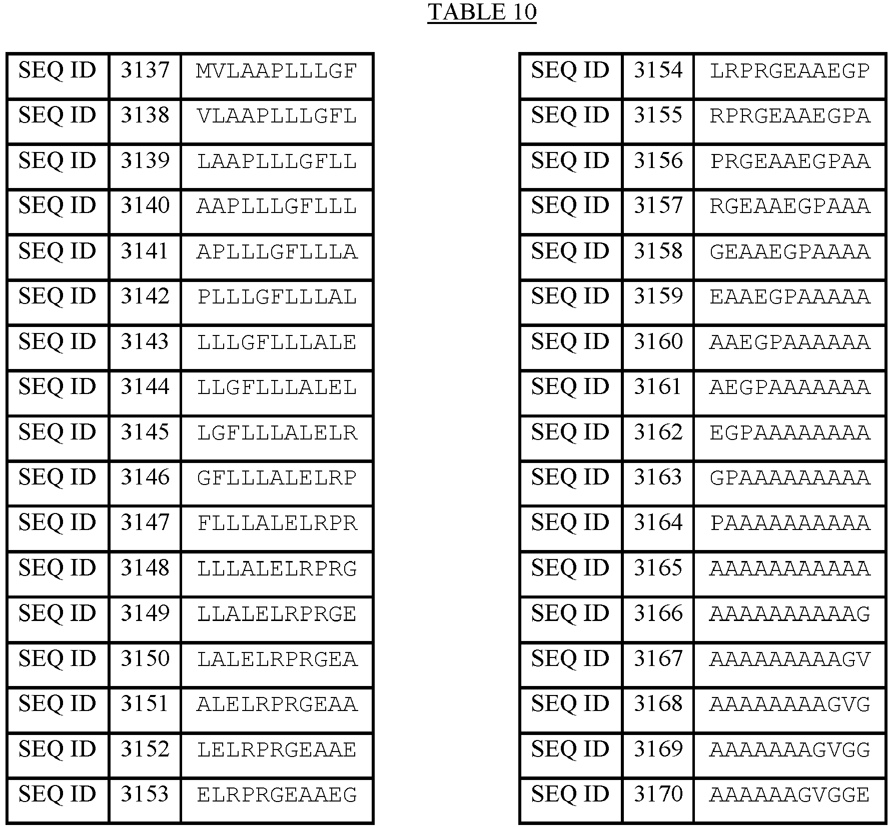

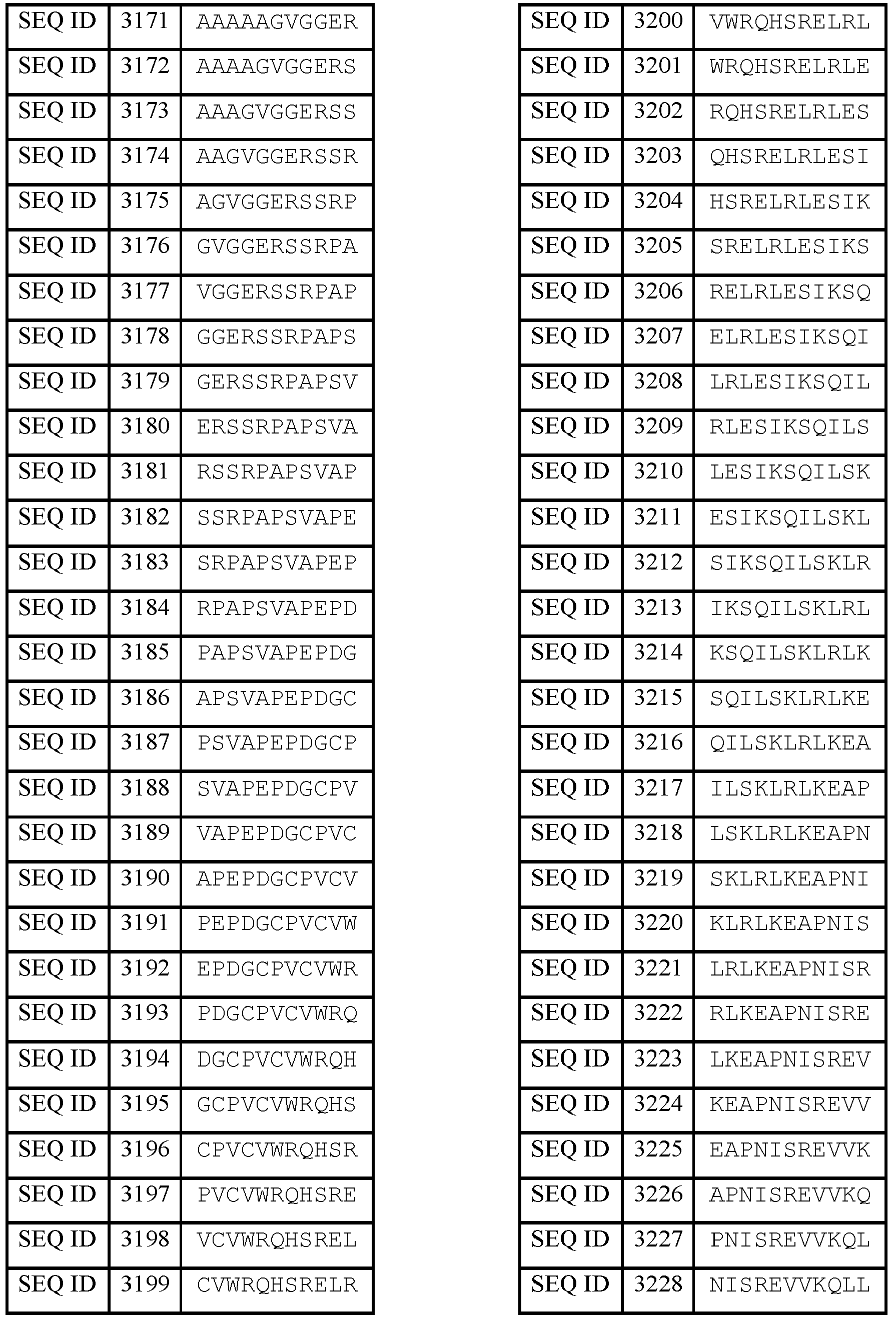

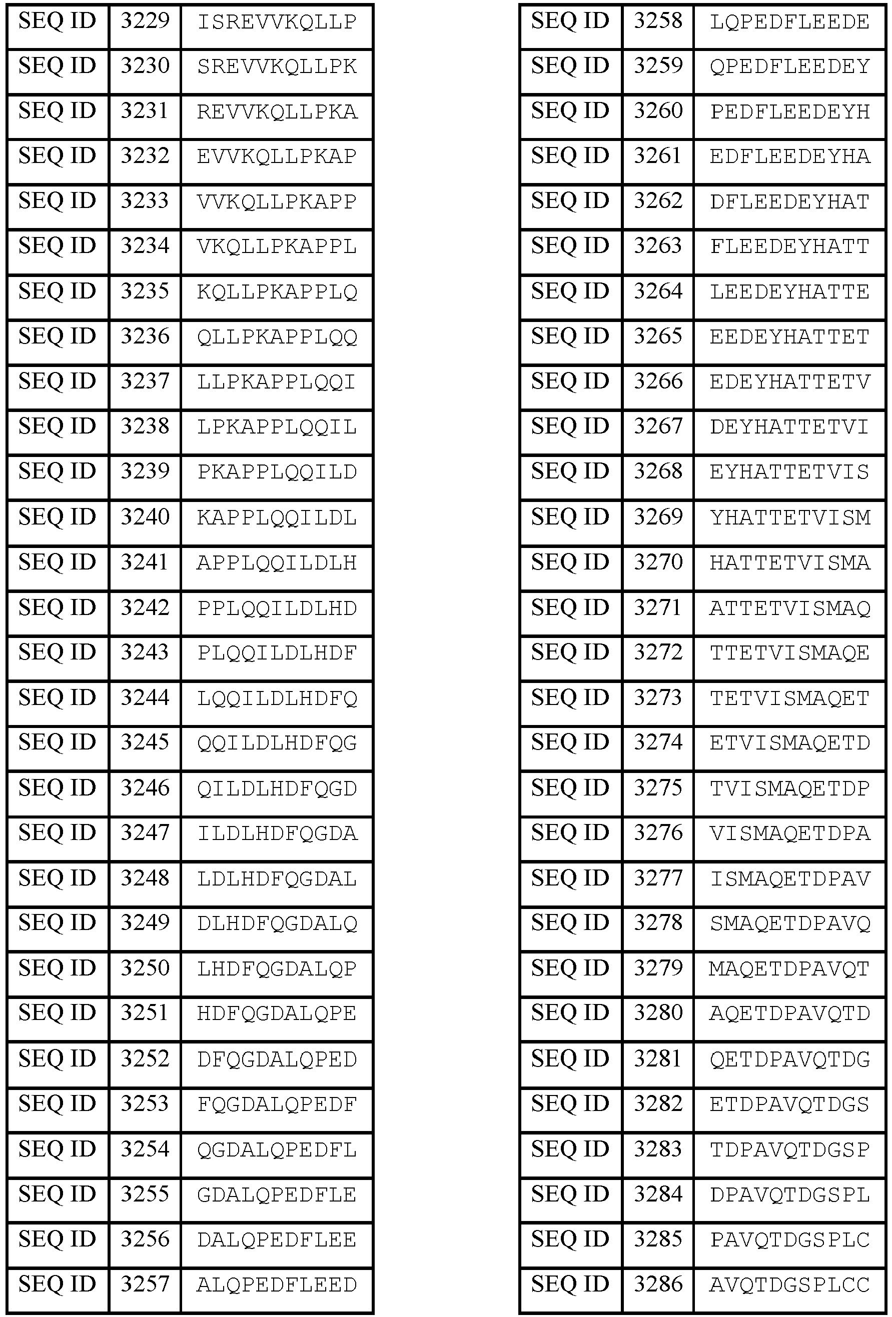

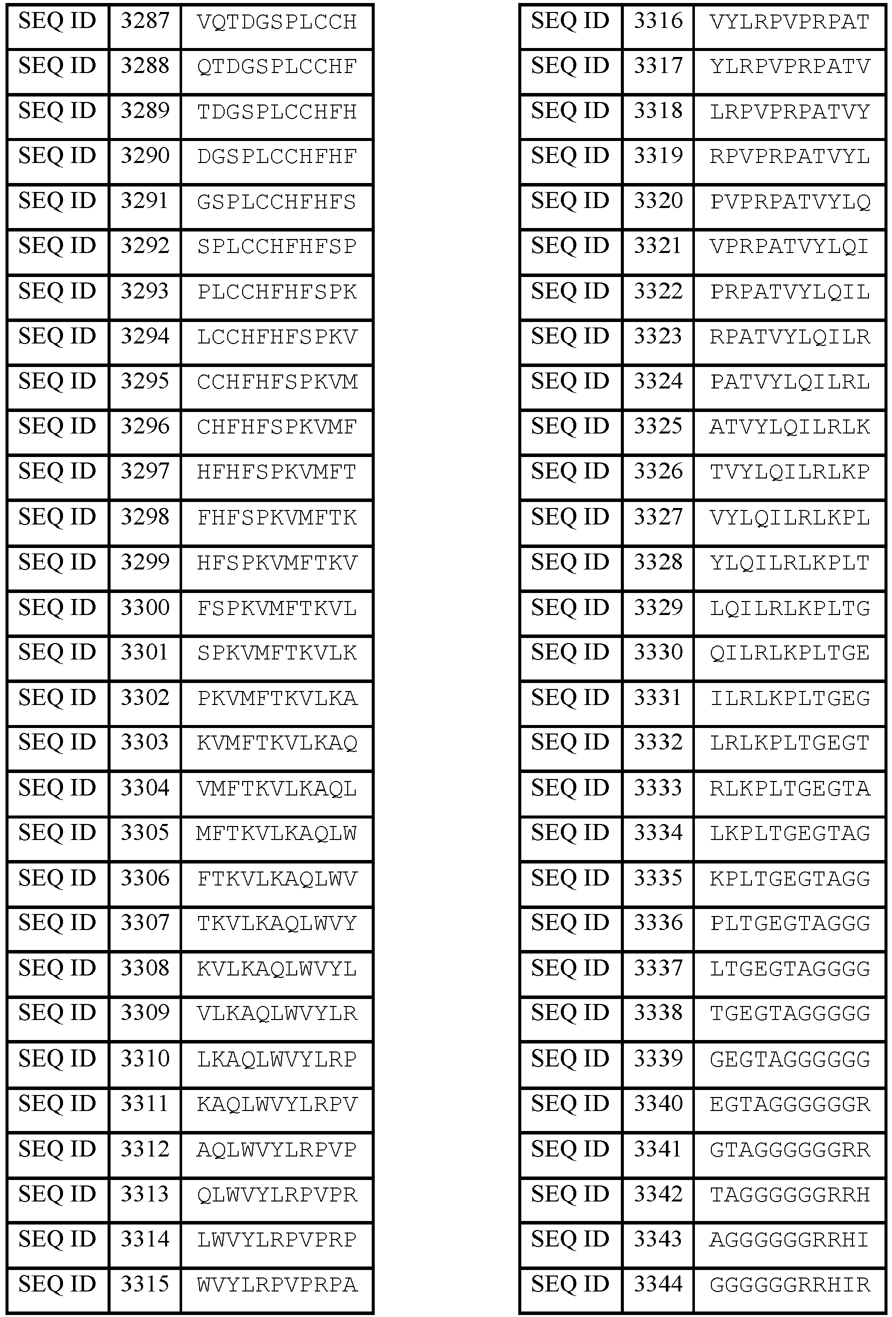

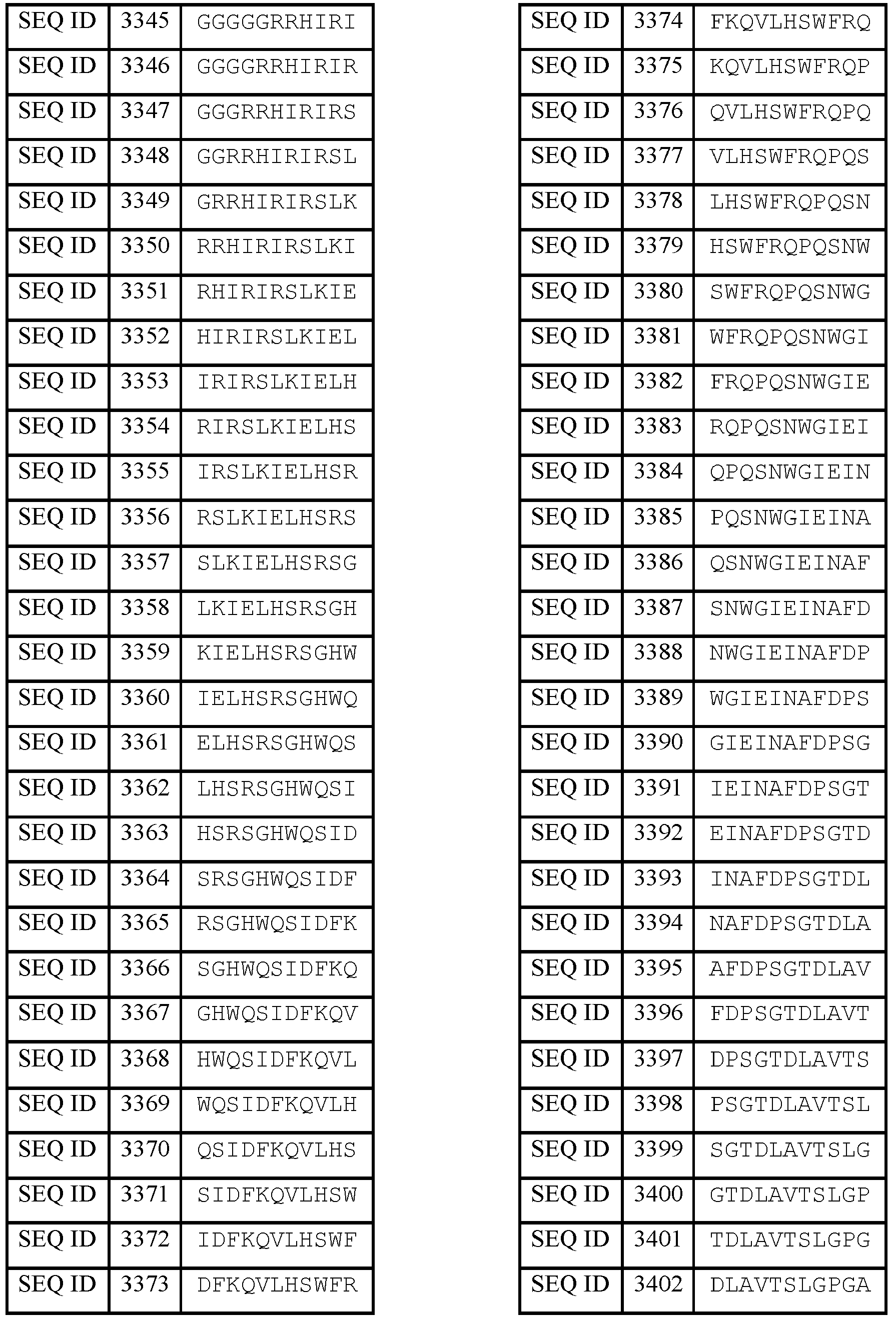

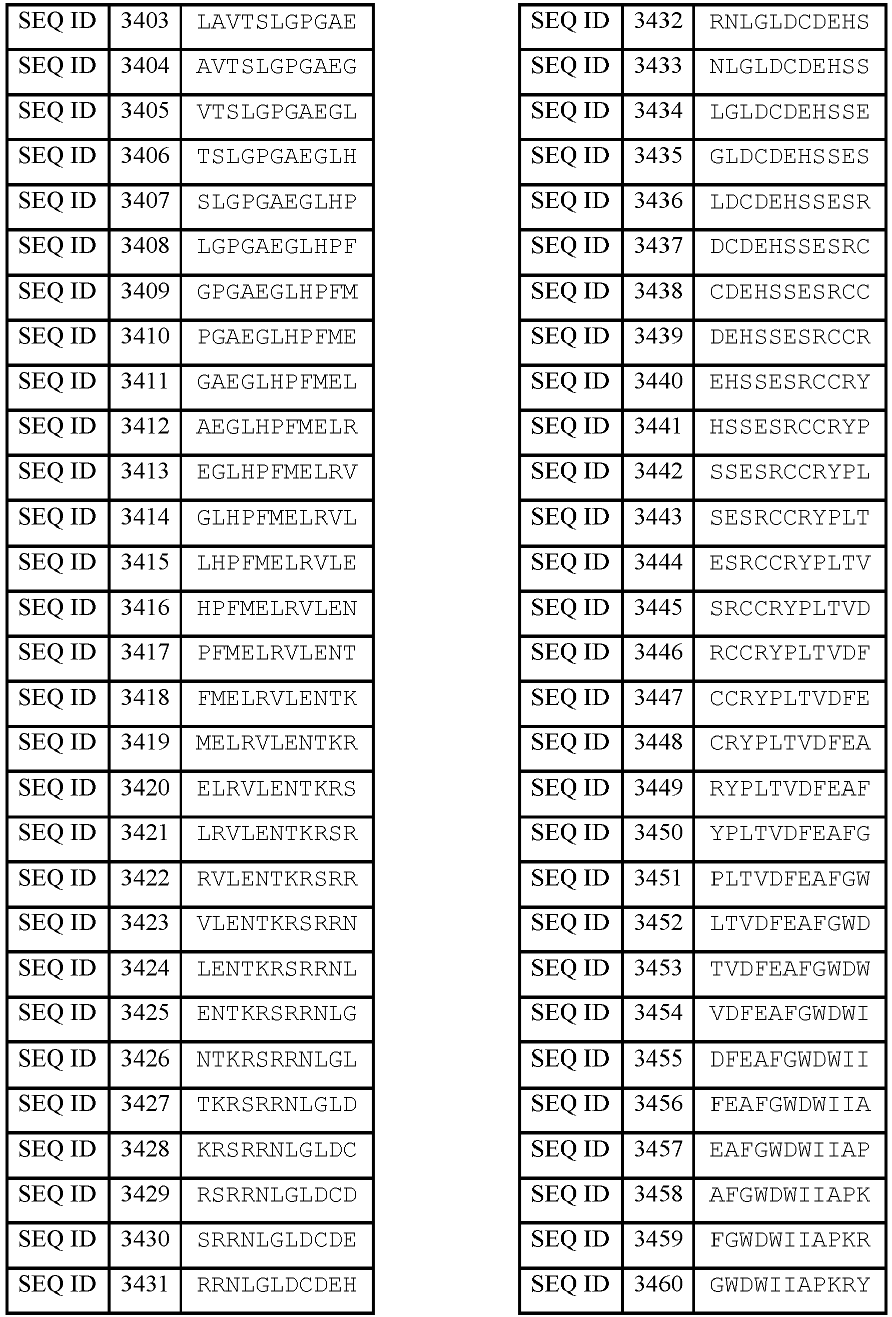

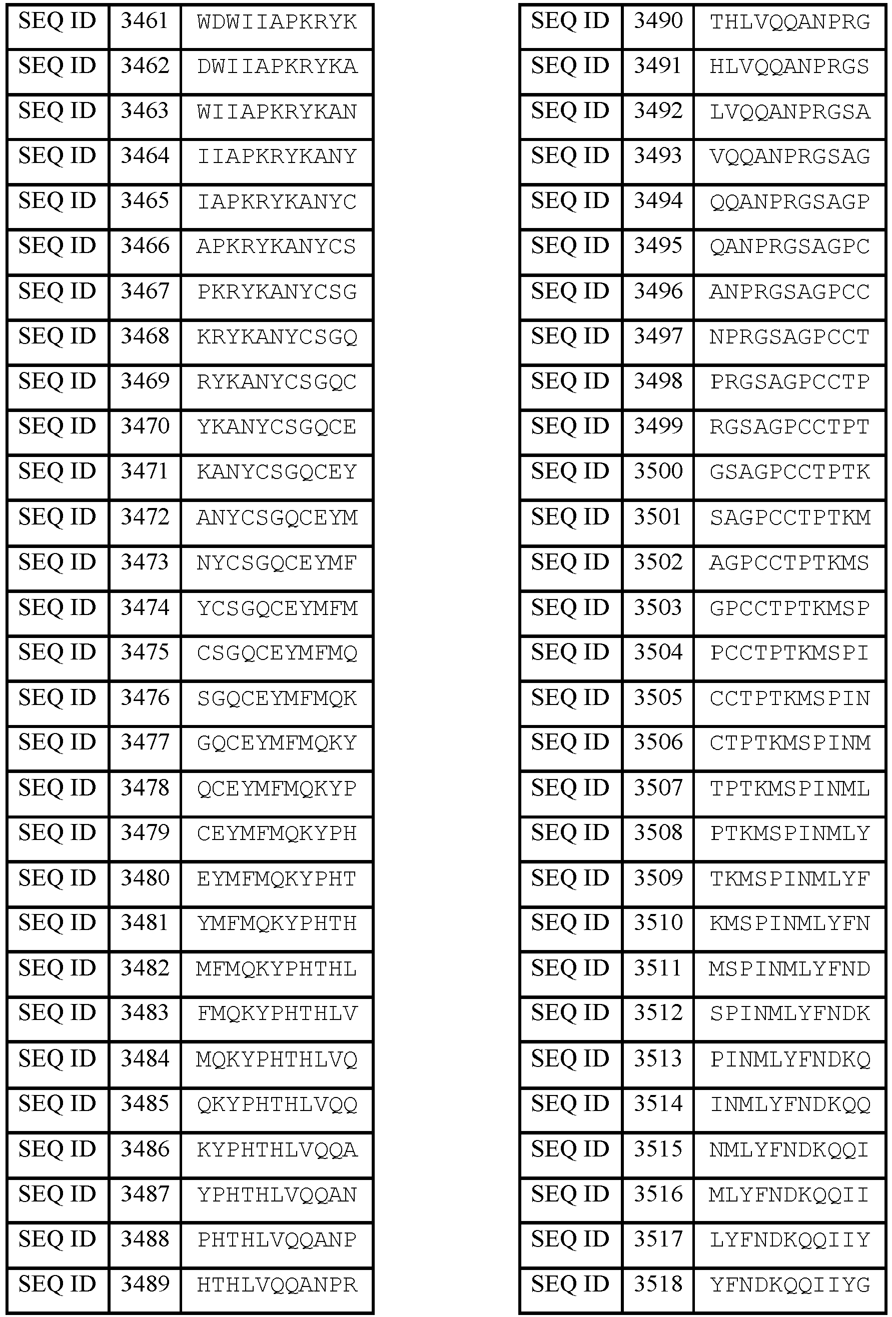

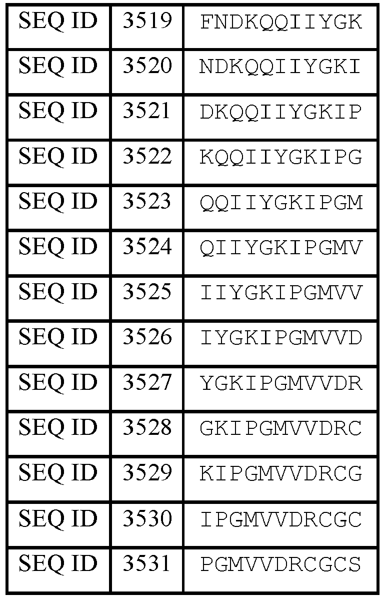

In some embodiments, the peptides may comprise one, two, three or more conservative substitutions of amino acids. As used herein, a "conservative substitution" is one in which substitution of one amino acid for another does not impair the function of the peptide, including substitution of an amino acid having a side chain of a certain nature (*e.g*., acidic, basic, aromatic, aliphatic uncharged, non-polar uncharged, hydrophilic uncharged) by another amino acid having a side chain of the same nature. Examples of conservative substitutions are shown in Table 11. In some embodiments, the convervative substitution is modified by oxidation. For example, in some embodiments, a proline residue in SEQ ID NOs 2-3531 may be replaced by an oxidized methionine (*e.g*., methionine sulfone, methionine sulfoxide, etc.) or an oxidized cysteine.

**TABLE 11. Conservative Substitutions**

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

In some embodiments, the peptides may comprise one, two, three or more *(e.g.,* one, two, three, *etc.*) non-natural and/or non-proteinogenic amino acids substituted or in place a comparable number of amino acids in SEQ ID NOs. 2-3531. In some embodiments, the peptides of the invention may comprise modified variants of SEQ ID NOs 2-3531 wherein at least one of the amino acids is replaced by the "D" (dextrorotary) analogue of the natural "L" optical isomer found in SEQ ID Nos 2-3531. In another embodiment, at least one *(e.g.,* one, two, three, *etc.*) of the amino acids found in SEQ ID Nos. 2-2531 are replaced with a non-naturally occurring and/or non-proteogenic amino acid according to Formulas (III) or (IV) as detailed below.

The peptides of the invention can be modified to improve the lipophilicity, stability, or to enhance penetration through the stratum corneum. In some embodiments, the peptides are modified with a fatty acid chain (*e.g.,* C₆₋₂₂), such as palmitoyl. In some embodiments, at least one of the nitrogen atoms in the amide bonds between adjacent amino acids may be methylated to improve metabolic stability. The peptides may also be phosphorylated, for example by forming one or more phophoserine, phosphothreonine and/or phosphotyrosine residues.

In some embodiments, the modified peptides will have the structure according to Formula (I):

R₁-Ω-R₂ (I)

where Ω represents a peptide comprising a chemically modified amino acid residue (*e.g*.,a peptide of SEQ ID 2-3531 wherein one or more amino acid residues have been oxidized) and R₁ and R₂ are independently either absent or are selected from hydrogen or C₁₋₂₆ (C₁₋₆ or C₆₋₁₂ or C₁₂₋₁₈ or C₁₈₋₂₂) hydrocarbons, optionally substituted with a group X₁ or with 1-20 (or 1-10 or 1-6 or 1-3) heteroatoms selected from halogen (*e.g*., fluorine), oxygen, nitrogen, phosphorous, sulfur, silicon and combinations thereof (more typically, oxygen and nitrogen). In some embodiments, one of R₁ and R₂ is a C₁₋₂₆ hydrocarbon. In some embodiments, only one of R₁ and R₂ is a C₁₋₂₆ hydrocarbon. In some embodiments, one of R₁ and R₂ is a C₁₋₂₆ hydrocarbon selected from the group consisting of alkyl, alkenyl, alkynyl, aryl, alkyl-aryl (*e.g*., benzyl), and aryl-alkyl optionally substituted with halogen (*e.g*., fluorine), oxygen, nitrogen, phosphorous, sulfur, and combinations thereof, in various embodiments comprising heteroatoms selected from halogen (*e.g*., fluorine), oxygen, nitrogen, phosphorous, sulfur, and combinations thereof, in various embodiments comprising from 1-10 or 1-6 or 1-3 heteroatoms. In some embodiments, R₁ and/or R₂ may comprise a group of the form R-(C=O)-, where R is a C₁₋₂₅ hydrocarbon as described above. In certain embodiments, the peptide derivative is N-acetylated. In one embodiment, R₁ and/or R₂ may comprise an acyl group, for example, one N-acylated or having the form CH₃-(CH₃)*n*-(C=O)- where "n" is an integer from 0-25 *(e.g.,* zero or from 0-17 or 7-17). In certain embodiments, R₁ and/or R₂ may comprise an acetyl group of the form CH₃-(C=O)-. In some embodiments, the peptide derivative comprises an acetyl group at the amino terminus. In some embodiments, the peptide derivative comprises an acetyl group at the carboxylic acid terminus. In one embodiment, R₁ and/or R₂ may comprise a palmitoyl group of the form CH₃-(CH₃)₁₄-(C=O)-. R₁ and/or R₂ may be attached to a nitrogen atom on the peptide so thereby form an amide bond of the form Ω-NH-(C=O)-R, formed, for example, through the reaction of an acid of the form R-(C=O)-OH (or activated derivative of the acid) with a nitrogen atom on the N-terminal amino group of the peptide or a nitrogen atom on a side chain (*e.g*., lysine) of the peptide. In some embodiments, R₁ and/or R₂ may be attached to the peptide through an amide bond of the form Ω-(C=O)-NH-R, formed, for example, by reaction of an amine of the form R-NH with the carboxyl terminus of the peptide or on a carboxyl-containing side chain (*e.g.*, aspartic acid or glutamic acid). In some embodiments, R₁ and/or R₂ may be attached to the peptide through an ester bond of the form Ω-(C=O)-O-R, formed, for example, through the reaction of an alcohol of the form R-OH with the carboxyl terminus of the peptide or carboxyl side chain (*e.g*., aspartic acid or glutamic acid). In some embodiments, R₁ and/or R₂ may be attached to the peptide through an ester bond of the form Ω-O-(C=O)-R, formed, for example, by the reaction of an acid of the form R-(C=O)-OH with a hydroxyl group on an amino acid side chain (*e.g*., serine or threonine). In any case where an acid is reacted, the acid may first be activated according to conventional practice by first converting it to an anhydride, acid halide, or activated ester, such as an N-hydroxysuccinimide ester, *etc.* It is also contemplated that R₁ and/or R₂ may be attached to the peptide through thioester bonds of the form Ω-S-(C=O)-R, thioether bonds of the form Ω-SR, ether bonds of the form Ω-O-R, and amines of the form of the form Ω-NR^{N}-R, to name but a few non-limiting examples. In various embodiments, R may be branched (*e.g*., ethylhexyl), cyclic, or straight chained. R and R^{N} may be, without limitation methyl, ethyl, propyl, butyl, pentyl, hexyl, heptyl, octyl, nonyl, decyl, undecyl, dodecyl, or C₁₃, or C₁₄, or C₁₅, or C₁₆, or C₁₇, or C₁₈, or C₁₉, or C₂₀, or C₂₁, or C₂₂, or C₂₃, or C₂₄, or C₂₅, or C₂₆ alkyl, akenyl, or alkynyl, etc, optionally substituted with a group X₁ or heteroatoms selected from halogen (*e.g*., fluorine), oxygen, nitrogen, phosphorous, sulfur, silicon, and combinations thereof, in various embodiments comprising from 1-10 or 1-6 or 1-3 heteroatoms. Any of the groups R, R₁, R₂ and R^{N} may be further substituted with from 1-3 groups X₁ where X₁ is selected independently at each occurence from hydrogen, -F; -Cl; -Br; -I; -OH, -OR^{∗}; -NH₂; -NHR^{∗}; -N(R^{∗})₂; -N(R^{∗})₃⁺; -N(R^{∗})-OH; -N(→O)(R^{∗})₂; -O-N(R^{∗})₂; -N(R^{∗})-O-R^{∗}; -N(R^{∗})-N(R^{∗})₂; -C=N-R^{∗}; -N=C(R^{∗})₂; -C=N-N(R^{∗})₂; -C(=NR^{∗})-N(R^{∗})₂; -SH; -SR^{∗}; -CN; -NC; -(C=O)-R^{∗}; -CHO; -CO₂H; -CO₂-; -CO₂R^{∗}; -(C=O)-S-R^{∗}; -O-(C=O)-H; -O-(C=O)-R^{∗}; -S-(C=O)-R^{∗}; -(C=O)-NH₂; -(C=O)-N(R^{∗})₂; -(C=O)-NHNH₂; -O-(C=O)-NHNH₂; - (C=S)-NH₂; -(C=S)-N(R^{∗})₂; -N(R^{∗})-CHO; -N(R^{∗})-(C=O)-R^{∗}; -(C=NR)-O-R^{∗}; -O-(C=NR^{∗})-R^{∗}, -SCN; -NCS; -NSO; -SSR^{∗}; -N(R^{∗})-C(=O)-N(R^{∗})₂; -N(R^{∗})-C(=S)-N(R^{∗})₂; -SO₂-R^{∗}; -O-S(=O)₂-R^{∗}; -S(=O)₂-OR^{∗}; -N(R^{∗})-SO₂-R^{∗}; -SO₂-N(R^{∗})₂; -O-SO₃-R^{∗}; -O-S(=O)₂-OR^{∗}; -O-S(=O)-OR^{∗}; -O-S(=O)-R^{∗}; -S(=O)-OR^{∗}; -S(=O)-R^{∗}; -NO; - NO₂; -NO₃; -O-NO; -O-NO₂; -N₃; -N₂-R^{∗}; -N(C₂H₄); -Si(R^{∗})₃; -CF₃; -O-CF₃; -PR^{∗}₂; - O-P(=O)(OR^{∗})₂; -P(=O)(OR^{∗})₂; C₁-C₈ perfluoroalkyl; an aliphatic C₁-C₈ hydrocarbon radical; a C₁-C₈ aromatic hydrocarbon radical; or a C₁-C₈ heteroaryl radical. R^{∗} is a C₁₋₁₀ hydrocarbon, such as methyl, ethyl, propyl, butyl, pentyl, hexyl, benzyl, phenyl, *etc.* Any two of R, R^{∗}, R^{N}, R₁, and R₂ may together form a 3-8 membered, optionally heterocyclic ring.

In some embodiments, R₁ or R₂ is attached covalently to the terminal carboxyl group. In some embodiments, R₁ and/or R₂ is attached to the terminal amino group. In some embodiments, R₁ and/or R₂ is attached to a side chain having a nitrogen, oxygen, or sulfur atom.

In some embodiments, R₁ and/or R₂ promotes adhesion to or penetration of an integument. In some embodiments, R₁ and/or R₂ comprise biotin, a beta-keto ester, or a polyarginine sequence (*e.g.*, having 3-15 arginines).

The peptide can be pegylated to enhance water-solubility. In some embodiments, R₁ and/or R₂ have the form -(OCH₂CH₂)*y*-Z or -(CH₂CH₂O)*y*-Z, where *"y"* is an integer from 1-20 (or from 1-10 or from 1-6 or from 1-3) and Z is H, R₃, X₁, or R₄-X₁, where R₃ and R₄ are independently branched, straight chained, or cyclic C₁₋₆ hydrocarbons (*e.g.*, methyl, ethyl, propyl, methylene, -(CH₂)ₙ- (n = 1-6), etc.). In some embodiments, R₁ and/or R₂ comprise mini-PEG (*i.e.,* 11-amino-3,6,9-trioxaundecanoic acid).

In some embodiments, the modified peptide may comprise a single modified amino acid residue. For example, the modified peptide may have the structure of formula (Ia):

R₁-(Ω₁)ₓ-Ωₘ-(Ω₂)_{y}-R₂ (Ia)

wherein Ω₁ and Ω₂ are independently either absent (*i.e.,* it is a bond) or independently selected at each occurrence from amino acid residues and x and y are independently integers from 0-10 or from 1-10 (preferably from 1-3 or 1-2) with the proviso that the sum of x and y is no more than 10, no more than 6, or no more than 4, or no more than 2; and
Ωₘ is a modified amino acid residue (such as an amino acid residue having an oxidized side chain). In certain embodiments, the sum of x and y is two, three, four, five, six, seven, eight, nine, or ten. In some embodiments, Ω₁ and Ω₂ may, for example, be independently selected at each occurrence from Alanine, Cysteine, Aspartic acid, Glutamic acid, Phenylalanine, Glycine, Histidine, Isoleucine, Lysine, Leucine, Methionine, Asparagine, Pyrrolysine, Proline, Glutamine, Arginine, Serine, Threonine, Selenocysteine, Valine, Tryptophan, and Tyrosine; and Ωₘ is preferably, but not necessarily, a sulfoxide or sulfone derivative of an amino acid.

A modified amino acid residue (*e.g.,* Ωₘ) may have the structure: 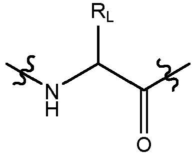 or wherein R_{L} is a modified (*e.g.,* oxidized) sidechain of an amino acids. The side chain is typically a C₁₋₁₂ hydrocarbon, optionally containing from 1-4 or 1-3 or 1-2 or 1 heteroatoms selected from O, N, and S. The side chain of amino acid to be oxidized may be the side chain of Alanine, Cysteine, Aspartic acid, Glutamic acid, Phenylalanine, Glycine, Histidine, Isoleucine, Lysine, Leucine, Methionine, Asparagine, Pyrrolysine, Proline, Glutamine, Arginine, Serine, Threonine, Selenocysteine, Valine, Tryptophan, or Tyrosine. For example, the side chain may be any of the side chains in Table 12. According to the present invention, the peptide within the topical composition has an amino acid sequence comprising or consisting of M(O)₂VV or M(O)VV, wherein the methionine amino acid residue of said sequence is oxidized.

**TABLE 12**

| Amino Acid | Side Chain Prior to Modification |
|---|---|
| Ala | -CH₃ |
| Ser | -CH₂OH |
| Thr | -CH(CH₃)(OH) |
| Cys | -CH₂SH |
| Val | -CH(CH₃)₂ |
| Leu | -CH₂CH(CH₃)₂ |
| Ile | 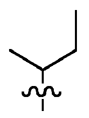 |
| Met | 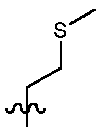 |
| Phe | 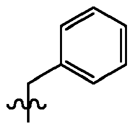 |
| Tyr | 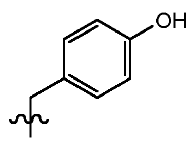 |
| Trp | 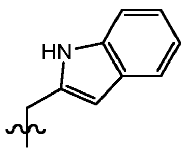 |
| Asp | 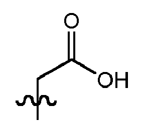 |
| Glu | 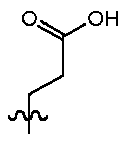 |
| Asn | 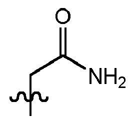 |
| Gln | 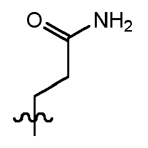 |
| His | 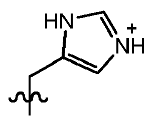 |
| Lys | 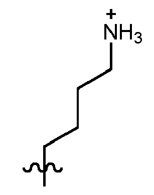 |
| Arg | 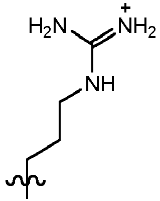 |

It will be understood that in the event of any inconsistency between the depicted side chains in Table 12 and the side chains of the indicated amino acid, both will be considered within the scope of possible side chains prior to modification. In some embodiments, the side chain is the side chain of Cys, Met, Phe, Tyr, Trp, Asp, Glu, Asn, Gln, His, or Lys. The chemical modification may produce a side chain substituted one or more times substituted one or more times with a group X₁ is selected independently at each occurence from hydrogen, -F; -Cl; - Br; -I; -OH, -OR^{∗}; -NH₂; -NHR^{∗}; -N(R^{∗})₂; -N(R^{∗})₃⁺; -N(R^{∗})-OH; -N(→O)(R^{∗})₂; -ON(R^{∗})₂; -N(R^{∗})-O-R^{∗}; -N(R^{∗})-N(R^{∗})₂; -C=N-R^{∗}; -N=C(R^{∗})₂; -C=N-N(R^{∗})₂; -C(=NR^{∗})-N(R^{∗})₂; -SH; -SR^{∗}; -CN; -NC; -(C=O)-R^{∗}; -CHO; -CO₂H; -CO₂-; -CO₂R^{∗}; -(C=O)-S-R^{∗}; -O-(C=O)-H; -O-(C=O)-R^{∗}; -S-(C=O)-R^{∗}; -(C=O)-NH₂; -(C=O)-N(R^{∗})₂; -(C=O)-NHNH₂; -O-(C=O)-NHNH₂; -(C=S)-NH₂; -(C=S)-N(R^{∗})₂; -N(R^{∗})-CHO; -N(R^{∗})-(C=O)-R^{∗}; -(C=NR)-O-R^{∗}; -O-(C=NR^{∗})-R^{∗}, -SCN; -NCS; -NSO; -SSR^{∗}; -N(R^{∗})-C(=O)-N(R^{∗})₂; -N(R^{∗})-C(=S)-N(R^{∗})₂; -SO₂-R^{∗}; -O-S(=O)₂-R^{∗}; -S(=O)₂-OR^{∗}; -N(R^{∗})-SO₂-R^{∗}; -SO₂-N(R^{∗})₂; -O-SO₃-R^{∗}; -O-S(=O)₂-OR^{∗}; -O-S(=O)-OR^{∗}; -O-S(=O)-R^{∗}; -S(=O)-OR^{∗}; -S(=O)-R^{∗}; -NO; -NO₂; -NO₃; -O-NO; -O-NO₂; -N₃; -N₂-R^{∗}; -N(C₂H₄); -Si(R^{∗})₃; -CF₃; -O-CF₃; -PR^{∗}₂; -O-P(=O)(OR^{∗})₂; -P(=O)(OR^{∗})₂; C₁-C₈ perfluoroalkyl; an aliphatic C₁-C₈ hydrocarbon radical; a C₁-C₈ aromatic hydrocarbon radical; or a C₁-C₈ heteroaryl radical;
wherein R^{∗} is a C₁₋₁₀ hydrocarbon. In some embodiments, the side chain is substituted with one or more oxygen atoms, for example, =O, -OH, or →O. The resulting oxidized side chain may containe sulfone, sulfoxide, nitro, nitroso, and/or oxo group, to name a few. According to the present invention, a side chain of the peptide within the topical composition is an oxidized methionine side chain, wherein R_{L} may be selected from:

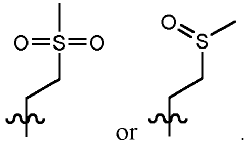

The chemically modified peptides Ω may be modified to improve stability or function by incorporating one or more additional amino acids to either or both ends of SEQ ID NO: 2-3531 according to Formula (II):

Ψ₁-Φ-Ψ₂ (II)

where Φ represents a peptide derivative of the invention having an amino acid sequence comprising or consisting of M(O)₂VV or M(O)VV *(e.g.,* a methionine-oxidized derivative of an amino acid sequence comprising any of SEQ ID NO: 369, 761, 762, 1155-1157, 1550 - 1553, 1945 - 1949, 2340 - 2345, 2735 - 2741, 3130 - 3136 or 3525 - 3531) and Ψ ₁ and Ψ ₂ are independently either absent or are selected from hydrogen, an amino acid, a non-natural amino acid, a non-proteinogenic amino acid, a di- or tri-peptide, or combinations thereof. Suitable amino acids include without limitation, Alanine, Cysteine, Aspartic acid, Glutamic acid, Phenylalanine, Glycine, Histidine, Isoleucine, Lysine, Leucine, Methionine, Asparagine, Pyrrolysine, Proline, Glutamine, Arginine, Serine, Threonine, Selenocysteine, Valine, Tryptophan, and Tyrosine. Each of the foregoing (except glycine) may be in the "L" or "D" optical isomeric configurations. The non-natural amino acid or non-proteinogenic amino acids may be, for example, a dextrorotary "D" optical isomer of a naturally occurring L-amino acid. The non-natural amino acid or non-proteinogenic amino acids may, for example, have the structure of formula (III) or (IV): 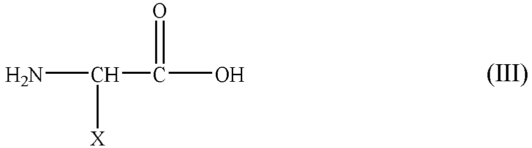 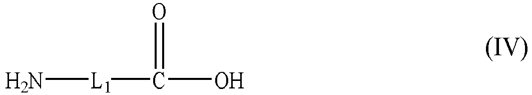 where X is selected from X₁, C₁₋₂₆ (C₁₋₆ or C₆₋₁₂ or C₁₂₋₁₈ or C₁₈₋₂₂) hydrocarbons, optionally substituted with a group X₁ or with from 1-20 (or 1-10 or 1-6 or 1-3) heteroatoms selected from halogen (e.g., fluorine, chlorine, bromine, iodine), oxygen, nitrogen, phosphorous, sulfur, silicon and combinations thereof. In some embodiments, X is ethyl, propyl, butyl, pentyl, hexyl, heptyl, octyl, nonyl, decyl, undecyl, dodecyl. In some embodiments, X is C₁₋₁₂ or C₂₆ alkyl, akenyl, akynyl, aryl, aryl-alkyl, alkyl-aryl, alkyl-aryl-alkyl, heteroaryl, alkyl-heteroaryl, heteroaryl-alkyl, alkyl-heteroaryl-alkyl, etc., optionally substituted with X₁, or with 1-20 (or 1-10 or 1-6 or 1-3) heteroatoms selected from halogen (*e.g.,* fluorine), oxygen, nitrogen, phosphorous, sulfur, and combinations thereof. In some embodiments, X comprises a fused ring system having two, three, or more 5- or 6-membered rings. L₁ is a hydrocarbon spacer comprising from 1-20 carbon atoms and optionally substituted with a group X₁ or from 1-20 (or 1-10 or 1-6 or 1-3) heteroatoms selected from halogen (*e.g.,* fluorine, chlorine, bromine, iodine), oxygen, nitrogen, phosphorous, sulfur, silicon and combinations thereof. In some embodiments, L₁ will have the form-(CH₃)*p*- where "p" is an integer from 1-20 or from 1-10 or from 1-6. In some embodiments, L₁ will comprise from 1-6 oxo or oxa groups. In one embodiment, the amino acid of formula (IV) is aminoethanoic acid, aminopropionic acid, aminobutyric acid, aminovaleric acid, aminocaproic acid, aminoenanthic acid, aminocaprylic acid, amino pelargonicacid, or aminocapric acid. In one embodiment, Ψ₁ and/or Ψ₂ comprises lysyl-aminovaleric acid or aminovaleric acid-lysyl. In some embodiments either terminus may be functionalized with an amino acid of the form H₂N-(CH₂)*_{q}*-CO₂H where "q" is an integer from 1-10, including amino valeric acid. In some embodiments, a lysine-amino valeric acid group is added at either terminus through a peptide bond. In some embodiments, Ψ₁ and/or Ψ₂ comprise oligomers having 2-16 or 2-8 or 2-6 or 2-4 amino acids, for example, naturally occurring amino acids. The peptide derivatives can also be cyclized.

The peptides of formula (II) may further be modified according to formula (I) such that they have the form of formula (V):

R₁-Ψ₁-Φ-Ψ₂-R₂ (V)

wherein, any of R₁, R₂, Ψ₁, and Ψ₂ may be absent but are otherwise defined as above.

Derivatized peptides may have one or more additional amino acids joined to the amino and/or carboxy terminus *via* peptide bonds. For example, polyarginine (n = 2-15) may be beneficially used to enhance penetration of the peptide into skin. In some embodiments, the peptides will comprise a hydrocarbon chain on the amino and/or carboxyl terminus, including, without limitation, C₁₋₂₄ or C₆₋₁₈ or C₁₂₋₁₈ aliphatic hydrocarbons, which may be straight chained or branched or cyclic. In some embodiments, the peptides include the reaction product of a peptide with a fatty acid or fatty alcohol. A fatty acid or alcohol, as used herein, contains 6-26 carbon atoms. For example, the N-terminus may be reacted with a C₆₋₂₄ fatty acid *(e.g.,* palmitic acid) to form an amide bond. The carboxyl terminus may be reacted with a C₆₋₂₄ fatty alcohol *(e.g.,* cetyl alcohol) to form an ester. These fatty derivatives may improve the lipophilicity of the peptide.

Topically acceptable salts and prodrugs of the peptide derivatives are also suitable. Salts will typically be acid addition salts formed by the reaction of the peptide derivative with an inorganic or an organic acid. Inorganic acids include mineral acids such as HCl and H₂SO₄, and the like. Organic acids include citric, benzoic, tartaric, malic, maleic, succinic, acetic, and propionic acid. The peptides may exist in zwitterionic form. Prodrugs include any esters or amides that hydrolyze *in vivo* to yield the peptide. Examples of suitable prodrugs can be found in the book entitled "Prodrugs and Targeted Delivery: Towards Better ADME Properties," Volume 47 (2011), published by WILEY-VCH Verlag & Co. In one embodiment, the prodrug is formed by reacting the peptide with glyoxylic acid to produce peptidyl-α-hydroxylglycine derivatives having improved stability. In other embodiments, the prodrugs may include terminal N-acetyl derivatives, side chain N-acetyl derivatives, N-hydroxy methylation or N-phthalidation of its N-terminus and/or side chain.

The active agent comprises a peptide having an amino acid sequence comprising or consisting of M(O)₂VV or M(O)VV or a derivative thereof, wherein the methionine amino acid residue of said sequence is oxidized. In some embodiments, the active agent may have the structure: 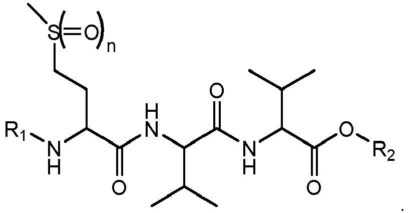 wheren n is 1 or 2;
R₁ is hydrogen or a group R^{∗}-(C=O)-;
R₂ is hydrogen or R^{∗}; and
R^{∗} is independently selected at each occurrence from C₁₋₂₀ hydrocarbons optionally containing from 1-8 heteroatoms. In some embodiments, the peptide derivative may have the structure: 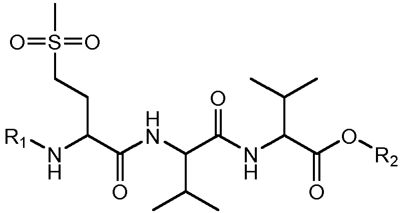 or 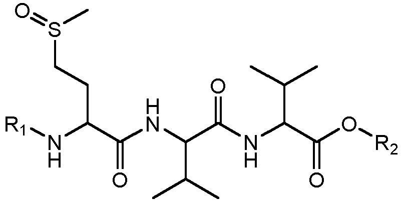 wherein R₁ and R₂ are as defined above. In some embodiments, R₂ is hydrogen. In some embodiments, R₁ is acyl (*e.g.,* C₁-C₂₀ acyl such as acetyl, ethyl acetyl, palmitoyl, oleoyl, myristyl, etc.).

It is within the skill in the art to prepare and derivatize the peptides using, for example, conventional protection and activation chemistry. Typically, the amino functionality of a first amino acid is protected with a removable amino protecting group and the carboxyl functionality of a second amino acid is protected with a removable carboxyl protecting group. Suitable amine protecting groups include, without limitation, benzoyloxycarbonyl (Cbz), tert-butoxycarbonyl (t-Boc), and 9-flourenylmethloxycarbonyl (FMOC). The carboxyl group may be protected by forming an acid or base labile ester such as a methyl, ethyl, benzyl, or trimethylsilyl esters. After protection, the first and second amino acids are reacted in a suitable solvent such as water or DMF in the presence of an *in situ* activating agent such as N,N'-dicyclohexylcarbodiimide (DCCI), diisopropylcarbodiimide (DIPCDI), or 1-ethyl-3-(3'-dimethylaminopropyl)carbodiimide (EDCI) to effect peptide bond formation. Reactive moieties on the side chains of either amino acid are protected with protecting groups such as *tert*-butyl or benzyl for OH and SH; methyl, ethyl, tert-butyl or benzyl for carboxyl groups, and 2,2,5,7,8-pentamethylchroman-6-sulphonyl for the -NHC(NH₂)=NH functionality of Arg. Following the coupling reaction, selective deprotection of the amino group of the first amino acid is accomplished by acid hydrolysis under conditions that do not remove the carboxyl protecting group of the second amino acid. The procedure is repeated with additional amino protected amino acids. Solid phase synthesis, such as the well-known Merrifield method, is especially useful for synthesizing the peptides of the invention. Lysine-amino valeric acid (K-ava) derivatives are described in U.S. Patent No. 8,551,956.

### Topical Compositions

Topical compositions of the present invention may comprise an active agent comprising a peptide having an amino acid sequence comprising or consisting of M(O)₂VV or M(O)VV of SEQ ID NO: 369, 761, 762, 1155-1157, 1550 - 1553, 1945 - 1949, 2340 - 2345, 2735 - 2741, 3130 - 3136 or 3525 - 3531 or a derivative thereof, wherein one or more amino acid residue of said sequence are oxidized. In some embodiments, the active agent may have the structure: 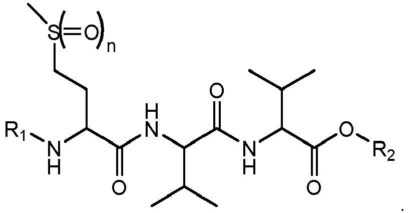 wheren n is 1 or 2;
R₁ is hydrogen or a group R^{∗}-(C=O)-;
R₂ is hydrogen or R^{∗}; and
R^{∗} is independently selected at each occurrence from C₁₋₂₀ hydrocarbons optionally containing from 1-8 heteroatoms. In some embodiments, the peptide derivative may have the structure: 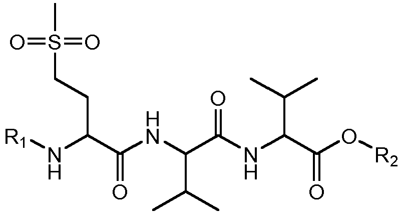 or 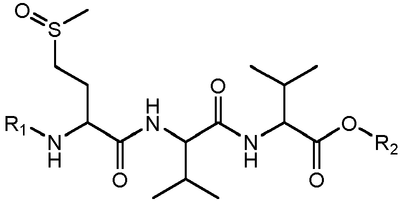 wherein R₁ and R₂ are as defined above. In some embodiments, R₂ is hydrogen. In some embodiments, R₁ is acyl (*e.g.,* C₁-C₂₀ acyl such as acetyl, ethyl acetyl, palmitoyl, oleoyl, myristyl, etc.). The active agent may be present in an effective amount of, for example, 0.00001%-20%.

The compositions according to the invention may be formulated in a variety of forms for topical application and will typically comprise from 0.000001% by weight to 20% by weight of the peptide derivative. More typically, the composition will comprise from 0.00001% peptide derivative by weight to 10% peptide derivative by weight, and more preferably from 0.00001% by weight to 5% peptide derivative by weight of the composition. In one embodiment, the active peptide or a fragment or derivative thereof will comprise from 0.001% by weight to 1% by weight or from 0.001% by weight or to 0.1% by weight of the composition. In some embodiments, the composition will comprise between 0.000001%-0.1% *(e.g.,* 0.00001%-0.1%, 0.00001%-0.01%, etc.) peptide derivative by weight of the composition. The compositions may comprise an effective amount of the peptide derivative, by which is meant an amount sufficient to stimulate production of collagen and/or hyaluronic acid in the skin *(e.g.,* from 0.000001% by weight to 20% by weight of the peptide derivative). In other embodiments, the amount of peptide or derivative thereof will be sufficient to diminish the appearance of dermatological signs of aging in a given area of skin when topically applied thereto daily for a period of at least eight weeks.

The peptide derivatives comprising or consisting of M(O)₂W or M(O)VV *(e.g.* of SEQ ID NOs: 369, 761, 762, 1155-1157, 1550 - 1553, 1945 - 1949, 2340 - 2345, 2735 - 2741, 3130 - 3136 or 3525 - 3531 wherein one or more the methionine amino acid residue are oxidized) may be provided in physiologically acceptable vehicles or carriers. The vehicle may be either hydrophobic or hydrophilic. Suitable, hydrophobic carriers include, for example, waxy non-ionic substances commonly used in cosmetics, such as esters and ethers of fatty alcohols and of fatty acids, with carbon chain length from C₄ to C₂₂, typically from C₈ to C₁₈, or from C₁₂ to C₁₈.

Examples of fatty hydrophobic carriers include isopropyl myristate, isopropyl palmitate, octyl palmitate, isopropyl lanolate, acetylated lanolin alcohol, the benzoate of C₁₂-C₁₅ alcohols, cetearyl octanoate, cetyl palmitate, myristyl myristate, myristyl lactate, cetyl acetate, propylene glycol dicaprylate/caprate, decyl oleate, acetylated lanolin, stearyl heptanoate, diisostearyl malate, octyl hydroxystearate, octyl hydroxystearate, isopropyl isostearate, and the like.

Suitable hydrophilic carriers may comprise, for example, water, lower alcohols (C₁₋₆) such as ethanol, mixtures of ethanol and water, glycols, and alkoxylated glycols commonly used in cosmetics, including ethylene glycol, diethylene glycol, triethylene glycol, propylene glycol, dipropylene glycol, and the like.

The topically acceptable vehicle may be in the form of an emulsion. Non-limiting examples of suitable emulsions include water-in-oil emulsions, oil-in-water emulsions, silicone-in-water emulsions, water-in-silicone emulsions, wax-in-water emulsions, water-oil-water triple emulsions or the like having the appearance of a cream, gel or microemulsions. As used herein, the term "oil" includes silicone oils unless otherwise indicated. The emulsion may include an emulsifier, such as a nonionic, anionic or amphoteric surfactant, or a gellant, typically in an amount from 0.001% to 5% by weight.

The topically acceptable vehicle may include water; vegetable oils; mineral oils; ester oils; ethers such as dicapryl ether and dimethyl isosorbide; alcohols such as ethanol and isopropanol; fatty alcohols such as cetyl alcohol, cetearyl alcohol, stearyl alcohol and behenyl alcohol; isoparaffins such as isooctane, isododecane (IDD) and isohexadecane; silicone oils such as cyclomethicone, dimethicone, dimethicone cross-polymer, polysiloxanes and their derivatives, including PDMS, dimethicone copolyol, dimethiconols, and amodimethiconols; hydrocarbon oils such as mineral oil, petrolatum, isoeicosane and polyolefins, e.g., (hydrogenated) polyisobutene; polyols such as propylene glycol, glycerin, butylene glycol, pentylene glycol, hexylene glycol, caprylyl glycol; waxes such as beeswax, carnauba, ozokerite, microcrystalline wax, polyethylene wax, and botanical waxes; or any combinations or mixtures of the foregoing. Aqueous vehicles may include one or more solvents miscible with water, including lower alcohols, such as ethanol, isopropanol, and the like. The vehicle may comprise from 50% to 99% by weight of the composition. In some embodiments, the compositions are anhydrous.

In one embodiment of the invention, the compositions may include one or more additional skin actives, including but not limited to, retinoids, botanicals, keratolytic agents, desquamating agents, keratinocyte proliferation enhancers, collagenase inhibitors, elastase inhibitors, depigmenting agents, anti-inflammatory agents, steroids, anti-acne agents, antioxidants, and advanced glycation end-product (AGE) inhibitors, to name but a few. The amounts of these various ingredients are those conventionally used in the cosmetic field to achieve their intended purpose, and range individually or collectively typically from 0.001 wt % to 20 wt % by weight of the composition. The nature of these ingredients and their amounts must be compatible with the production and function of the compositions of the disclosure.

Exemplary anti-aging components include, without limitation, botanicals (*e.g., Butea* frondosa extract, *Tiliacora* triandra extract, *Portulaca* oleracea, *Melicope* elleryana, etc.); phytol; phytonic acid; retinoids; hydroxy acids (including alpha-hydroxy acids and beta-hydroxy acids), salicylic acid and alkyl salicylates; exfoliating agents (*e.g.,* glycolic acid, 3,6,9-trioxaundecanedioic acid, etc.), estrogen synthetase stimulating compounds (*e.g.,* caffeine and derivatives); compounds capable of inhibiting 5 alpha-reductase activity (*e.g.,* linolenic acid, linoleic acid, finasteride, and mixtures thereof); and barrier function enhancing agents *(e.g.,* ceramides, glycerides, cholesterol and its esters, alpha-hydroxy and omega-hydroxy fatty acids and esters thereof, etc.), to name a few.

Exemplary retinoids include, without limitation, retinoic acid (*e.g.,* all-trans, or 9-cis, or 13-cis), and derivatives thereof, retinaldehyde, retinol (Vitamin A) and esters thereof, such as retinyl palmitate, retinyl acetate and retinyl propionate, and salts thereof. Particular mention may be made of retinol. When present, the retinoids will typically be included in amounts from 0.0001% to 5% by weight, more typically from 0.01% to 2.5% by weight, or from 0.1% to 1.0% by weight. Compositions according to this embodiment will typically include an antioxidant such as ascorbic acid and/or BHT and/or a chelating agent such as EDTA or a salt thereof *(e.g.,* disodium EDTA) in amounts effective to stabilize the retinoid *(e.g.,* 0.0001% - 5%). The composition may include from 0.001-10% by weight phytol.

In another embodiment, the topical compositions of the present invention may also include one or more of the following: a skin penetration enhancer; an emollient, such as isopropyl myristate, petrolatum, volatile or non-volatile silicones oils (*e.g.,* methicone, dimethicone), ester oils, mineral oils, and fatty acid esters; a humectant, such as glycerin, hexylene glycol or caprylyl glycol; a skin plumper, such as palmitoyl oligopeptide, collagen, collagen and/or glycosaminoglycan (GAG) enhancing agents; an exfoliating agent; and an antioxidant.

Suitable exfoliating agents include, for example, alpha-hydroxy acids, beta-hydroxy acids, oxa-acids, oxadiacids, and their derivatives such as esters, anhydrides and salts thereof. Suitable hydroxy acids include, for example, glycolic acid, lactic acid, malic acid, tartaric acid, citric acid, 2-hydroxyalkanoic acid, mandelic acid, salicylic acid and derivatives thereof. One exemplary exfoliating agent is glycolic acid. When present, the exfoliating agent may comprise from 0.001% to 20% by weight of the composition.

Examples of antioxidants that may be used in the present compositions include compounds having phenolic hydroxy functions, such as ascorbic acid and its derivatives/esters; beta-carotene; catechins; curcumin; ferulic acid derivatives (*e.g.,* ethyl ferulate, sodium ferulate); gallic acid derivatives (*e.g.,* propyl gallate); lycopene; reductic acid; rosmarinic acid; tannic acid; tetrahydrocurcumin; tocopherol and its derivatives, including tocopheryl acetate; uric acid; or any mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur. In one embodiment, the composition comprises thiodipropionic acid or a mono- or diester thereof such as dilauryl thiodipropionic acid. Antioxidants may comprise, individually or collectively, from 0.001% to 10 % (w/w), or from 0.01% to 5% (w/w) of the total weight of the composition.

Other additives include: vitamins, such as tocopherol and ascorbic acid; vitamin derivatives such as ascorbyl monopalmitate, tocopheryl acetate, and Vitamin E palmitate; thickeners such as hydroxyalkyl cellulose, carboxymethylcellulose, carbombers, and vegetable gums such as xanthan gum; gelling agents, such as ester-terminated polyester amides; structuring agents; metal chelating agents such as EDTA or salts thereof; fillers and powders, colorants, pH adjusters (citric acid, ethanolamine, sodium hydroxide, etc.); film formers, moisturizers, minerals, viscosity and/or rheology modifiers, anti-acne agents, antiinflammatories, depigmenting agents, pharmaceutical agents, surfactants, botanicals, sunscreens, insect repellents, skin cooling compounds, skin protectants, conditioners, lubricants, fragrances, excipients, preservatives, stabilizers, emulsifiers, and mixtures thereof. The foregoing may individually or collectively comprise from 0.0001% to 20% by weight of the composition.

Details with respect to these and other suitable cosmetic ingredients can be found in the "International Cosmetic Ingredient Dictionary and Handbook," 10th Edition (2004), published by the Cosmetic, Toiletry, and Fragrance Association (CTFA), at pp. 2177-2299. The amounts of these various substances are those that are conventionally used in the cosmetic or pharmaceutical fields, for example, they can constitute individually or in the aggregate, from 0.01% to 20% of the total weight of the composition.

A sunscreen may be included to protect the skin from damaging ultraviolet rays. The sunscreen may provide both UVA and UVB protection, by using either a single sunscreen or a combination of sunscreens. Among the sunscreens that can be employed in the present compositions are avobenzone, cinnamic acid derivatives (such as octylmethoxy cinnamate), octyl salicylate, homosalate, oxybenzone, octocrylene, titanium dioxide, zinc oxide, or any mixtures thereof. The sunscreen may be present from 1 wt % to 30 wt % of the total weight of the composition.

In one embodiment, the topical composition will have a pH range from 1 to 13, with a pH in the range of from 2 to 12 being typical. In some embodiment, the composition will have a pH in the range of from 3.5 to 7 or from 7-10.5. In some embodiments, the pH will be in the range of 3-4, or 4-5, or 5-6, or 6-7, or 7-8, or 8-9, or 9-10, or 10-11, or 11-12. Suitable pH adjusters such as sodium hydroxide, citric acid and triethanolamine may be added to bring the pH within the desired range.

Another embodiment of the present disclosure is directed to the delivery of the described compositions by the use of targeted delivery systems, for example, liposomes, microspheres (see, *e.g.,* U.S. Pat. No. 5,770,222 to Unger et al.), and the like, so that the components and/or active constituents can more readily reach and affect the subcutaneous layer of the area of application, *e.g.,* face or neck, or the other area of the skin.

The compositions may be formulated in a variety of product forms, such as, for example, a lotion, cream, serum, spray, aerosol, cake, ointment, essence, gel, paste, patch, pencil, towelette, mask, stick, foam, elixir, concentrate, and the like, particularly for topical administration. The composition is typically formulated as a lotion, cream, ointment, serum, or gel.

### Non-Therapeutic Methods of Treatment

The invention also provides a non-therapeutic method for ameliorating and/or preventing signs of human skin photo- and intrinsic aging comprising topically applying the compositions of the invention. The compositions of the invention are preferably applied to affected skin areas once or twice daily for as long as is necessary to achieve desired anti-aging results. In one embodiment, the compositions of the invention will be applied to the skin in an amount from 0.001 to 100 mg/cm², more typically from 0.01 to 20 mg/cm², or from 0.1 to 10 mg/cm².

In some embodiments, non-therapeutic methods for enhancing the production of pro-collagen, collagen and/or HA in human skin comprise topically applying to an area of the skin in need thereof (*e.g.,* sagging skin, thinning skin, skin suffering from wrinkles and fine lines, etc.) a topical composition comprising a topically acceptable vehicle, and an effective amount of a peptide derivative comprising or consisting of M(O)₂VV or M(O)VV *(e.g.,* a peptide comprising M(O)₂VV or M(O)VV of any of SEQ ID NOs: 369, 761, 762, 1155-1157, 1550 - 1553, 1945 - 1949, 2340 - 2345, 2735 - 2741, 3130 - 3136 or 3525 - 3531 wherein the methionine amino acid residue of MVV in said sequence is chemically modified by oxidation), for a time sufficient to enhance the levels of pro-collagen, collagen, and/or HA in the dermis. The treatment may be at least once or twice daily and may last for a period of at least four weeks, typically at least eight weeks, twelve weeks, or longer.

The compositions may be applied topically to improve the aesthetic appearance of human skin. The non-therapeutic method may comprise topically applying to an area of the skin in need thereof a composition comprising an effective amount of the peptide derivative comprising or consisting of M(O)₂VV or M(O)VV *(e.g.,* a peptide comprising M(O)₂VV or M(O)VV of any of SEQ ID NOs: 369, 761, 762, 1155-1157, 1550 - 1553, 1945 - 1949, 2340 - 2345, 2735 - 2741, 3130 - 3136 or 3525 - 3531wherein the methionine amino acid residue of MVV in said sequence is chemically modified by oxidation) for a time sufficient to improve the aesthetic appearance of said human skin. The composition may optionally further comprise a retinoid *(e.g.,* from 0.0001-5%) and/or an alpha-hydroxy acid *(e.g.,* glycolic acid) *(e.g.,* from 0.0001-25%) and/or a beta-hydroxy acid *(e.g.,* salicylic acid or a derivative) *(e.g.,* from 0.0001-15%).

The improvement in aesthetic appearance of human skin may be an improvement of any attribute or characteristic of skin, including without limitation:
(a) treatment, reduction, and/or prevention of fine lines or wrinkles;
(b) reduction of skin pore size;
(c) improvement in skin thickness, plumpness, and/or tautness;
(d) improvement in skin smoothness, suppleness and/or softness;
(e) improvement in skin tone, radiance, and/or clarity;
(f) improvement in procollagen, and/or collagen production;
(g) improvement in maintenance and remodeling of elastin;
(h) improvement in skin texture and/or promotion of retexturization;
(i) improvement in skin barrier repair and/or function;
(j) improvement in appearance of skin contours;
(k) restoration of skin luster and/or brightness;
(l) replenishment of essential nutrients and/or constituents in the skin;
(m) improvement of skin appearance decreased by aging and/or menopause;
(n) improvement in skin moisturization;
(o) increase in skin elasticity and/or resiliency;
(p) treatment, reduction, and/or prevention of skin sagging;
(q) improvement in skin firmness; and
(r) reduction of pigment spots and/or mottled skin; and
(s) improvement of optical properties of skin by light diffraction or reflection.

As used herein, "aesthetic improvement" may be measured by evaluation of before and after pictures by panels of dermatologists, or by other objective measures known in the art.

In a related implementation, a non-therapeutic method is provided for the treatment of wrinkles and/or fine lines on the skin human skin (typically, skin of the face) comprising topically applying to an area of the skin in need thereof (*e.g.,* applying to a wrinkle or fine line) a composition comprising the peptide derivative comprising or consisting of M(O)₂VV or M(O)VV *(e.g.,* a peptide comprising M(O)₂VV or M(O)VV of any of SEQ ID NOs: 369, 761, 762, 1155-1157, 1550 - 1553, 1945 - 1949, 2340 - 2345, 2735 - 2741, 3130 - 3136 or 3525 - 3531wherein the methionine amino acid residue of MVV in said sequence is chemically modified by oxidation), for a time sufficient to reduce the visibility, number, or depth of said wrinkles and/or fine lines. The treatment may be a least once or twice daily and may last for a period of at least four weeks, typically at least eight weeks, twelve weeks, or longer. The composition may optionally further comprise a retinoid (*e.g.,* retinol or retinyl palmitate) and/or an alpha-hydroxy acid *(e.g.,* glycolic acid) and/or a beta-hydroxy acid *(e.g.,* salicylic acid or derivative) in amounts effective to improve the appearance of skin. In some embodiments, non-therapeutic methods reduce the severity of, reduce the number of, or prevent or forestall the onset of, wrinkles or fine lines on human skin. The composition may be topically applied to an area of the skin in need thereof *(e.g.,* directly to wrinkled skin), an effective amount *(e.g.,* 0.000001% - 1% by weight, w/w) of a peptide derivative comprising or consisting of M(O)₂VV or M(O)VV *(e.g.,* a peptide comprising M(O)₂VV or M(O)VV any of SEQ ID NOs: 369, 761, 762, 1155-1157, 1550 - 1553, 1945 - 1949, 2340 - 2345, 2735 - 2741, 3130 - 3136 or 3525 - 3531 wherein the methionine amino acid residues of MVV in said sequence is chemically modified by oxidation) in combination with an effective amount (*e.g.,* 0.01% - 5% by weight, w/w) of retinol and/or an effective amount (*e.g.,* 0.001% - 20% by weight, w/w) of an alpha-hydroxy acid *(e.g.,* glycolic acid) and/or a beta-hydroxy acid *(e.g.,* salicylic acid). The effect of a composition on the formation or appearance of fine lines and wrinkles can be evaluated qualitatively, *e.g.,* by visual inspection, or quantitatively, *e.g.,* by microscopic or computer assisted measurements of wrinkle morphology (*e.g.,* the number, depth, length, area, volume and/or width of wrinkles per unit area of skin).

Topical application of a composition comprising a peptide derivative comprising or consisting of M(O)₂VV or M(O)VV *(e.g.,* a peptide comprising any of SEQ ID NOs: 369, 761, 762, 1155-1157, 1550 - 1553, 1945 - 1949, 2340 - 2345, 2735 - 2741, 3130 - 3136 or 3525 - 3531wherein the methionine amino acid residues of MVV in said seqnence is chemically modified by oxidation), typically in a physiologically acceptable vehicle, over an affected area of skin may remediate, reverse, reduce, ameliorate, or prevent dermatological signs of aging. Generally, the improvement in the condition and/or appearance of skin is selected from the group consisting of: reducing dermatological signs of chronological aging, photo-aging, hormonal aging, and/or actinic aging; preventing and/or reducing the appearance of lines and/or wrinkles; reducing the noticeability of facial lines and wrinkles, facial wrinkles on the cheeks, forehead, perpendicular wrinkles between the eyes, horizontal wrinkles above the eyes, and around the mouth, marionette lines, and particularly deep wrinkles or creases; improving the appearance of suborbital lines and/or periorbital lines; reducing the appearance of crow's feet; rejuvenating and/or revitalizing skin, particularly aging skin; reducing skin fragility; preventing and/or reversing of loss of glycosaminoglycans and/or collagen; ameliorating the effects of estrogen imbalance; preventing skin atrophy; preventing, reducing, and/or treating hyperpigmentation or hypopigmentation; minimizing skin discoloration; improving skin tone, radiance, clarity and/or tautness; preventing, reducing, and/or ameliorating skin sagging; improving skin firmness, plumpness, suppleness and/or softness; improving procollagen and/or collagen production; improving skin texture and/or promoting retexturization; improving skin barrier repair and/or function; improving the appearance of skin contours; restoring skin luster and/or brightness; minimizing dermatological signs of fatigue and/or stress; resisting environmental stress; replenishing ingredients in the skin decreased by aging and/or menopause; improving communication among skin cells; increasing cell proliferation and/or multiplication; increasing skin cell metabolism decreased by aging and/or menopause; retarding cellular aging; improving skin moisturization; enhancing skin thickness; slowing or halting skin thinning; increasing skin elasticity and/or resiliency; enhancing exfoliation; improving microcirculation; decreasing and/or preventing cellulite formation; and any combinations thereof. In some embodiments, each of the forgoing is associated with female skin.

It is also contemplated that the compositions of the invention will be useful for treating thin skin by topically applying the composition comprising the active peptides derivatives comprising or consisting of M(O)₂VV or M(O)VV to thin skin of an individual in need thereof. "Thin skin" is intended to include skin that is thinned due to chronological aging, menopause, or photo-damage and skin that is thinning prematurely. In some embodiments, the treatment is for thin skin in men, whereas other embodiments treat thin skin in women, pre-menopausal or post-menopausal, as it is believed that skin thins differently with age in men and women, and in particular in women at different stages of life.

The non-therapeutic method of the invention may be employed prophylactically to forestall aging including in individuals that have not manifested signs of skin aging, most commonly in individuals under 25 years of age. The method may also reverse or treat signs of aging once manifested as is common in individuals over 25 years of age, or to slow the progression of dermatological aging in such individuals.

In one embodiment, the compositions of the invention comprising active peptide derivatives comprising or consisting of M(O)₂VV or M(O)VV *(e.g.,* a peptide comprising any of SEQ ID NOs: 2-3531 wherein in one or more amino acid residues of SEQ ID NOs 2-3531 are chemically modified such as by oxidation) are applied to human skin to reduce sebum production or improve the appearance of skin affected by cellulite, and/or reduce unwanted lipogenesis or increase lipolysis. In this embodiment, the peptides of the invention can be formulated in topically acceptable vehicles (as described herein) and may include one or more additional agents such as anti-acne ingredients (*e.g.,* salicylic acid, benzoyl peroxide and other peroxides, sulfur, retinoids, etc.) in the case of a facial composition, or, in the case of a cellulite treatment, the formulation may comprise any ingredients suitable for treatment of cellulite, including without limitation, perilla oil and other unsaturated fatty oils and omega-3 fatty acids such as alpha-linolenic acid; caffeine; theophylline; xanthines; retinoids (*e.g.,* retinol); and the like. A cellulite treatment may typically be applied topically to skin suffering from cellulite, including skin of the buttocks and thighs for a period of time sufficient to improve the appearance thereof, including for example, daily treatment for at least four weeks, at least eight weeks, at least twelve weeks, or longer. In one embodiment, the compositions are topically applied to treat acne.

In certain embodiments, the compositions described herein comprising active peptide derivatives comprising or consisting of M(O)₂VV or M(O)VV can be used to treat and/or prevent hyper-pigmentation of skin and/or of the hair, for example, to lighten skin or hair. In some embodiments, the compositions are topically applied to the skin or hair, for example to an area of hyper-pigmented skin or hair. Hyper-pigmentation includes any coloration of an individual's skin or hair that is darker than desired by the individual and that is caused by melanocytes. Hyper-pigmented areas of the skin include areas of discrete or mottled hyperpigmentation. Areas of discrete hyper-pigmentation can be distinct, uniform areas of darker color and may appear as brown spots or freckles on the skin, including marks commonly called pigment spots or "age spots." Areas of mottled hyper-pigmentation of the skin can be dark blotches that are larger and more irregular in size and shape than areas of discrete pigmentation. Areas of hyper-pigmentation also include areas of tanned skin, for example, skin tanned due to UV exposure. Hyper-pigmented hair includes any shade of hair that is darker than desired.

Treating hyper-pigmentation or hyper-pigmented skin/hair refers to eradicating, reducing, ameliorating, or reversing one or more of the unwanted features associated with hyper-pigmentation, such as producing a perceptible lightening of the skin or hair in the affected area. Lightening hyper-pigmented areas of the skin may be effective in diminishing age spots; lightening a suntan; evening or optimizing skin tones, *e.g.,* in areas of mottled hyper-pigmentation; in treating melasmic and chloasmic patches, freckles, after-burn scars, and post-injury hyper-pigmentation. Preventing hyper-pigmentation or hyper-pigmented skin refers to affording skin, not yet affected by hyper-pigmentation, a benefit that serves to avoid, delay, forestall, or minimize one or more unwanted features associated with skin hyper-pigmentation, such as reducing the darkness or size of hyper-pigmented areas that eventually develop.

In some embodiments, the compositions of the invention are used in a rotational, alternating, or sequential treatment regimen comprising topical application of the compositions of the invention for a first period of time *(e.g.,* at least once daily for at least one day), followed by a second period of time in which at least one additional treatment modality is administered for at least one additional day following said first period of time. The second treatment modality may comprise topical application of any skin benefit agent, such as a retinoid (*e.g.,* retinol), phytol, antioxidants (*e.g.,* ascorbic acid or TDPA or esters thereof), botanicals, such as *Tiliacora triandra,* niacinamide, vitamins such as Vitamin E and Vitamin E acetate, salicylic acid, salicylates and derivatives thereof, moisturizers, emollients, etc.

The composition is intended for use as a non-therapeutic treatment. In one embodiment, the composition is an article intended to be rubbed, poured, sprinkled, or sprayed on, introduced into, or otherwise applied to the human body for cleansing, beautifying, promoting attractiveness, or altering the appearance, in accordance with the US FD&C Act, §201(i).

### EXAMPLES

The following example illustrates a specific aspect of the instant description. The example should not be construed as limiting, as the example merely provides specific understanding and practice of the embodiments and its various aspects.

### EXAMPLE 1: In vitro measurements on human dermal fibroblasts

The peptides of the invention were synthesized by GenScript (Piscataway, NJ).

Human dermal fibroblast cells were grown in a 96 well plate in DMEM media (available from Corning, NY) supplemented with 10% Fetal Bovine Serum (FBS) and L-glutamine (0.07×10⁵ cells/plate). After reaching 75% confluence, cells were transferred into DMEM media without FBS and incubated for 4-6 hours. Next, cells were treated with a peptide or prodrug thereof (MVV, Ac-MVV) or peptide derivative or prodrug thereof (M(O)₂VV, Ac-M(O)₂VV, Ac-M(O)VV) at 0.00001%, 0.0001%, 0.0005%, or 0.001% final concentration in DMEM media without FBS for 48h. After treatment, the media were collected and cell viability was measured using MTT. The amount of collagen or hyaluronic acid (HA) secreted was tested in the media using an HTRF human pro-collagen I kit or an HTRF HABP kit, respectively (each available from Cisbio Inc., Bedford, MA).

The results for HA production following administration to the fibroblasts are summarized in Table 13 as percent change of HA production for each active relative to vehicle control. p values are given with respect to the change in HA on the fibroblasts with vehicle control administration or with respect to MVV administration.

**TABLE 13**

| Active | Concentration (wt %) | HA % change | p value (vehicle) | p value (MVV) |
|---|---|---|---|---|
| MVV | 0.001% | 9.00% | 0.000518 | |
| | 0.0005% | 3.66% | 0.111352 | |
| | 0.0001% | 1.63% | 0.442766 | |
| | 0.00001% | 5.79% | 0.05904 | |
| Ac-MVV | 0.001% | 12.14% | 0.000223 | 0.0176 |
| | 0.0005% | 6.62% | 0.014093 | 0.0149 |
| | 0.0001% | 14.49% | 2.8E-05 | 4.34E-05 |
| | 0.00001% | 5.47% | 0.206767 | 0.0365 |
| M(O)₂VV | 0.001% | 19.43% | 1.88E-05 | 0.281 |
| | 0.0005% | 10.25% | 0.000195 | 0.316 |
| | 0.0001% | 17.49% | 9.83E-07 | 0.000784 |
| | 0.00001% | 14.01% | 1.1E-05 | 0.957 |
| Ac-M(O)₂VV | 0.001% | 11.04% | 0.005651 | 0.409 |
| | 0.0005% | 7.90% | 0.069938 | 0.253 |
| | 0.0001% | 7.31% | 0.058824 | 0.111 |
| | 0.00001% | 10.33% | 0.014505 | 0.316 |
| Ac-M(O)VV | 0.001% | 21.87% | 0.000145 | 0.0114 |
| | 0.0005% | 17.69% | 0.001447 | 0.0205 |
| | 0.0001% | 16.95% | 0.000128 | 0.000145 |
| | 0.00001% | 17.50% | 6.2E-05 | 0.00658 |

The results for collagen production following administration to the fibroblasts are summarized in Table 14 as percent change of collagen production for each active relative to vehicle control. p values are given with respect to the change in collagen on the fibroblasts with vehicle control administration or MVV administration.

**TABLE 14**

| Active | Concentration (wt %) | Collagen % change | p value (vehicle) | p value (MVV) |
|---|---|---|---|---|
| MVV | 0.001% | 6.22% | 0.034145 | |
| | 0.0005% | 31.22% | 0.164416 | |
| | 0.0001% | 10.56% | 0.0167 | |
| | 0.00001% | 7.22% | 0.011684 | |
| Ac-MVV | 0.001% | 2.21% | 0.366131 | 0.200 |
| | 0.0005% | -2.64% | 0.400509 | 0.231 |
| | 0.0001% | 10.60% | 0.028688 | 0.996 |
| | 0.00001% | -8.24% | 0.004029 | 0.000196 |
| M(O)₂VV | 0.001% | 6.07% | 0.010456 | 0.951 |
| | 0.0005% | 1.56% | 0.474114 | 0.287 |
| | 0.0001% | 12.51% | 0.000636 | 0.702 |
| | 0.00001% | 5.74% | 0.051757 | 0.645 |
| Ac-M(O)₂VV | 0.001% | 13.65% | 0.000235 | 0.0947 |
| | 0.0005% | -0.56% | 0.785196 | 0.257 |
| | 0.0001% | 11.29% | 7.69E-06 | 0.874 |
| | 0.00001% | 12.29% | 2.74E-05 | 0.111 |
| Ac-M(O)VV | 0.001% | 13.49% | 6E-06 | 0.0364 |
| | 0.0005% | 14.02% | 0.000116 | 0.532 |
| | 0.0001% | 4.59% | 0.026163 | 0.220 |
| | 0.00001% | 7.20% | 0.008935 | 0.994 |

As shown in Table 14, peptides derivatives of the invention effectively increase pro-collagen I acid production in human dermal fibroblast cells greater than the underivatized version. FIGS. 1A-1D graphically illustrate the percent increases in HA and collagen at each tested derivative concentration shown in Tables 13 and 14.

### EXAMPLE 2: Full Thickness Skin Model Assays

A series of experiments was conducted to assess the effects of peptide derivatives on the production of hyaluronic acid (HA) on full thickness 3D skin cultures. Five different treatment regimens were assessed: (1) 0.01% by weight M(O)₂VV in vehicle; (2) 0.002% by weight M(O)₂VV in vehicle; (3) 0.01% by weight Ac-M(O)₂VV in vehicle; (4) 0.002% by weight Ac-M(O)₂VV in vehicle; and (5) vehicle alone (DMSO). Compositions at the indicated weight percentages in DMSO were applied to 3D skin equivalents daily for 3 consecutive days.

Human 3D skin EFT400FT tissues (MatTek, MA) were cultured following the manufacturer's instructions. Each treatment regimen was topically applied to six 3D skin samples. Different 3D skin samples were assigned to one of treatment regimens (1)-(5) as described above, with six skin tissue models allocated to each treatment regimen (*i.e.,* n=6). Following treatment, media were collected and the amount of hyaluronic acid secreted from the cells was measured using a HTRF HABP kit (available from Cisbio Inc., Bedford, MA).

The results for HA production are shown in Table 15. p values are given in relation to administration with vehicle control alone or in relation to MVV.

**TABLE 15**

| Peptide Derivative | Concentration (wt % (ppm)) | % HA change | p value |
|---|---|---|---|
| M(O)₂VV | 0.01% (100ppm) | 10.27567 | 6.11E-05 |
| M(O)₂VV | 0.002% (20ppm) | 6.236025 | |
| Ac-M(O)₂VV | 0.01% (100ppm) | 12.46642 | 7.17E-05 |
| Ac-M(O)₂VV | 0.002% (20ppm) | 1.386264 | |

### EXAMPLE 3: Ex vivo Assays

Samples of explanted skin tissue were obtained from an abdominal plasty of a 47 year old Caucasian woman. Samples (33 in total) were maintained in tissue culture medium at 37°C in a humid, 5% CO2 atmosphere for the duration of the study.

The skin tissue explants were then distributed into appropriate controls or treated samples in triplicates. The control received only a vehicle (DMSO), and the treatment samples received vehicle comprising either M(O)₂VV or Ac-M(O)₂VV at 0.0005%, 0.001%, or 0.01% by weight of the composition. Administration of vehicle or vehicle with the peptide occurred on days 0, 1, 2, 5, and 7.

Stained tissue sections (in triplicates) were examined for qualitative and semi-qualitative differences between treated and control samples and low and high power using Zeiss Mimax and Midi BF microscope and software system. For HA evaluation, samples were washed and processed with Methylthiazolyldiphenyl-tetrazolium bromide (MTT). MTT is converted to water-insoluble dark blue colored MTT-formazan by mitochondrial dehydrogenases. Subsequently the blue crystals have been solubilized and the color intensity, that is directly proportional to skin vitality, has been measured with a plate reader at a wavelength of 570 nm. For each analyzed skin sample the obtained absorbance value has been normalized upon the weight in grams. Twelve skin suctions were stained with an acian blue stain that dyes acid mucins such as hyaluronan to develop the HA score. Dermal collagen was also measured usin a picrosirius red histochemical stain that dyes collagen fibers in purple-red. The papillary dermis was selected for the analysis as this is the portion of the dermis more subjected to collagen variation in response to treatment.

8-bit grey-scale photomicrographs were captured and transformed from RGB to the standard CIE L^{∗}a^{∗}b^{∗} color space. The L^{∗} value for each pixel in the ROI was evaluated using ImageJ software (National Institutes of Health, Bethesda, MD) with the Color Inspector 3D plug-in. The amount of hyaluron present was evaluated based on the intensity and distribution of blue color in the dermis using an imaging method analogous to that used for procollagen I. Scores reflecting the amount of hyaluronan detected were assigned using an image analysis algorithm proprietary to Cutech Srl. Higher HA scores is indicative of a higher HA content. Similarly, the amount of procollagen I was evaluated based on the intensity and distribution of immunostained procollagen I in the area corresponding to the papillary dermis, just below the epidermis. As above, 8-bit grey-scale photomicrographs were captured and transformed from RGB to the standard CIE L^{∗}a^{∗}b^{∗} color space. The L^{∗} value for each pixel in the ROI was evaluated using ImageJ software (National Institutes of Health, Bethesda, MD) with the Color Inspector 3D plug-in. Scores reflecting the amount of procollagen I detected were assigned using an image analysis algorithm proprietary to Cutech Srl.

Table 16 shows the measured HA scores in various samples and FIG. 2 graphically represents the results of the explant measurements for HA measured. Columns marked with "^{∗∗}" were highly significant (p<0.05) with respect to untreated samples and with respect to samples administered vehicle alone (DMSO).

**TABLE 16**

| | | Mean | SEM | % Change Over Untreated | % Change Over DMSO |
|---|---|---|---|---|---|
| Untreated | | 9.9 | 0.3 | | |
| DMSO | | 10.2 | 0.7 | 3% | |
| M(O)₂VV | 5 ppm | 12 | 1.2 | 21% | 18% |
| | 10 ppm | 11.6 | 1.4 | 17% | 14% |
| | 100 ppm | 9.8 | 1.1 | -1% | -4% |
| Ac-M(O)₂VV | 5 ppm | 9.5 | 0.6 | -4% | -7% |
| | 10 ppm | 12.7 | 0.7 | 28% | 25% |
| | 100 ppm | 11.1 | 1.3 | 12% | 9% |

Table 17 shows the measured collagen scores in various samples and FIG. 3 graphically represents the results of the explant measurements for collagen measured. Columns marked with "^{∗∗}" were highly significant (p<0.05) with respect to untreated samples and with respect to samples administered vehicle alone (DMSO.

**TABLE 17**

| | | Mean | SEM | % Change Over Untreated | % Change Over DMSO |
|---|---|---|---|---|---|
| Untreated | | 56.1 | 1 | | |
| DMSO | | 54.3 | 1.6 | -18% | |
| M(O)₂VV | 5 ppm | 53.6 | 1.2 | -25% | -7% |
| | 10 ppm | 57.9 | 1.4 | 18% | 35% |
| | 100 ppm | 55.6 | 1.4 | -5% | 13% |
| | 5 ppm | 61.7 | 0.9 | 57% | 73% |
| Ac-M(O)₂VV | 10 ppm | 61.9 | 1.2 | 59% | 75% |
| | 100 ppm | 59.6 | 0.9 | 35% | 52% |

## Claims

1. A topical composition comprising an active agent comprising a peptide having an amino acid sequence comprising or consisting of M(O)₂VV or M(O)VV, wherein the methionine amino acid residue of said sequence is oxidized.

2. The topical composition of claim 1, wherein the active agent is present in an amount from 0.0001% to 10% by weight of the composition.

3. The topical composition of claim 1 or 2, wherein the topically acceptable vehicle of said topical composition comprises a water-in-oil, oil-in-water, silicone-in-water, or water-in-silicone emulsion, further comprising an emulsifier.

4. The topical composition of any one of claims 1-3, further comprising an additional active ingredient selected from the group consisting of alpha-hydroxy acids, thiodipropionic acid or esters thereof, salicylic acid, niacinamide, hexyl resorcinol, phytol, and retinoids.

5. The topical composition of any one of claims 1-4, wherein said sequence consists of from 3 to 10 amino acids.

6. The topical composition of any one of claims 1-5, wherein said active agent is N-acetylated.

7. The topical composition of any one of claims 1-4, wherein said active agent has the structure: 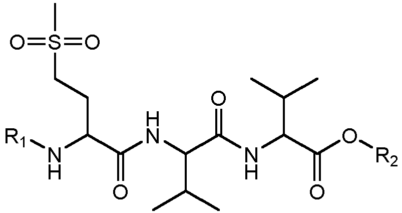
wherein R₁ is hydrogen or a group R^{∗}-(C=O)-;
R₂ is hydrogen or R^{∗}; and
R^{∗} is independently selected at each occurrence from C₁₋₂₀ hydrocarbons optionally containing from 1-8 heteroatoms.

8. The topical composition according to claim 7, wherein R₁ is R^{∗}-(C=O)- and R^{∗} is C₁₋₂₀ alkyl.

9. The topical composition of any one of claims 1-4, wherein said active agent has the structure: 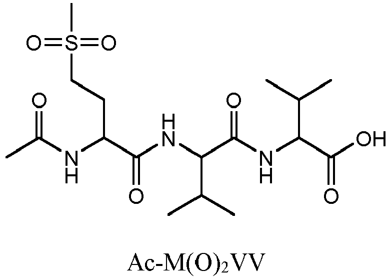 , or

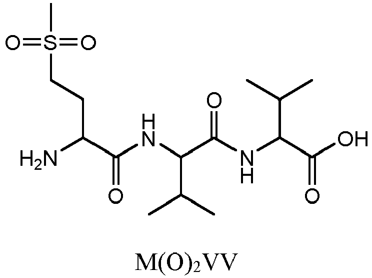

10. The topical composition of any one of claims 1-4, wherein said active agent has the structure: 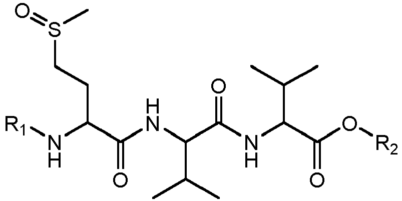
wherein R₁ is hydrogen or a group R^{∗}-(C=O)-;
R₂ is hydrogen or R^{∗}; and
R^{∗} is independently selected at each occurrence from C₁₋₂₀ hydrocarbons optionally containing from 1-8 heteroatoms.

11. The topical composition of any one of claims 1-4, wherein said peptide derivative has the structure: 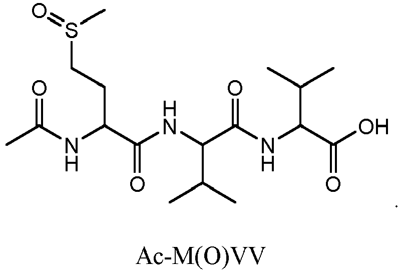 , or

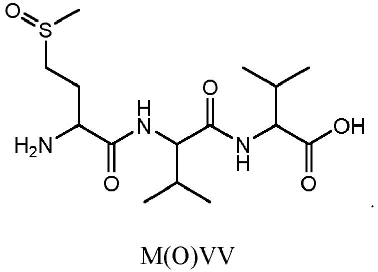

12. A non-therapeutic method for diminishing the appearance of dermatological signs of aging comprising topically applying to the skin in need thereof a topical composition according to any one of claims 1-11.

13. The method according to claim 12, wherein said dermatological signs of aging include fine lines and/or wrinkles.

14. The method according to claim 12 or 13, wherein said peptide derivative increases collagen production in skin, and/or wherein said peptide derivative increases hyaluronic acid production in skin.

15. The method according to any one of claims 12-14, wherein said composition is applied at least once daily for at least eight weeks.

## Patentansprüche

1. Topische Verbindung enthaltend einen aktiven Wirkstoff enthaltend ein Peptid, das eine Aminosäuresequenz hat, die M(O)₂VV oder M(O)VV enthält oder die aus M(O)₂VV oder M(O)VV besteht, wobei die Methioninaminosäuregruppe der Sequenz oxidiert ist.

2. Topische Verbindung nach Anspruch 1, wobei der aktive Wirkstoff in einer Menge von 0,0001% bis 10% pro Gewicht der Verbindung vorhanden ist.

3. Topische Verbindung nach einem der Ansprüche 1 bis 2, wobei der tropisch akzeptable Trägerstoff der topischen Verbindungen eine Wasser-in-Öl-, eine Öl-in-Wasser-, eine Silikonin-Wasser-, oder eine Wasser-in-Silikon-Emulsion enthält, wobei die Verbindung weiter einen Emulgator enthält.

4. Topische Verbindung nach einem der Ansprüche 1 bis 3, weiter enthaltend einen zusätzlichen aktiven Bestandteil ausgewählt aus der Gruppe bestehend aus alpha-Hydroxysäuren, Thiodipropionsäure oder Ester davon, Salicylsäure, Nikotinamid, 4-Hexylresorcin, Phytol, und Retinoiden.

5. Topische Verbindung nach einem der Ansprüche 1 bis 4, wobei die Sequenz aus 3 bis 10 Aminosäuren besteht.

6. Topische Verbindung nach einem der Ansprüche 1 bis 5, wobei der aktive Wirkstoff N-acetyliert ist.

7. Topische Verbindung nach einem der Ansprüche 1 bis 4, wobei der aktive Wirkstoff die Struktur 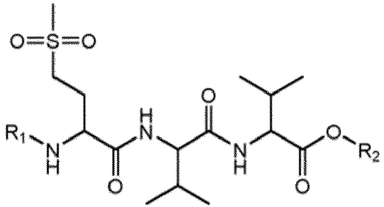 hat,
wobei R₁ Wasserstoff oder eine Gruppe R^{∗}-(C=O)- ist;
R₂ Wasserstoff oder R^{∗} ist; und
R^{∗} unabhängig bei jedem Vorkommen ausgewählt ist aus C₁₋₂₀ Kohlenwasserstoffen, die optional 1 bis 8 Heteroatome enthalten.

8. Topische Verbindung nach Anspruch 7, wobei R₁ R^{∗}-(C=O)- ist und R^{∗} C₁₋₂₀-Alkyl ist.

9. Topische Verbindung nach einem der Ansprüche 1 bis 4, wobei der aktive Wirkstoff die Struktur 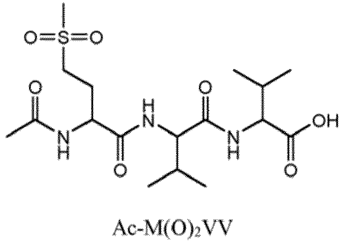 oder 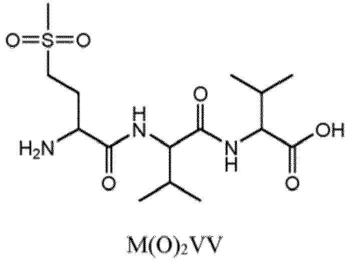 hat.

10. Topische Verbindung nach einem der Ansprüche 1 bis 4, wobei der aktive Wirkstoff die Struktur 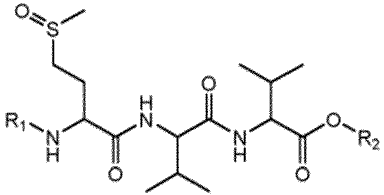 hat,
wobei R₁ Wasserstoff oder eine Gruppe R^{∗}-(C=O)- ist;
R₂ Wasserstoff oder R^{∗} ist; und
R^{∗} unabhängig bei jedem Vorkommen ausgewählt ist aus C₁₋₂₀ Kohlenwasserstoffen, die optional 1 bis 8 Heteroatome enthalten.

11. Topische Verbindung nach einem der Ansprüche 1 bis 4, wobei das Peptidderivat die Struktur 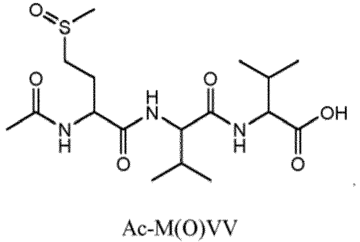 oder 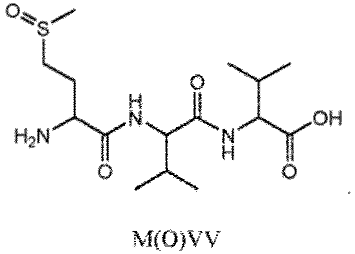 hat.

12. Nicht-therapeutisches Verfahren zur Reduzierung des Auftretens von dermatologischen Anzeichen des Alterns enthaltend das topische Anwenden einer topischen Verbindung nach einem der Ansprüche 1 bis 11 auf der Haut, die dies benötigt.

13. Verfahren nach Anspruch 12, wobei die dermatologischen Anzeichen des Alterns feine Linien und/oder Falten enthalten.

14. Verfahren nach einem der Ansprüche 12 oder 13, wobei das Peptidderivat die Kollagenproduktion in der Haut erhöht, und/oder wobei das Peptidderivat die Hyaluronsäureproduktion in der Haut erhöht.

15. Verfahren nach einem der Ansprüche 12 bis 14, wobei die Verbindung mindestens einmal täglich für mindestens acht Wochen angewendet wird.

## Revendications

1. Composition topique comprenant un agent actif comprenant un peptide doté d'une séquence d'acides aminés comprenant ou consistant en M(O)₂VV ou M(O)VV, dans laquelle le résidu d'acide aminé méthionine de ladite séquence est oxydé.

2. Composition topique selon la revendication 1, dans laquelle l'agent actif est présent dans une quantité de 0,0001 % à 10 % en poids de la composition.

3. Composition topique selon la revendication 1 ou 2, dans laquelle l'excipient acceptable d'un point de vue topique de ladite composition topique comprend une émulsion eau-en-huile, huile-en-eau, silicone-en-eau ou eau-en-silicone, comprenant en outre un émulsifiant.

4. Composition topique selon l'une quelconque des revendications 1 à 3, comprenant en outre un ingrédient actif supplémentaire choisi dans le groupe constitué par les acides alpha-hydroxy, l'acide thiodipropionique ou ses esters, l'acide salicylique, le niacinamide, l'hexyl-résorcinol, le phytol et les rétinoïdes.

5. Composition topique selon l'une quelconque des revendications 1 à 4, dans laquelle ladite séquence consiste en 3 à 10 acides aminés.

6. Composition topique selon l'une quelconque des revendications 1 à 5, dans laquelle ledit agent actif est N-acétylé.

7. Composition topique selon l'une quelconque des revendications 1 à 4, dans laquelle ledit agent actif présente la structure : 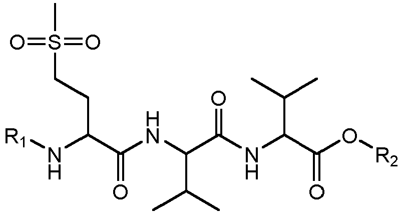
dans laquelle R₁ est un hydrogène ou un groupe R*-(C=O)- ;
R₂ est un hydrogène ou R* ; et
R* est choisi indépendamment à chaque occurrence parmi les hydrocarbures en C₁₋₂₀ contenant éventuellement de 1 à 8 hétéroatomes.

8. Composition topique selon la revendication 7, dans laquelle R₁ est R*-(C=O)- et R* est un alkyle en C₁₋₂₀.

9. Composition topique selon l'une quelconque des revendications 1 à 4, dans laquelle ledit agent actif présente la structure : 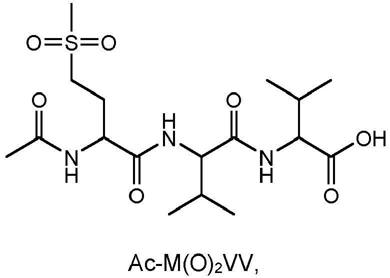 ou

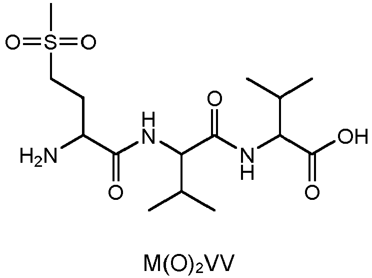

10. Composition topique selon l'une quelconque des revendications 1 à 4, dans laquelle ledit agent actif présente la structure : 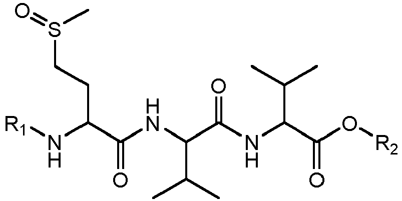
dans laquelle R₁ est un hydrogène ou un groupe R*-(C=O)- ;
R₂ est un hydrogène ou R* ; et
R* est choisi indépendamment à chaque occurrence parmi les hydrocarbures en C₁₋₂₀ contenant éventuellement de 1 à 8 hétéroatomes.

11. Composition topique selon l'une quelconque des revendications 1 à 4, dans laquelle ledit dérivé peptidique présente la structure : 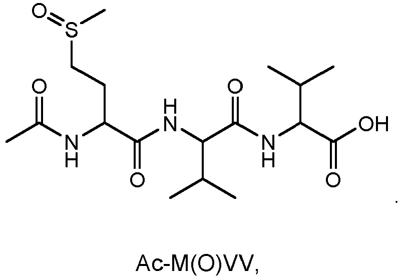 ou

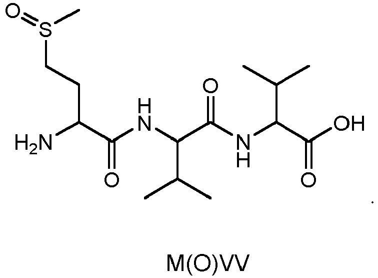

12. Procédé non thérapeutique pour diminuer l'apparition des signes dermatologiques du vieillissement comprenant l'application topique sur la peau qui en a besoin d'une composition topique selon l'une quelconque des revendications 1 à 11.

13. Procédé selon la revendication 12, dans lequel lesdits signes dermatologiques de vieillissement comprennent des ridules et/ou des rides.

14. Procédé selon la revendication 12 ou 13, dans lequel ledit dérivé peptidique augmente la production de collagène dans la peau, et/ou dans lequel ledit dérivé peptidique augmente la production d'acide hyaluronique dans la peau.

15. Procédé selon l'une quelconque des revendications 12 à 14, dans lequel ladite composition est appliquée au moins une fois par jour pendant au moins huit semaines.
